(19) Europäisches Patentamt
European Patent Office
Office européen des brevets

(11) Veröffentlichungsnummer: **0 058 373**
**A1**

(12) # EUROPÄISCHE PATENTANMELDUNG

(21) Anmeldenummer: **82100924.8**

(22) Anmeldetag: **09.02.82**

(51) Int. Cl.³: **C 07 C 87/28**
C 07 C 101/24, C 07 C 85/04
C 07 C 85/00, A 61 K 31/135
A 61 K 31/195, A 61 K 31/22

(30) Priorität: **12.02.81 CH 939/81**

(43) Veröffentlichungstag der Anmeldung:
**25.08.82 Patentblatt 82/34**

(84) Benannte Vertragsstaaten:
**AT CH DE FR LI LU NL SE**

(71) Anmelder: **CIBA-GEIGY AG**
**Patentabteilung Postfach**
**CH-4002 Basel(CH)**

(72) Erfinder: **Bucher, Karl, Prof. Dr.**
**Wielandplatz 2**
**CH-4054 Basel(CH)**

(72) Erfinder: **Matter, Max, Dr.**
**Lindenweg 3**
**CH-4052 Basel(CH)**

(72) Erfinder: **Brunner, Berhard, Dr.**
**Seegarten**
**CH-3626 Hünibach(CH)**

(72) Erfinder: **Frey, Alfred, Dr.**
**Obere Hauptgasse 8**
**CH-3600 Thun(CH)**

(74) Vertreter: **Zumstein, Fritz, Dr. et al,**
**Bräuhausstrasse 4**
**D-8000 München 2(DE)**

(54) **Substituierte aliphatische Sekundäramine und ihre Salze, diese enthaltende pharmazeutische Präparate und ihre Verwendung.**

(57) Verbindungen der Formel I

$$R_1\text{-}CH(R_2)\text{-}N(R_3)\text{-}R_4 \qquad (I)$$

worin $R_1$ unsubstituiertes oder substituiertes Phenyl bedeutet, $R_2$ Wasserstoff, Niederalkyl, unsubstituiertes oder substituiertes Phenyl oder freies oder verestertes Carboxy darstellt, und worin $R_3$ Wasserstoff oder Niederalkyl und $R_4$ eine Gruppe der Formel $-alk_1\text{-}X\text{-}alk_2\text{-}R_5$ bedeutet oder $R_3$ und $R_4$ gemeinsam eine Gruppe der Formel $-alk_3\text{-}NH\text{-}alk_4$-darstellen, wobei $alk_1$ und $alk_2$ Niederalkyliden bedeuten, $alk_3$ und $alk_4$ für Niederalkylen stehen, X eine direkte Bindung, Methylen oder unsubstituiertes oder substituiertes Phenylen darstellt, $R_5$ Hydroxy, Amino oder substituiertes Amino der Formel $-N(R_6)\text{-}CH(R_7)\text{-}R_8$ bedeutet, in der $R_6$ für Wasserstoff steht oder, sofern $R_3$ Wasserstoff ist, auch Niederalkyl oder unsubstituiertes oder substituiertes Phenylniederalkyl bedeuten kann, $R_7$ Wasserstoff, Niederalkyl, unsubstituiertes oder substituiertes Phenyl oder freies oder verestertes Carboxy darstellt und $R_8$ unsubstituiertes oder substituiertes Phenyl bedeutet, und ihre pharmazeutisch verwendbaren Salze haben atmungserleichternde Eigenschaften und eignen sich zur Bekämpfung von Atmungsstörungen, vorzugsweise als Wirkstoff in und/oder zur Herstellung von atmungserleichternden pharmazeutischen Präparaten.

EP 0 058 373 A1

CIBA-GEIGY AG
Basel (Schweiz)

4-13286/1+2

# Substituierte aliphatische Sekundäramine und ihre Salze, diese enthaltende pharmazeutische Präparate und ihre Verwendung

Die Erfindung betrifft Verbindungen der Formel I

$$R_1-CH(R_2)-N(R_3)-R_4 \qquad (I)$$

worin $R_1$ unsubstituiertes oder substituiertes Phenyl bedeutet, $R_2$ Wasserstoff, Niederalkyl, unsubstituiertes oder substituiertes Phenyl oder freies oder verestertes Carboxy darstellt, und worin $R_3$ Wasserstoff oder Niederalkyl und $R_4$ eine Gruppe der Formel $-alk_1-X-alk_2-R_5$ bedeutet oder $R_3$ und $R_4$ gemeinsam eine Gruppe der Formel $-alk_3-NH-alk_4-$ darstellen, wobei $alk_1$ und $alk_2$ Niederalkyliden bedeuten, $alk_3$ und $alk_4$ für Niederalkylen stehen, X eine direkte Bindung, Methylen oder unsubstituiertes oder substituiertes Phenylen darstellt, $R_5$ Hydroxy, Amino oder substituiertes Amino der Formel $-N(R_6)-CH(R_7)-R_8$ bedeutet, in der $R_6$ für Wasserstoff steht oder, sofern $R_3$ Wasserstoff ist, auch Niederalkyl oder unsubstituiertes oder substituiertes Phenylniederalkyl bedeuten kann, $R_7$ Wasserstoff, Niederalkyl, unsubstituiertes oder substituiertes Phenyl oder freies oder verestertes Carboxy darstellt und $R_8$ unsubstituiertes oder substituiertes Phenyl bedeutet.

Die Erfindung betrifft ebenso Verbindungen der Formel I, worin $R_1$, $R_2$, $R_3$, $R_4$, $R_5$, $R_6$, $R_7$, $R_8$, $alk_1$, $alk_2$, X, $alk_3$ und $alk_4$ obige Bedeutungen haben, mit der Massgabe, dass in Verbindungen, worin $R_3$ Wasserstoff oder Niederalkyl, $R_4$ eine Gruppe $-alk_1-X-alk_2-R_5$ und $R_5$ eine Gruppe $-N(R_6)-CH(R_7)-R_8$ bedeutet, mindestens einer der Reste $R_1$ und $R_8$ von unsubstituiertem oder N-substituiertem Aminoalkoxyphenyl oder mindestens einer der Reste $R_2$, $R_3$, $R_6$ und $R_7$ von Wasserstoff und Niederalkyl verschieden ist, wenn X unsubstituiertes oder substituiertes Phenylen bedeutet, und mindestens einer der Reste $R_1$ und $R_8$ von unsubstituiertem oder durch Methoxy und/oder Chlor mono- oder disubstituiertes Phenyl oder mindestens einer der Reste $R_2$, $R_3$, $R_6$ und $R_7$ von Wasserstoff verschieden ist, wenn $alk_1$ und $alk_2$ für Methylen stehen und X Methylen oder eine direkte Bindung bedeutet, und mit der weiteren Massgabe, dass in Verbindungen, worin $R_3$ und $R_4$ gemeinsam eine Gruppe $-alk_3-NH-alk_4-$ darstellen und $alk_3$ Aethylen ist, $R_1$ von Phenyl oder $R_2$ von Phenyl und Wasserstoff und ferner $R_1$ von 2,3,4-Trimethoxyphenyl, o-Amino-dichlor-phenyl, o-Acylamino-dichlor-phenyl und o-Diacylamino-dichlor-phenyl oder $R_2$ von Wasserstoff verschieden ist, wenn $alk_4$ Aethylen bedeutet, und $R_1$ von Phenyl oder $R_2$ von Wasserstoff verschieden ist, wenn $alk_4$ 1,3-Propylen ist, wobei im Falle, dass $R_4$ eine Gruppe $-alk_1-X-alk_2-R_5$, $alk_1$ und $alk_2$ Methylen und $R_5$ eine Gruppe der Formel $-N(R_6)-CH(R_7)-R_8$ darstellt, $R_1$ und $R_8$ keine gleichen aus unsubstituiertem oder in 3-, 4-, 5- und/oder 6-Stellung durch Isopropyl, Methoxy, Hydroxy, Chlor, Diäthylamino, Dimethylamino und/oder Nitro mono-, di-, tri- oder tetra substituierten Phenylresten ausgewählte Substituenten bedeuten, wenn $R_2$, $R_3$, $R_6$ und $R_7$ Wasserstoff bedeuten und X für Methylen steht, und $R_1$ und $R_8$ keine gleichen aus p-Aminophenoxy- und p-Nitrophenoxyphenyl ausgewählte Reste bedeuten, wenn $R_2$ und $R_7$ Wasserstoff und $R_3$ und $R_6$ Wasserstoff oder Niederalkyl bedeuten und X für Methylen oder eine direkte Bindung steht, und ihre pharmazeutisch verwendbaren Salze, mit der Massgabe, dass Säureadditionssalze mit organischen Carbonsäuren im Anion keinen β-Lactamring aufweisen, zur Anwendung in einem Verfahren zur therapeutischen Behandlung des menschlichen oder tierischen Körpers, sie enthaltende pharmazeutische Präparate und ihre Verwendung zur

Bekämpfung von Krankheiten, bzw. als Wirkstoff in oder zur Herstellung von pharmazeutischen Präparaten.

Die Erfindung betrifft gleichermassen Verbindungen der Formel I, worin $R_1$ und $R_2$ obige Bedeutungen haben, $R_3$ Wasserstoff oder Niederalkyl ist und $R_4$ eine Gruppe der Formel $-alk_1-X-alk_2-N(R_6)-CH(R_7)-R_8$ darstellt, worin $alk_1$, X, $alk_2$, $R_6$, $R_7$ und $R_8$ obige Bedeutungen haben, mit der Massgabe, dass in Verbindungen der Formel I, worin X unsubstituiertes oder substituiertes Phenylen darstellt, mindestens einer der Reste $R_1$ und $R_8$ von unsubstituiertem oder N-substituiertem Aminoalkoxyphenyl verschieden ist, wenn $R_2$, $R_3$, $R_6$ und $R_7$ für Wasserstoff oder Niederalkyl stehen, dass ferner in Verbindungen der Formel I, worin $alk_1$ und $alk_2$ Methylen bedeuten und X Methylen oder eine direkte Bindung darstellen, mindestens einer der Reste $R_1$ und $R_8$ von unsubstituiertem oder durch Methoxy und/oder Chlor mono- oder disubstituiertem Phenyl und von p-Hydroxyphenyl verschieden ist, wenn $R_2$, $R_3$, $R_6$ und $R_7$ Wasserstoff darstellen, und dass weiterhin in Verbindungen der Formel I, worin $alk_1$ und $alk_2$ Methylen bedeuten und X eine direkte Bindung darstellt, mindestens einer der Reste $R_1$ und $R_8$ von Phenyl oder mindestens einer der Reste $R_3$ und $R_6$ von Wasserstoff verschieden ist, wenn beide Reste $R_2$ und $R_7$ jeweils Wasserstoff, Phenyl oder Methyl bedeuten, oder $R_2$ Aethyl und $R_7$ Wasserstoff ist, wobei $R_1$ und $R_8$ keine gleichen aus unsubstituiertem odet in 3-, 4-, 5- und/oder 6-Stellung durch Isopropyl, Methoxy, Hydroxy, Chlor, Diäthylamino, Dimethylamino und/oder Nitro substituierten Phenylresten ausgewählte Substituenten bedeuten, wenn $R_2$, $R_3$, $R_6$ und $R_7$ Wasserstoff bedeuten und X für Methylen steht, $R_1$ und $R_8$ keine gleichen aus p-Anilino, p-Hydroxyphenyl und p-Methylphenyl ausgewählten Substituenten darstellen, wenn $R_2$, $R_3$, $R_6$ und $R_7$ für Wasserstoff stehen und X eine direkte Bindung bedeutet, und $R_1$ und $R_8$ keine gleichen aus p-Aminophenoxy- und p-Nitrophenoxyphenyl ausgewählte Reste bedeuten, wenn $R_2$ und $R_7$ Wasserstoff und $R_3$ und $R_6$ Wasserstoff oder Niederalkyl bedeuten und X für Methylen oder eine direkte Bindung steht, und deren Salze sowie Verfahren zu ihrer Herstellung.

Phenylen ist vorzugsweise 1,3- oder 1,4-Phenylen, kann aber auch 1,2-Phenylen sein.

Als Substituenten von Phenyl und Phenyl in Phenylniederalkyl sowie von Phenylen kommen beispielsweise aliphatische und cycloaliphatische Reste, gegebenenfalls veräthertes oder verestertes Hydroxy, gegebenenfalls substituiertes Amino, Nitro und/oder Trifluormethyl in Betracht.

Verestertes Carboxy ist beispielsweise aliphatisch oder cycloaliphatisch verestertes Carboxy, wie gegebenenfalls substituiertes Niederalkoxycarbonyl oder Niederalkenyloxy- bzw. Niederalkinyloxycarbonyl oder 3- bis 8-gliedriges Cycloalkoxycarbonyl.

Aliphatische Reste sind beispielsweise gegebenenfalls durch Hydroxy, Niederalkoxy oder Niederalkanoyloxy substituierte aliphatische Kohlenwasserstoffreste, wie Niederalkyl, Niederalkenyl, Niederalkinyl, Mono- oder Dihydroxyniederalkyl, Niederalkoxyniederalkyl oder Niederalkanoyloxyniederalkyl.

Cycloaliphatische Reste sind beispielsweise 3- bis 8-gliedrige Cycloalkylreste.

Araliphatische Reste sind beispielsweise gegebenenfalls wie für Phenyl angegebene substituierte, vorzugsweise unsubstituierte oder durch Niederalkyl, Niederalkoxy, Halogen und/oder Trifluormethyl substituierte, Phenylniederalkylreste.

Gegebenenfalls veräthertes oder verestertes Hydroxy ist beispielsweise mit einem gegebenenfalls substituierten aliphatischen Alkohol veräthertes Hydroxy, wie Niederalkoxy, Niederalkenyloxy, Niederalkinyloxy, Mono- oder Dihydroxyniederalkoxy, Niederalkoxyniederalkoxy, Niederalkanoylniederalkoxy oder an benachbarte Kohlenstoffatome gebundenes Niederalkyl(id)endioxy, bzw. mit einer Halogenwasserstoffsäure oder einer organischen Carbonsäure verestertes Hydroxy, wie Halogen, Niederalkanoyloxy oder gegebenenfalls wie für Phenyl angegeben substituiertes, vorzugsweise unsubstituiertes oder durch Niederalkyl, Niederalkoxy, Halogen und/oder Trifluormethyl substituiertes, Benzoyloxy.

Gegebenenfalls substituiertes Amino ist beispielsweise gegebenenfalls aliphatisch substituiertes Amino, wie Amino, Mono- oder Diniederalkylamino oder Niederalkylenamino, oder Acylamino, wie Mono- oder Diniederalkanoylamino oder gegebenenfalls wie für Phenyl angegeben substituiertes, vorzugsweise unsubstituiertes oder durch Niederalkyl, Niederalkoxy, Halogen und/oder Trifluormethyl substituiertes, Benzoylamino.

Niederalkyl ist z.B. Methyl, Aethyl, n-Propyl, Isopropyl, n-Butyl, Isobutyl, sec.-Butyl, tert.-Butyl, ferner Pentyl, Hexyl oder Heptyl.

Niederalkenyl ist z.B. Vinyl, Allyl, Methallyl und Crotyl.

Phenylniederalkyl ist z.B. Benzyl, 2-Phenyläthyl, 2- oder 3-Phenylpropyl oder Triphenylmethyl.

3- bis 8-gliedriges Cycloalkyl ist z.B. Cyclopropyl, Cyclopentyl, Cyclohexyl oder Cycloheptyl.

Mono- und Dihydroxyniederalkyl ist z.B. 1- oder 2-Hydroxyäthyl, 1-, 2- oder 3-Hydroxypropyl, 1,2-Dihydroxyäthyl oder 1,2-Dihydroxypropyl.

Niederalkoxyniederalkyl ist z.B. Methoxyäthyl, 2-Aethoxyäthyl, 3-Methoxypropyl, 3-Aethoxypropyl oder 1,2-Dimethoxyäthyl.

Niederalkanoyloxyniederalkyl ist z.B. Acetyloxymethyl, 2-Acetyloxy-

äthyl oder Pivaloyloxymethyl.

Niederalkoxy ist z.B. Methoxy, Aethoxy, n-Propoxy, Isopropoxy, n-, sec.-, iso- oder tert.Butoxy, sowie Pentyloxy, Hexyloxy oder Heptyloxy.

Niederalkenyloxy ist z.B. Allyloxy oder Methallyloxy.

Niederalkinyloxy ist z.B. Propargyloxy.

Mono- oder Dihydroxyniederalkoxy ist z.B. Hydroxyäthoxy, 2- bzw. 3-Hydroxypropoxy, Hydroxyisopropoxy, 2,3-Dihydroxypropoxy oder 1,3-Dihydroxy-2-propoxy.

Niederalkoxyniederalkoxy ist z.B. Methoxymethoxy, 2-Methoxyäthoxy, 2-Aethoxyäthoxy oder 3-Methoxypropoxy.

Niederalkyl(id)endioxy ist z.B. Methylendioxy, Aethylendioxy, 1,3-Propylendioxy und Isopropylidendioxy.

Niederalkanoylniederalkoxy ist z.B. Acetylmethoxy, Acetyläthoxy oder Pivaloyläthoxy.

Niederalkanoyloxy ist z.B. Acetyl-, Propionyl, Butyryl-, Isobutyryl- oder Pivaloyloxy.

Niederalkoxycarbonyl ist z.B. Methoxycarbonyl, Aethoxycarbonyl, n-Propoxycarbonyl oder tert.-Butoxycarbonyl.

Niederalkenyloxycarbonyl ist z.B. Methallyl- oder Allyloxycarbonyl.

Niederalkinyloxycarbonyl ist z.B. Propargyloxycarbonyl. 3- bis 8-gliedriges Cycloalkoxycarbonyl ist z.B. Cyclopentoxycarbonyl, Cyclohexoxycarbonyl oder Cycloheptoxycarbonyl, ferner Cyclopropoxycarbonyl.

Mono- und Diniederalkylamino ist z.B. Methylamino, Aethylamino, n-Butylamino, Dimethylamino oder Diäthylamino.

Niederalkylenamino ist z.B. Pyrrolidino oder Piperidino.

Mono- oder Diniederalkanoylamino ist z.B. Formylamino, Acetylamino, Diacetylamino oder Succinimino.

Halogen ist z.B. Halogen mit Atomnummer bis und mit 35, wie Fluor, Chlor oder Brom.

Die Verbindungen der Formel I können Isomere bilden, beispielsweise in Form eines optischen Isomeren, z.B. je nach Anzahl der asymmetrischen Kohlenstoffatome als Enantiomere oder Diastereomere, oder als Gemische derselben, z.B. als Racemate oder Diastereomerengemische, vorliegen.

Salze von Verbindungen der Formel I sind beispielsweise deren pharmazeutisch verwendbare Säureadditionssalze, ferner pharmazeutisch verwendbare Salze von Verbindungen der Formel I, welche Carboxy und/oder phenolische Hydroxygruppen enthalten, mit Basen, ebenso innere Salze von Verbindungen der Formel I, die Carboxy aufweisen. Säureadditionssalze sind beispielsweise pharmazeutisch verwendbare Additionssalze mit anorganischen Säuren, wie Hydrohalogenide, z.B. Hydrochloride oder Hydrobromide, Hydrogensulfate, Phosphate, Borate und dergleichen, ebenso pharmazeutisch verwendbare Additionssalze mit organischen Säuren, mit der Massgabe, dass salzbildende organische Carbonsäuren keinen β-Lactamring aufweisen, insbesondere Säureadditionssalze mit offenkettigen oder carbocyclischen organischen Säuren, wie mit gegebenenfalls hydroxylierten aliphatischen Carbonsäuren, wie Niederalkanmono- oder -dicarbonsäuresalze, z.B. Acetate, Malonate oder Succinate, Niederalken-mono oder -dicarbonsäuresalze, z.B. Fumarate oder Maleinate, Oxo- oder Hydroxyniederalkandicarbonsäuresalze, wie Pyruvate, Tartrate,

Maleate und dergleichen, ferner Salze mit organischen Sulfonsäuren, wie aliphatische oder aromatische Sulfonsäuresalze, z.B. Methansulfonate, Benzolsulfonate, p-Toluolsulfonate und dergleichen, Sulfaminate, z.B. N-Cyclohexylsulfaminate, oder Salze mit gegebenenfalls 1-, 3- und/oder 8-substituierten 3,7-Dihydro-1H-purin-2,6-dionen, z.B. mit 8-Chlor-3,7-dihydro-1,3-dimethyl-1H-purin-2,6-dion. Pharmazeutisch verwendbare Basesalze sind beispielsweise Alkalimetall- oder Erdalkalimetallsalze, z.B. Natrium-, Kalium-, Magnesium- oder Calciumsalze, im Falle von Carboxy aufweisenden Verbindungen der Formel I ferner Ammoniumsalze mit Ammoniak oder organischen Aminen, wie Mono-, Di- und Triniederalkylaminen, z.B. Aethyl-, Diäthyl- oder Trimethylamin, oder Mono-, Di- und Tri-(hydroxyniederalkyl)-aminen, z.B. Aethanolamin, Di- oder Triäthanolamin.

Die Verbindungen der Formel I sind zum Teil bekannt und werden vorwiegend als Zwischenprodukte zur Herstellung von Feinchemikalien unterschiedlicher Verwendbarkeit vorgeschlagen.

Der Erfindung liegt die überraschende Feststellung zugrunde, dass die Verbindungen der Formel I und ihre pharmazeutisch verwendbaren Salze ausgeprägte atmungserleichternde Eigenschaften aufweisen. So verstärken sie die Atmungsleistung ohne die Atmungsfrequenz im allgemeinen signifikant zu erhöhen. Die Verbindungen der Formel I unterscheiden sich darin wesentlich von den sogenannten Atmungsstimulantien, wie N,N-Diäthylpyridin-3-carboxamid (Nikethamid) und der Kombination (Prethcamid) von N-Aethyl-N-(1-dimethylcarbamylpropyl)-crotonamid (Crotethamid) und N-Propyl-N-(1-dimethylcarbamylpropyl)-crotonamid (Cropropamid), deren Einfluss auf die Atmungsleistung vor allem in einer Erhöhung der Atmungsfrequenz besteht. Einzig von α-Phenyl-α-(2-piperidinyl)-essigsäuremethylester (Methylphenidat) ist eine ähnliche Wirkung bekannt. Diese Verbindung gehört jedoch, ebenso wie die obengenannten Atmungsstimulantien, einer gänzlich andersartigen Substanzklasse an. Sie weist zudem ausgeprägte, nicht in jedem Fall erwünschte,

zentralstimulierende Wirkungen und zudem unerwünschte Eigenschaften auf.

Die atmungserleichternden Eigenschaften der Verbindungen der Formel I und ihrer Salze können im Tierversuch nachgewiesen werden, zum Beispiel in der Versuchsanordnung nach Agents und Actions 3, 28-34 (1973).

Danach werden Kaninchen beiderlei Geschlechts von 2 bis 3 kg Körpergewicht mit Aethylurethan (1,2 g/kg s.c.) narkotisiert, in Rückenlage aufgebunden und trachealkanüliert. Die Atmungstätigkeit wird anhand des Pleuradruckes ($P_{pl}$) registriert. Alle 20 Minuten wird die Trachea verschlossen und für 30 Sekunden verschlossen gehalten. Der erste Trachealverschluss ergibt den individuellen Normalwert der Atmungsleistung. Jeweils 3 Minuten vor jedem nachfolgenden Trachealverschluss wird innerhalb von 60 Sekunden intravenös eine wässrige Lösung der Prüfsubstanz verabfolgt. Von Messung zu Messung wird die Dosis um den Faktor 3 erhöht, wobei man die Ausgangsdosis so wählt, dass mit etwa der 7. Applikation die letale Dosis ($D_l$) erreicht wird. Zur eigentlichen Wirksamkeitsermittlung werden über die Gesamtdauer des Trachealverschlusses für jeden Atemzug der mittlere pleurale Unterdruck während der In- und Exspiration einschliesslich der darauffolgenden Pause ($\bar{p}_i$), der maximal entwickelte pleurale Unterdruck während der Inspirationsanstrengung ($p_i^{max}$) und die Atmungsfrequenz (fr) gemessen. Alle drei Kenngrössen werden alsdann über die gesamte Trachealverschlusszeit gemittelt. Die verstärkende Wirkung der Prüfsubstanz auf die Atmungsleistung gibt sich in einer Erhöhung des mittleren pleuralen Unterdruckes ($\Delta\bar{p}_i$) und des maximalen pleuralen Unterdrucks ($\Delta p_i^{max}$) gegenüber dem als 100% festgesetzten Normalwert zu erkennen.

In diesem Modell ergaben sich die in der folgenden Tabelle zusammengestellten Werte für die erzielbare Erhöhung des mittleren bzw. maximalen pleuralen Unterdruckes ($\Delta\bar{p}_i$) bzw. ($\Delta p_i^{max}$) und die Aenderung der Atmungsfrequenz ($\Delta fr$) sowie für die minimale Dosis, bei der $\bar{p}_i$ signifikant zunimmt ($D_e$ in g/kg) und die letale Dosis ($D_l$ in g/kg):

| Prüfsubstanz | $\Delta\bar{p}_i$ | $\Delta p_i^{max}$ | $\Delta fr$ | $D_e$ | $D_l$ |
|---|---|---|---|---|---|
| I (s. Seite 23) [a] | 4 % | 14 % | + 5 % | 0,003 | über 0,01 |
| II(s. Seite 23) [a] | 10 % | 0 % | 0 % | 0,002 | über 0,04 |
| III(s. Seite 23): [a] | 17 % | 3 % | + 2 % | 0,0005 | 0,01 |
| IV (s. Seite 23) [a] | 20 % | 27 % | + 5 % | 0,0002 | 0,002 |
| V (s. Seite 23) [a] | 13 % | 21 % | + 2 % | 0,001 | 0,04 |
| VI (s. Seite 23) [a] | 7 % | 11 % | + 1 % | 0,002 | über 0,01 |
| VII (s. Seite 23) [a] | 13 % | 23 % | + 1 % | 0,003 | 0,1 |
| VIII(s.Seite 23) [a] | 13 % | 10 % | + 3 % | 0,001 | 0,03 |
| IX (s. Seite 23) [a] | 18 % | 16 % | + 3 % | 0,002 | 0,03 |
| X (s. Seite 24) [a] | 14 % | 28 % | - 9 % | 0,001 | 0,01 |
| XI (s. Seite 24) [a] | 11 % | 9 % | - 3 % | 0,005 | über 0,01 |
| XII(s. Seite 24) [a] | 9 % | 2 % | + 7 % | 0,001 | 0,03 |
| Methylphenidat [c] | 17 % | 9 % | - 1 % | 0,003 | 0,05 |
| Nikethamid | 19 % | 12 % | +84 % | 0,1 | 0,3 |
| Prethcamid | 5 % | 2 % | +15 % | 0,1 | über 0,1 |
| Strychnin | 2 % | 6 % | - 4 % | 0,0001 | 0,003 |

a= appliziert als Bismethansulfonat, b= appliziert als Methansulfonat, c= appliziert als Hydrochlorid

Die atmungserleichternde Wirkung der Verbindungen der Formel I wurde am Menschen in einer klinischen Vorprüfung mit einer ausgewählten Verbindung bestätigt. Beispielsweise wurden an 4 Probanden nach Verabfolgung von N,N'-Bis-(Diphenylmethyl)-1,2-propylendiamin (V, s. Seite 23) folgende Beobachtungen gemacht:

Proband 1 (männlich, Alter 73, Lungenemphysem) erhielt p.o. 10 mg Wirkstoff. 1 Stunde später betrug die Vitalkapazität sowie die Sekundenkapazität 3,3 l bzw. 2,7 l gegenüber 2,5 l bzw. 2,1 l als Ausgangswerten. Nebenwirkungen wurden nicht festgestellt.

Proband 2 (männlich, Alter 77, Lungenemphysem und Cor pulmonale) erhielt i.v. 10 mg Wirkstoff in Form einer wässrigen Lösung des Bismethansulfonates. 1 Stunde später betrug die Vitalkapazität sowie die Sekundenkapazität 2,5 l bzw. 2,4 l gegenüber 2,1 l bzw. 2,0 l als Ausgangswerten. Nebenwirkungen wurden nicht festgestellt.

Proband 3 (männlich, Alter 72, Lungenemphysem) mit dyspnoischem Befinden erhielt im Blindversuch p.o.Placebo sowie 10 mg Wirkstoff. 2 Stunden nach Pacebo betrug die Vitalkapazität sowie die Sekundenkapazität 2,1 l bzw. 1,3 l gegenüber 2,1 l bzw. 1,2 l als Ausgangswerten, das subjektive Befinden war unverändert. 2 Stunden nach Wirkstoff betrug die Vitalkapazität sowie die Sekundenkapazität 2,6 l bzw. 1,25 l gegenüber 2,2 l bzw. 1,2 l, der Proband gab " leichteres"Atmen an und fühlte sich leistungsfähiger.

Proband 4 (männlich, Alter 84, Lungenemphysem) mit dyspnoischem Befinden erhielt im Blindversuch 10 mg Wirkstoff sowie Placebo. 2 Stunden nach Wirkstoff betrug die Vitalkapazität sowie die Sekundenkapazität 1,85 l bzw. 1,0 l gegenüber 1,6 l bzw. 0,7 l, der Proband gab "leichteres, freieres" Atmen an. 2 Stunden nach Placebo betrug die Vitalkapazität sowie die Sekundenkapazität 1,65 l bzw. 0,75 l gegenüber 1,6 l bzw. 0,1 l; das subjektive Befinden war unverändert.

Die Verbindungen der Formel I sind dementsprechend auf Grund ihrer spezifischen die Atmungsleistung erhöhenden Wirkung als Arzneimittelwirkstoffe in atmungserleichternden pharmazeutischen Präparaten zur prophylaktischen und therapeutischen Beeinflussung von Atmungsstörungen verschiedenartigster Genese, insbesondere alterbedingter Atmungsstörungen, geeignet. Sie können aber auch zur gezielten Erhöhung der Atmungsleistung, beispielsweise bei Vorliegen ungünstiger Atmungsbedingungen, verwendet werden.

Die Erfindung betrifft in erster Linie Verbindungen der Formel I

$$R_1-CH(R_2)-N(R_3)-R_4 \qquad (I),$$

worin $R_1$ unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenyl bedeutet, $R_2$ Wasserstoff, Niederalkyl, unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenyl oder aliphatisch, cycloaliphatisch oder araliphatisch verestertes Carboxy darstellt, und worin $R_3$ Wasserstoff oder Niederalkyl und $R_4$ eine Gruppe der Formel $-alk_1-X-alk_2-R_5$ bedeutet oder $R_3$ und $R_4$ gemeinsam eine Gruppe der Formel $-alk_3-NH-alk_4-$ darstellen, wobei $alk_1$ und $alk_2$ Niederalkyliden bedeuten, $alk_3$ und $alk_4$ für Niederalkylen stehen, X eine direkte Bindung, Methylen oder unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenylen darstellt, $R_5$ Hydroxy, Amino oder substituiertes Amino der Formel $-N(R_6)-CH(R_7)-R_8$ bedeutet, in der $R_6$ für Wasserstoff steht oder, sofern $R_3$ Wasserstoff ist, auch Niederalkyl oder unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenylniederalkyl bedeuten kann,

$R_7$ Wasserstoff, Niederalkyl, unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenyl oder aliphatisch, cycloaliphatisch oder araliphatisch verestertes Carboxy darstellt, und $R_8$ unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenyl darstellt, mit der Massgabe,dass in Verbindungen,worin $R_3$ Wasserstoff oder Niederalkyl, $R_4$ eine Gruppe $-alk_1-X-alk_2-R_5$ und $R_5$ eine Gruppe $-N(R_6)-CH(R_7)-R_8$ bedeutet, mindestens einer der Reste $R_1$ und $R_8$ von unsubstituiertem oder N-substituiertem Aminoalkoxyphenyl oder mindestens einer der Reste $R_2$, $R_3$, $R_6$ und $R_7$ von Wasserstoff oder Niederalkyl verschieden ist, wenn X unsubstituiertes oder wie angegeben substituiertes Phenylen bedeutet, und mindestens einer der Reste $R_1$ und $R_8$ von unsubstituiertem oder durch Methoxy und/oder Chlor mono- oder disubstituiertes Phenyl oder mindestens einer der Reste $R_2$, $R_3$, $R_6$ und $R_7$ von Wasserstoff verschieden ist, wenn $alk_1$ und $alk_2$ für Methylen stehen und X Methylen oder eine direkte Bindung darstellt, und mit der weiteren Massgabe, dass in Verbindungen, worin $R_3$ und $R_4$ gemeinsam eine Gruppe $-alk_3-NH-alk_4-$ darstellen und $alk_3$ Aethylen ist, $R_1$ von Phenyl oder $R_2$ von Phenyl und Wasserstoff und ferner $R_1$ von 2,3,4-Trimethoxyphenyl, o-Amino-dichlor-phenyl, o-Acylamino-dichlor-phenyl und o-Diacylamino-dichlor-phenyl oder $R_2$ von Wasserstoff verschieden ist, wenn $alk_4$ Aethylen bedeutet, und $R_1$ von Phenyl oder $R_2$ von Wasserstoff verschieden ist, wenn $alk_4$ 1,3-Propylen ist, wobei im Falle dass $R_4$ eine Gruppe $-alk_1-X-alk_2-R_5$, $alk_1$ und $alk_2$ Methylen und $R_5$ eine Gruppe der Formel $-N(R_6)-CH(R_7)-R_8$ darstellt, $R_1$ und $R_8$ keine gleichen aus unsubstituiertem oder in 3-, 4-, 6- oder 6-Stellung durch Isopropyl, Methoxy, Hydroxy, Chlor, Diäthylamino und/oder Dimethylamino substituierten Phenylresten ausgewählte Substituenten bedeuten, wenn $R_2$, $R_3$, $R_6$ und $R_7$ Wasserstoff bedeuten und X für Methylen steht, und $R_1$ und $R_8$ keine gleichen aus p-Aminophenoxy- und p-Nitrophenoxyphenyl ausgewählte Reste bedeuten, wenn $R_2$ und $R_7$ Wasserstoff und $R_3$ und $R_6$ Wasserstoff oder Niederalkyl bedeuten und X für Methylen oder eine direkte Bindung steht, und ihre pharmazeutisch verwendbaren Salze, mit der Massgabe, dass Säureadditionssalze mit organischen Carbonsäuren im Anion keinen β-Lactamring aufweisen, zur Anwendung in einem Verfahren zur therapeutischen Behandlung des menschlichen oder tierischen Körpers, sie enthaltende pharmazeutische Präparate und ihre Verwendung zur Bekämpfung von Krankheiten, bzw. als Wirkstoff in oder zur Herstellung von pharmazeutischen Präparaten.

Die Erfindung betrifft in erster Linie gleichermassen Verbindungen der Formel I, worin $R_1$ und $R_2$ obige Bedeutungen haben, $R_3$ Wasserstoff oder Niederalkyl ist und $R_4$ eine Gruppe der Formel $-alk_1-X-alk_2-N(R_6)-CH(R_7)-R_8$ darstellt, worin $alk_1$, X, $alk_2$, $R_6$, $R_7$ und $R_8$ obige Bedeutungen haben, mit der Massgabe, dass in Verbindungen der Formel I, worin X unsubstituiertes oder wie angegeben substituiertes Phenylen ist, mindestens einer der Reste $R_1$ und $R_8$ von unsubstituiertem oder N-substituiertem Aminoalkoxyphenyl verschieden ist, wenn $R_2$, $R_3$, $R_6$ und $R_7$ Wasserstoff oder Niederalkyl bedeuten, dass ferner in Verbindungen, worin $alk_1$ und $alk_2$ Methylen bedeuten und X Methylen oder eine direkte Bindung darstellt, mindestens einer der Reste $R_1$ und $R_8$ von unsubstituiertem ider durch Methoxy und/oder Chlor mono- oder disubstituiertem Phenyl und von p-Hydroxyphenyl verschieden ist, wenn $R_2$, $R_3$, $R_6$ und $R_7$ Wasserstoff darstellen, und mit der weiteren Massgabe, dass in Verbindungen, worin $alk_1$ und $alk_2$ Methylen bedeuten und X eine direkte Bindung darstellt, mindestens einer der Reste $R_1$ und $R_8$ von Phenyl oder mindestens einer der Reste $R_3$ und $R_6$ von Wasserstoff verschieden ist, wenn beide Reste $R_2$ und $R_7$ jeweils Wasserstoff, Phenyl oder Methyl oder $R_2$ Aethyl und $R_7$ Wasserstoff bedeuten, wobei $R_1$ und $R_8$ keine gleichen aus unsubstituiertem oder in 3-, 4-, 5- und/oder 6-Stellung durch Isopropyl, Methoxy, Hydroxy, Chlor, Diäthylamino und/oder Dimethylamino oder in 4- und/oder 6-Stellung durch Chlor und/oder Nitro substituierten Phenylresten ausgewählte Substituenten bedeuten,

wenn $R_2$, $R_3$, $R_6$ und $R_7$ Wasserstoff bedeuten und X für Methylen steht, $R_1$ und $R_8$ keine gleichen aus p-Anilino, p-Hydroxyphenyl und p-Methylphenyl ausgewählten Substituenten darstellen, wenn $R_2$, $R_3$, $R_6$ und $R_7$ für Wasserstoff stehen und X eine direkte Bindung bedeutet, und $R_1$ und $R_8$ keine gleichen aus p-Aminophenoxy- und p-Nitrophenoxyphenyl ausgewählte Reste bedeuten, wenn $R_2$ und $R_7$ Wasserstoff und $R_3$ und $R_6$ Wasserstoff oder Niederalkyl bedeuten und X für Methylen oder eine direkte Bindung steht, und deren Salze, vorzugsweise pharmazeutisch verwendbare Säureadditionssalze, sowie Verfahren zu ihrer Herstellung.

Die Erfindung betrifft vor allem Verbindungen der Formel I, worin $R_1$ unsubstituiertes oder durch Niederalkyl, wie Methyl, Niederalkenyl, wie Vinyl oder Allyl, Niederalkinyl, wie Propargyl, Mono- oder Dihydroxyniederalkyl, wie Hydroxymethyl oder 2-Hydroxyäthyl, Niederalkoxyniederalkyl, wie Aethoxymethyl, Niederalkanoyloxyniederalkyl, wie Acetoxymethyl oder 2-Acetoxyäthyl, 3- bis 8-gliedriges Cycloalkyl, wie Cyclohexyl, Hydroxy, Niederalkoxy, wie Methoxy, Niederalkenyloxy, wie Allyloxy, Niederalkinyloxy, wie Propargyloxy, Mono- oder Dihydroxyniederalkoxy, wie 2-Hydroxyäthoxy, Niederalkoxyniederalkoxy, wie 2-Methoxyäthoxy, an benachbarte Kohlenstoffatome gebundenes Niederalkyl(id)endioxy, wie Aethylendioxy oder 2,2-Propylendioxy, Halogen der Atomnummer bis und mit 35, wie Chlor, Niederalkanoyloxy, wie Acetoxy, Amino, Mono- oder Diniederalkylamino, wie Methyl- oder Dimethylamino, und/oder Mono- oder Diniederalkanoylamino, wie Acetylamino oder Diacetylamino, substituiertes Phenyl bedeutet, $R_2$ Wasserstoff, Niederalkyl, unsubstituiertes oder wie für $R_1$ angegeben substituiertes Phenyl, Niederalkoxycarbonyl, wie Methoxy- oder Aethoxycarbonyl, oder 3- bis 8-gliedriges Cycloalkoxycarbonyl, wie Cyclohexyloxycarbonyl, darstellt, $R_3$ Wasserstoff, Niederalkyl oder Phenylniederalkyl wie Methyl, ist und $R_4$ eine Gruppe der Formel $-alk_1-X-alk_2-R_5$ bedeutet, oder $R_3$ und $R_4$ gemeinsam eine Gruppe der Formel $-alk_3-NH-alk_4-$ darstellen, wobei $alk_1$ und $alk_2$ Niederalkyliden, wie Methylen, bedeuten, $alk_3$ und $alk_4$ für Niederalkylen, wie Aethylen oder 1,3-Propylen, stehen, X

Methylen, eine direkte Bindung oder unsubstituiertes oder durch Niederalkyl, wie Methyl, Niederalkoxy, wie Methoxy, Halogen und/oder Trifluormethyl substituiertes Phenylen darstellt, $R_5$ Hydroxy, Amino oder substituiertes Amino der Formel $-N(R_6)-CH(R_7)-R_8$ bedeutet, $R_6$ für Wasserstoff steht oder, sofern $R_3$ Wasserstoff ist, auch Niederalkyl, wie Methyl bedeuten kann, $R_7$ Wasserstoff, Niederalkyl, wie Methyl, oder unsubstituiertes oder wie für $R_2$ angegeben substituiertes Phenyl, Niederalkoxycarbonyl, wie Methoxy oder Aethoxycarbonyl oder 3- bis 8-gliedriges Cycloalkoxycarbonyl, wie Cyclohexylcarbonyl, darstellt und $R_8$ unsubstituiertes oder wie für $R_1$ angegeben substituiertes Phenyl bedeutet, mit der Massgabe, dass in Verbindungen der Formel I, worin $R_3$ und $R_4$ gemeinsam eine Gruppe $-alk_3-NH-alk_4-$ darstellen und $alk_3$ Aethylen bedeutet, $R_1$ von Phenyl oder $R_2$ von Phenyl und Wasserstoff und ferner $R_1$ von 2,3,4-Trimethoxyphenyl, -Amino-dichlor-phenyl, o-Niederalkanoylamino-dichlor-phenyl und o-Diniederalkanoylamino-dichlor-phenyl oder $R_2$ von Wasserstoff verschieden ist, wenn $alk_4$ Aethylen bedeutet, und $R_1$ von Phenyl oder $R_2$ von Wasserstoff verschieden ist, wenn $alk_4$ 1,3-Propylen ist, und mit der weiteren Massgabe, dass in Verbindungen der Formel I, worin $R_4$ eine Gruppe $-alk_1-X-alk_2-R_5$ und $R_5$ eine Gruppe $-N((R_6)-CH(R_7)-R_8$ bedeutet, mindestens einer der Reste $R_1$ und $R_8$ von unsubstituiertem oder durch Methoxy und/oder Chlor mono- oder disubstituiertem Phenyl oder mindestens einer der Reste $R_2$, $R_3$, $R_6$ und $R_7$ von Wasserstoff verschieden ist, wenn $alk_1$ und $alk_2$ für Methylen stehen und X Methylen oder eine direkte Bindung darstellt, wobei im Falle, dass $R_4$ eine Gruppe $-alk_1-X-alk_2-R_5$, $alk_1$ und $alk_2$ Methylen und $R_5$ eine Gruppe der Formel $-N(R_6)-CH(R_7)-R_8$ darstellt, $R_1$ und $R_8$ keine gleichen aus unsubstituierten oder in 3-, 4-, 5- und/oder 6-Stellung durch Isopropyl, Methoxy, Hydroxy, Chlor, Diäthylamino und/oder Dimethylamino substituierten Phenylresten ausgewählte Substituenten bedeuten, wenn $R_2$, $R_3$, $R_6$ und $R_7$ Wasserstoff bedeuten und X für Methylen steht, oder ihrer pharmazeutisch verwendbaren Säureadditionssalze, mit der Massgabe, dass Additionssalze mit organischen Carbonsäuren keinen β-Lactamring aufweisen, zur Anwendung in einem Verfahren

zur therapeutischen Behandlung des menschlichen oder tierischen Körpers, ihre Verwendung zur Bekämpfung von Krankheiten bzw. zur Herstellung von Arzneimitteln sowie sie enthaltende pharmazeutische Präparate.

Die Erfindung betrifft gleichermassen vor allem Verbindungen der Formel I, worin $R_1$ und $R_2$ die vorstehenden Bedeutungen haben, $R_3$ Wasserstoff oder Niederalkyl, wie Methyl, ist und $R_4$ eine Gruppe der Formel $-alk_1-X-alk_2-N(R_6)-CH(R_7)-R_8$ darstellt, worin $alk_1$, X, $alk_2$, $R_6$, $R_7$ und $R_8$ vorstehende Bedeutungen haben, mit der Massgabe, dass in Verbindungen der Formel I, woirn $alk_1$ und $alk_2$ Methylen bedeuten und X Methylen oder eine direkte Bindung darstellt mindestens einer der Reste $R_1$ und $R_8$ von unsubstituiertem oder durch Methoxy und/oder Chlor mono- oder disubstituiertem Phenyl und von p-Hydroxyphenyl verschieden ist, wenn $R_2$, $R_3$, $R_6$ und $R_7$ Wasserstoff darstellen, und mit der weiteren Massgabe, dass in Verbindungen der Formel I, worin $alk_1$ und $alk_2$ Methylen bedeuten und X eine direkte Bindung darstellt, mindestens einer der Reste $R_1$ und $R_8$ von Phenyl oder mindestens einer der Reste $R_3$ und $R_6$ von Wasserstoff verschieden ist, wenn beide Reste $R_2$ und $R_7$ jeweils Wasserstoff, Methyl oder Phenyl bedeuten, oder $R_2$ Aethyl und $R_7$ Wasserstoff ist, wobei $R_1$ und $R_8$ keine gleichen aus unsubstituiertem oder in p-Stellung, den m-Stellungen oder einer o-Stellung durch Isopropyl, Methoxy, Hydroxy, Chlor, Diäthylamino und/oder Dimethylamino substituierten Phenylresten ausgewählte Substituenten bedeuten, wenn $R_2$, $R_3$, $R_6$ und $R_7$ Wasserstoff bedeuten und X für Methylen steht, und $R_1$ und $R_8$ keine gleichen aus p-Anilino, p-Hydroxyphenyl und p-Methylphenyl ausgewählten Substituenten darstellen, wenn $R_2$, $R_3$, $R_6$ und $R_7$ für Wasserstoff stehen und X eine direkte Bindung bedeutet, und deren Säureadditionssalze sowie Verfahren zu ihrer Herstellung.

Die Erfindung betrifft insbesondere Verbindungen der Formel I, worin $R_1$ unsubstituiertes oder durch Niederalkyl, wie Methyl, Niederalkenyl, wie Allyl, 3- bis 8-gliedriges Cycloalkyl, wie Cyclohexyl, Niederalkoxy, wie Methoxy, Niederalkenyloxy, wie Allyloxy, an benachbarte Kohlenstoffatome gebundenes Niederalkylendioxy, wie

Aethylendioxy oder 2,2-Propylendioxy, Halogen der Atomnummer bis und mit 35, wie Chlor, Amino und/oder Mono- oder Diniederalkylamino, wie Methyl- oder Diäthylamino, substituiertes Phenyl bedeutet, $R_2$ Wasserstoff oder unsubstituiertes oder wie für $R_1$ angegeben substituiertes Phenyl darstellt, $R_3$ Wasserstoff oder Niederalkyl, wie Methyl ist und $R_4$ eine Gruppe der Formel $-alk_1-X-alk_2-R_5$ bedeutet, oder $R_3$ und $R_4$ gemeinsam eine Gruppe der Formel $alk-_3-NH-alk_4-$ darstellen, wobei $alk_1$ und $alk_2$ Niederalkyliden, wie Methylen, bedeuten, $alk_3$ und $alk_4$ Niederalkyliden, wie Aethylen oder 1,3-Propylen, bedeuten, X eine direkte Bindung, Methylen oder unsubstituiertes Phenylen ist, $R_5$ eine Gruppe $-N(R_6)-CH(R_7)-R_8$ darstellt, $R_6$ für Wasserstoff steht oder, sofern $R_3$ Wasserstoff ist, auch Niederalkyl, wie Methyl, bedeuten kann, $R_7$ Wa-serstoff, Niederalkyl, wie Methyl, unsubstituiertes oder wie für $R_1$ angegeben substituiertes Phenyl, Niederalkoxycarbonyl, wie Methoxy- oder Aethoxycarbonyl, oder 3- bis 8-gliedriges Cycloalkoxycarbonyl, wie Cyclohexyloxycarbonyl, darstellt und $R_8$ unsubstituiertes oder wie für $R_1$ angegeben substituiertes Phenyl bedeutet, mit der Massgabe, dass in Verbindungen der Formel I, worin $R_3$ und $R_4$ gemeinsam eine Gruppe alk3-NH-$alk_4$ darstellen und $alk_3$ Aethylen bedeutet,$R_1$ von Phenyl oder $R_2$ von Phenyl und Wasserstoff und ferner $R_1$ von 2,3,4-Trimethoxyphenyl und o-Amino-dichlor-phenyl oder $R_2$ von Wasserstoff verschieden ist, wenn $alk_4$ Aethylen bedeutet, und dass $R_1$ von Phenyl oder $R_2$ von Wasserstoff verschieden ist, wenn $alk_4$ 1,3-Propylen ist, und mit der weiteren Massgabe, dass in Verbindungen der Formel I, worin $R_4$ eine Gruppe $-alk_1-X-alk_2-R_5$ und $R_5$ eine Gruppe $-N(R_6)-CH(R_8)-R_7$ bedeutet, mindestens einer der Reste $R_1$ und $R_8$ von unsubstituiertem oder durch Methoxy und/oder Chlor mono- oder disubstituiertem Phenyl oder mindestens einer der Reste $R_2$, $R_3$, $R_6$ und $R_7$ von Wasserstoff verschieden ist, wenn $alk_1$ und $alk_2$ für Methylen stehen, und X Methylen oder eine direkte Bindung darstellt, wobei im Falle dass $R_4$ eine Gruppe $-alk_1-X-alk_2-R_5$, $alk_1$ und $alk_2$ Methylen und $R_5$ eine Gruppe der Formel $-N(R_6)-CH(R_7)-R_8$ darstellt, $R_1$ und $R_8$ keine gleichen aus unsubstituiertem oder in 3-, 4-, 5- und/oder 6-Stellung

durch Isopropyl, Methoxy, Chlor, Diäthylamino und/oder Dimethylamino substituierten Phenylresten ausgewählte Substituenten bedeuten, wenn $R_2$, $R_3$, $R_6$ und $R_7$ Wasserstoff bedeuten und X für Methylen steht, oder ihre pharmazeutisch verwendbaren Säureadditionssalze, mit der Massgabe, dass Additionssalze mit organischen Carbonsäuren keinen β-Lactamring aufweisen, zur Anwendung in einem Verfahren zur therapeutischen Behandlung des menschlichen oder tierischen Körpers, ihre Verwendung zur Bekämpfung von Krankheiten bzw. zur Herstellung von Arzneimitteln sowie sie enthaltende pharmazeutische Präparate.

Die Erfindung betrifft gleichermassen insbesondere Verbindungen der Formel I, worin $R_1$ und $R_2$ vorstehende Bedeutungen haben, $R_3$ Wasserstoff oder Niederalkyl, wie Methyl, ist und $R_4$ eine Gruppe der Formel $-alk_1-X-alk_2-N(R_6)-CH(R_7)-R_8$ darstellt, worin $alk_1$, X, $alk_2$, $R_6$, $R_7$ und $R_8$ obige Bedeutungen haben, mit der Massgabe, dass in Verbindungen der Formel I, worin $alk_1$ und $alk_2$ Methylen bedeuten, und X Methylen oder eine direkte Bindung darstellt, mindestens einer der Reste $R_1$ und $R_8$ von unsubstituiertem oder durch Methoxy und/oder Chlor mono- oder disubstituiertem Phenyl verschieden ist, wenn $R_2$, $R_3$, $R_6$ und $R_7$ Wasserstoff darstellen, und mit der weiteren Massgabe, dass in Verbindungen der Formel I, worin $alk_1$ und $alk_2$ Methylen bedeuten und X eine direkte Bindung darstellt, mindestens einer der Reste $R_1$ und $R_8$ von Phenyl oder mindestens einer der Reste $R_3$ und $R_6$ von Wasserstoff verschieden ist, wenn beide Reste $R_2$ und $R_7$ jeweils Wasserstoff oder Phenyl bedeuten, wobei $R_1$ und $R_8$ keine gleichen aus unsubstituiertem oder in 3-, 4-, 5- und/oder 6-Stellung durch Isopropyl, Methoxy, Chlor, Diäthylamino und/oder Dimethylamino substituierten Phenylresten ausgewählte Substituenten bedeuten, wenn $R_2$, $R_3$, $R_6$ und $R_7$ Wasserstoff bedeuten und X für Methylen steht, und $R_2$ und $R_8$ keine gleichen aus p-Anilino und p-Methylphenyl ausgewählten Reste bedeuten, wenn $R_2$, $R_3$, $R_6$ und $R_7$ für Wasserstoff stehen und X eine direkte Bindung ist und deren Säureadditionssalze sowie Verfahren zu ihrer Herstellung.

Die Erfindung betrifft ganz besonders Verbindungen der Formel I, worin $R_1$ unsubstituiertes oder durch Niederalkyl mit bis und mit 4 Kohlenstoffatomen, wie Methyl und/oder Halogen der Atomnummer bis und mit 35, wie Chlor, substituiertes Phenyl bedeutet, $R_2$ Wasserstoff oder gegebenenfalls wie für $R_1$ angegeben substituiertes Phenyl bedeutet, $R_3$ Wasserstoff oder Niederalkyl mit bis und mit 4 Kohlenstoffatomen, wie Methyl, bedeutet und $R_4$ eine Gruppe der Formel $-CH_2-X-CH_2-R_5$ ist oder $R_3$ und $R_4$ gemeinsam eine Gruppe der Formel $-alk_3-NH-CH_2CH_2-$ darstellen, worin X eine direkte Bindung, Methylen oder Phenylen bedeutet, $R_5$ Amino oder substituiertes Amino der Formel $-N(R_6)-CH(R_7)-R_8$ ist, $R_6$ für Wasserstoff steht oder, sofern $R_3$ Wasserstoff ist, auch Niederalkyl mit bis und mit 4 Kohlenstoffatomen, wie Methyl, bedeuten kann, $R_7$ Wasserstoff, Niederalkyl mit bis und mit 4 Kohlenstoffatomen, wie Methyl, unsubstituiertes oder wie für $R_1$ angegeben substituiertes Phenyl oder Niederalkoxycarbonyl mit bis und mit 4 Kohlenstoffatomen im Alkylteil, wie Methoxycarbonyl, bedeutet, $R_8$ unsubstituiertes oder wie für $R_1$ angegeben substituiertes Phenyl darstellt und $alk_3$ Niederalkylen mit 2 bis 4 Kohlenstoffatomen, welches die beiden Stickstoffatome durch 2 oder 3 Kohlenstoffatome trennt, wie Methylen, Aethylen oder 1,3-Propylen, darstellt,
mit der Massgabe, dass in Verbindungen, worin $R_3$ Wasserstoff oder Niederalkyl mit bis und mit 4 Kohlenstoffatomen und $R_4$ eine Gruppe der Formel $-CH_2-X-CH_2-N(R_6)-CH(R_7)-R_8$ bedeutet, mindestens einer der Reste $R_1$ und $R_8$ von unsubstituiertem oder durch Chlor mono- oder disubstituiertem Phenyl oder mindestens einer der Reste $R_2$, $R_3$, $R_6$ und $R_7$ von Wasserstoff verschieden ist, wenn X für Methylen oder eine direkte Bindung steht, und mit der weiteren Massgabe, dass in Verbindungen, worin $R_3$ und $R_4$ gemeinsam eine Gruppe $-alk_3-NH-CH_2CH_2-$ bedeuten, $R_1$ von Phenyl oder $R_2$ von Phenyl und Wasserstoff verschieden ist, wenn $alk_3$ Aethylen ist, und $R_1$ von Phenyl oder $R_2$ von Wasserstoff verschieden ist, wenn $alk_4$ 1,3-Propylen ist, wobei $R_1$ und $R_8$ keine gleichen aus unsubstituiertem oder in p-Stellung, den m-Stellungen oder einer o-Stellung durch Isopropyl oder Chlor substituiertem Phenyl aus-

BAD ORIGINAL

gewählte Substituenten bedeuten, wenn $R_2$, $R_3$, $R_6$ und $R_7$ Wasserstoff bedeuten und X Methylen darstellt, oder ihre pharmazeutisch verwendbaren Säureadditionssalze, mit der Massgabe, dass Additionssalze mit organischen Carbonsäuren keinen β-Lactamring aufweisen, zur Anwendung in einem Verfahren zur therapeutischen Behandlung des menschlischen oder tierischen Körpers, ihre Verwendung zur Bekämpfung von Krankheiten bzw. zur Herstellung von Arzneimitteln sowie sie enthaltende pharmazeutische Präparate.

Die Erfindung betrifft gleichermassen ganz besonders Verbindungen der Formel I, worin $R_1$ und $R_2$ vorstehende Bedeutungen haben, $R_3$ Wasserstoff oder Niederalkyl mit bis und mit 4 Kohlenstoffatomen, wie Methyl, bedeutet und $R_4$ eine Gruppe der Formel $-CH_2-X-CH_2-N(R_6)-CH(R_7)-R_8$ darstellt, wobei X, $R_6$, $R_7$ und $R_8$ vorstehende Bedeutungen haben, mit der Massgabe, dass mindestens einer der Reste $R_1$ und $R_8$ von unsubstituiertem oder durch Chlor mono- oder disubstituiertem Phenyl oder mindestens einer der Reste $R_2$, $R_3$, $R_6$ und $R_7$ von Wasserstoff verschieden ist, wenn X für Methylen oder eine direkte Bindung steht, und mit der weiteren Massgabe, dass mindestens einer der Reste $R_1$ und $R_8$ von Phenyl oder mindestens einer der Reste $R_3$ und $R_6$ von Wasserstoff verschieden ist, wenn beide Reste $R_2$ und $R_7$ jeweils Wasserstoff oder Phenyl darstellen und X eine direkte Bindung bedeutet, und mindestens einer der Reste $R_1$ und $R_8$ von p-Methylphenyl verschieden ist, wenn $R_2$, $R_3$, $R_6$ und $R_7$ Wasserstoff bedeuten und X Methylen ist, wobei $R_1$ und $R_8$ keine gleichen aus unsubstituiertem oder in p-Stellung, den m-Stellungen oder einer o-Stellung durch Isopropyl oder Chlor substituiertem Phenyl ausgewählte Substituenten bedeuten, wenn $R_2$, $R_3$, $R_6$ und $R_7$ Wasserstoff bedeuten und X Methylen darstellt, und ihre, insbesondere pharmazeutisch verwendbaren, Säureadditionssalze sowie Verfahren zu ihrer Herstellung.

Die Erfindung betrifft namentlich das

N,N'-Bis-(Diphenylmethyl)-1,3-propylendiamin (I),
N,N'-Bis-(Diphenylmethyl)-p-xylylen-diamin (II),
N-Diphenylmethyl-N'-(1-phenyläthyl)-äthylendiamin (III),
N-Diphenylmethyl-N'-(α-methoxycarbonylbenzyl)-äthylendiamin (IV),
N,N'-Bis-(Diphenylmethyl)-1,2-propylendiamin (V),
N,N'-Bis-(Diphenylmethyl)-m-xylylen-diamin (VI),
N,N-Dibenzyl-N'-diphenylmethyl-äthylendiamin (VII)
N,N'-Di-(o-Methylbenzyl)-äthylendiamin (VIII) und das
N,N'-Dibenzyl-N-methyl-äthylendiamin (IX) sowie ihre Salze, vor allem ihre pharmazeutisch verwendbaren Säureadditionssalze, insbesondere zur Anwendung in einem Verfahren zur therapeutischen Behandlung des menschlichen oder tierischen Körpers, Verfahren zu ihrer Herstellung, ihre Verwendung als Arzneimittelwirkstoffe oder zur Herstellung von Arzneimitteln und sie enthaltende pharmazeutischen Präparate, ferner die Verwendung von

N,N'-Bis-(Diphenylmethyl)-äthylendiamin (X),
N-Diphenylmethyl-äthylendiamin (XI),
N,N'-Di-(p-methylbenzyl)-äthylendiamin (XII)
sowie ihrer pharmazeutisch verwendbaren Säureadditionssalze, mit der Massgabe, dass Additionssalze mit organischen Carbonsäuren im Anion keinen β-Lactamring aufweisen, zur Bekämpfung von Krankheiten, z.B. zur Anwendung in einem Verfahren zur therapeutischen Behandlung des menschlichen oder tierischen Körpers, z.B. als Arzneimittelwirkstoffe, insbesondere als Wirkstoffe in oder zur Herstellung atmungserleichternder Arzneimittel, sowie sie enthaltende pharmazeutische Präparate.

Die Verbindungen der Formel I können nach an sich bekannten Methoden hergestellt werden, beispielsweise indem man eine Verbindung der Formel

$Y_1$-alk$_1$-X-alk$_2$-N($R_6$)-CH($R_7$)-$R_8$ (II)

mit einer Verbindung der Formel

$R_1$-CH($R_2$)-$Y_2$ (III)

oder eine Verbindung der Formel

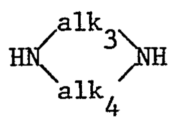

(II')

mit einer Verbindung der Formel

$R_1$-CH($R_2$)-Y (III')

kondensiert, wobei einer der Reste $Y_1$ und $Y_2$ eine Gruppe Y und der andere eine Gruppe der Formel -N($R_3$)-H darstellt und Y eine nukleophil ersetzbare Gruppe bedeutet, erforderlichenfalls ein verfahrensgemäss erhältliches Isomerengemisch in die Komponenten auftrennt und gewünschtenfalls eine verfahrensgemäss erhältliche Verbindung in eine andere Verbindung der Formel I und/oder eine verfahrensgemäss erhältliche Verbindung in ein Salz oder ein verfahrensgemäss erhältliches Salz in die freie Verbindung oder in ein anderes Salz überführt.

Nukleophil ersetzbare Gruppen Y sind beispielsweise reaktionsfähig veresterte Hydroxygruppen, wie mit einer anorganischen Säure oder mit einer organischen Sulfonsäure veresterte Hydroxygruppen, ferner Ammonium- und Sulfoniumgruppen. Mit einer anorganischen Säure verestertes Hydroxy ist beispielsweise mit einer Halogenwasserstoffsäure verestertes Hydroxy, z.B. Chlor, Brom oder Jod, ferner Fluorsulfonyloxy. Mit einer organischen Sulfonsäure verestertes Hydroxy ist beispielsweise aliphatisches Sulfonyloxy, wie Methan-, Aethan- und Aethensulfonyloxy, oder gegebenenfalls durch Niederalkyl, Halogen

und/oder Nitro substituiertes Benzolsulfonyloxy, z.B. Benzol-, p-Toluol- oder p-Brombenzolsulfonyloxy. Ammoniumgruppen sind vorzugsweise quaternäre Ammoniumgruppen, wie Triniederalkylammonium, z.B. Triäthylammonium, oder quaternäre Ammoniumgruppen heteroaromatischer Basen, z.B. Pyridinium. Sulfoniumgruppen sind beispielsweise Diniederalkylsulfoniumgruppen, z.B. Dimethylsulfonium.

Die Kondensation erfolgt in üblicher Weise, beispielsweise in Gegenwart eines inerten Lösungsmittels, erforderlichenfalls in Gegenwart eines Kondensationsmittels unter Kühlen oder Erwärmen und/oder unter Inertgas, wie Stickstoff. Als Kondensationsmittel kommen insbesondere basische Kondensationsmittel in Betracht, wie Hydroxide, Carbonate oder Hydrogencarbonate von Alkalimetallen und Erdalkalimetallen, z.B. Natrium-, Kalium- oder Calciumhydroxid, oder Natrium- oder Kaliumcarbonat, ferner tertiäre organische Stickstoffbasen, wie Triniederalkylamine, z.B. Triäthylamin, oder tertiäre Stickstoff-Heteroaromaten, z.B. Pyridin oder Chinolin, in Betracht. Die Kondensation wird vorzugsweise im Temperaturbereich von etwa 0 bis +120°C, vor allem bei etwa +10 bis +100°C, durchgeführt.

Die Ausgangsstoffe der Formel II können beispielsweise hergestellt werden, indem man eine Verbindung der Formel

$$Y_1\text{-alk}_1\text{-X-alk}_2\text{-}Y_1' \qquad \text{(IV)},$$

worin $Y_1$ eine Gruppe H-N($R_3$)- und $Y_1$' eine Gruppe -N($R_6$)-H bedeutet, und worin die Gruppe -N($R_6$)-H leichter phenalkylierbar ist als die Gruppe H-N($R_3$)-, oder ein Salz davon, mit einer Verbindung der Formel

$$Y_2'\text{-CH}(R_7)\text{-}R_8 \qquad \text{(V)}$$

kondensiert, worin $Y_2$' einen nukleophil ersetzbaren Rest, z.B. Halogen, darstellt, vorzugsweise in der vorstehend für die Kondensation von

Verbindungen der Formeln II und III angegebenen Weise. Man kann aber auch von einer Verbindung der Formel IV ausgehen, worin $Y_1$ und $Y_1'$ nukleophil ersetzbare Reste Y, z.B. Halogen bedeuten, $Y_1'$ aber reaktiver ist als $Y_1$, und diese mit einer Verbindung der Formel V, worin $Y_2'$ eine Gruppe $H-N(R_6)-$ darstellt und $R_6$ von Wasserstoff verschieden ist, umsetzen. In einer Verfahrensvariante, die sich besonders zur Herstellung von Verbindungen der Formel II, worin $Y_1$ eine Gruppe $H-N(R_3)-$ darstellt und $R_6$ gleich $R_3$ sowie $alk_2$ gleich $alk_1$ ist, eignet, setzt man einen Ueberschuss, z.B. die 5- bis 10-molare Menge, eines entsprechenden Diamins der Formel IV ($Y_1$=$Y_1'$= $-N(R_3)-H$) mit einem Halogenid der Formel V ($Y_2'$ = Halogen) um, und trennt den Aminüberschuss ab.

Die gemäss einer der vorstehenden Bildungsweisen erhältlichen Zwischenprodukte der Formel II können dabei ohne Isolierung mit der Reaktionskomponente der Formel III umgesetzt werden.

Eine andere Variante des vorstehenden Verfahrens der Bildung und Weiterumsetzung von Zwischenprodukten der Formel II, die sich besonders für die Herstellung von Verbindungen der Formel I eignet, worin $R_4$ eine Gruppe der Formel $-alk_1-X-alk_2-N(R_6)-CH(R_7)-R_8$ darstellt, und $R_7$ gleich $R_2$ und $R_8$ gleich $R_1$ ist, besteht darin, dass man eine Verbindung der Formel IV, worin $Y_1$ eine Gruppe $H-N(R_3)-$ und $Y_1'$ eine Gruppe $-N(R_6)-H$ ist, mit der doppelt molaren Menge einer Verbindung der Formel III, worin $Y_2$ einen nukleophil ersetzbaren Rest Y, z.B. Halogen bedeutet, oder zur Herstellung von Verbindungen der Formel I, worin zusätzlich $R_6$ gleich $R_3$ ist, eine Verbindung der Formel IV, worin $Y_1$ und $Y_1'$ je einen nukleophil ersetzbaren Rest, z.B. Halogen, bedeuten, mit der doppelt molekularen Menge einer Verbindung der Formel III, worin $Y_2$ eine Gruppe $H-N(R_3)-$ ist, umsetzt.

Diejenigen Verbindungen der Formel I, worin $R_4$ eine Gruppe der Formel $-alk_1-X-alk_2-N(R_6)-CH(R_7)-R_8$ bedeutet, können ferner hergestellt werden, indem man in einer Verbindung der Formel

$R_1-Y_3-X'-Y_4-R_8$ (VI),

worin $Y_3$ einen in eine Gruppe der Formel $-CH(R_2)-N(R_3)-alk_1-$ überführbaren Rest $Y'_3$ , X' eine Gruppe X oder einen in Methylen überführbaren Rest X" darstellt oder $Y_3$ eine Gruppe der Formel $-CH(R_2)-N(R_3)-alk_1-$ und X' einen in Methylen überführbaren Rest bedeutet, und worin $Y_4$ für eine Gruppe der Formel $-alk_2-N(R_6)-CH(R_7)-$ oder für einen in diese überführbaren Rest $Y'_4$ steht, oder in einem Salz davon $Y'_3$ in eine Gruppe der Formel $-CH(R_2)-N(R_3)-alk'_1-$, X" in Methylen und/oder $Y'_4$ in eine Gruppe der Formel $-alk_2-N(R_6)-CH(R_7)-$ überführt, erforderlichenfalls ein verfahrensgemäss erhältliches Isomerengemisch in die Komponenten auftrennt und gewünschtenfalls eine verfahrensgemäss erhältliche Verbindung in eine andere Verbindung der Formel I und/oder eine verfahrensgemäss erhältliche freie Verbindung in ein Salz oder ein verfahrensgemäss erhältliches Salz in die freie Verbindung oder in ein anderes Salz überführt.

Als Reste $Y'_3$ kommen beispielsweise solche der Formeln $-C(=Y_6)-N(R_3)-alk_1-$, $-CH(R_2)N(R_3)-C(=Y_6)-$, $-C(Y_5)(R_2)-N(R_3)-alk_1-$, $-CH(R_2)-N(R_3)-alk'_1-$, $C(R_2)=N-alk_1-$ und $-CH(R_2)-N=alk''-$, als Reste X" beispielsweise solche der Formeln $-CH(Y_5)-$ und $-C(=Y_6)-$ und als Reste $Y'_4$ solche der Formeln $-alk_2-N(R_6)-C(=Y_6)-$, $-C(=Y_6)-N(R_6)-CH(R_7)-$, $-alk_2-N(R_6)-C(Y_5)(R_7)-$, $-alk'_2-N(R_6)-CH(R_7)-$, $alk_2-N=C(R_7)-$ und $-alk''=N-CH(R_7)-$ in Betracht, worin $Y_5$ einen einwertigen bzw. $Y_6$ einen zweiwertigen durch Wasserstoff ersetzbaren Rest bedeutet, $alk'_1$ bzw. $alk'_2$ einen durch mindestens einen Rest $Y_5$ bzw. $Y_6$ substituiertes Niederalkyliden darstellt, und alk" Niederalkylidin bedeuten. Reste $Y_5$ sind beispielsweise gegebenenfalls funktionell abgewandelte Hydroxy- oder Mercaptogruppen, ferner organische Sulfonylgruppen sowie Carboxy. Reste $Y_6$ sind beispielsweise Oxogruppen oder über eine Doppelbindung gebundene funktionell abgewandelte Oxogruppen.

Funktionell abgewandelte Hydroxygruppen sind beispielsweise verätherte oder veresterte Hydroxygruppen, während als funktionell abgewandelte Mercaptogruppen insbesondere verätherte Mercaptogruppen in Betracht kommen. Verätherte Hydroxygruppen sind beispielsweise Niederalkoxygruppen, wie Methoxy oder Aethoxy. Veresterte Hydroxygruppen sind beispielsweise mit einer Mineralsäure oder organischen Carbon- oder Sulfonsäure veresterte Hydroxygruppen. Organische Carbonsäuren sind z.B. gegebenenfalls substituierte Benzoesäuren oder Niederalkancarbonsäuren, z.B. Benzoe- oder Essigsäure. Organische Sulfonsäuren sind z.B. Benzol-, p-Toluol-, p-Brombenzol-, Methan-, Aethan- oder Aethensulfonsäure. Mineralsäuren sind vorzugsweise Halogenwasserstoffsäuren, z.B. Chlor-, Brom- oder Jodwasserstoffsäure. Verätherte Mercaptogruppen sind beispielsweise niederalkylierte oder niederalkenylierte Mercaptogruppen, wie Methylthio, Aethylthio oder Aethylenthio.

Funktionell abgewandelte Oxogruppen sind beispielsweise Thionogruppen, Semicarbazonogruppen oder gegebenenfalls in β-Stellung durch organisches Sulfonyl, wie Benzol-, p-Toluol-, p-Brombenzol- oder Methansulfonyl, substituierte Hydrazonogruppen.

Die Ueberführung von $Y_3'$, X" und gegebenenfalls $Y_4'$ in die genannten Gruppen erfolgt in an sich bekannter Weise, beispielsweise durch Reduktion, d.h. Umsetzung mit einem geeigneten Reduktionsmittel. Als solche kommen beispielsweise in Betracht: Nascierender, beispielsweise durch Einwirkung einer Verbindung mit labilem Wasserstoff auf Metalle, z.B. einer Protonensäure, wie einer Halogenwasserstoffsäure oder Niederalkancarbonsäure, auf Eisen oder gegebenenfalls amalgamiertes Zink, Magnesium oder Aluminium, oder von Wasser auf, vorzugsweise amalgamiertes Aluminium, Magnesium oder Natrium, z.B. auf Natriumamalgam, erzeugter oder, beispielsweise durch einen Hydrierungskatalysator, wie einen Nickel- oder Edelmetallkatalysator, z.B. durch Raney-Nickel oder gegebenenfalls in chemisch oder an einen Träger gebundener Form, z.B. als Oxyd, vorliegendes Platin, wie durch Platin auf Kohle oder durch

Platinoxyd, oder durch homogene Edelmetallkatalysatoren, wie Triphenylphosphin-Platinchlorid oder Triphenylphosphin-Rhodiumchlorid, katalytisch erregter Wasserstoff, ferner niedrigwertige Uebergangsmetallverbindungen, wie Zinn-II- oder Chrom-II-salze, z.B. Zinn-II-chlorid, oder Hydride wie Calciumhydrid, der Borhydrid-Tetrahydrofurankomplex, oder der Komplex aus 9-Butyl-9-borabicyclo[3.3.1]nonan und Butyllithium, oder Dileichtmetallhydride, wie Lithiumaluminiumhydrid, gegebenenfalls im Gemisch mit Aluminiumchlorid, Natrium-bis-(2-methoxyäthoxy)-aluminiumhydrid oder Natrium-tris-(2-dimethylaminoäthoxy)-aluminiumhydrid, Natriumborhydrid, Lithiumtriäthylborhydrid oder Natriumcyanoborhydrid in Hexamethylphosphorsäuretriamid.

Die Umsetzung kann in aus der Literatur jeweils als geeignet bekannter Weise erfolgen.

So können gegebenenfalls veresterte oder verätherte, an ein benzylisches Kohlenstoffatom gebundene Hydroxygruppen $Y_5$ sowie ketonische Oxogruppen $Y_6$ aufweisende Gruppe $Y_3'$, X" und/oder $Y_4'$ insbesondere durch übliche Umsetzung mit, z.B. wie vorstehend angegeben, katalytisch erregtem Wasserstoff, beispielsweise mit Wasserstoff in Gegenwart von Palladium auf Kohle, erforderlichenfalls in einem inerten Lösungsmittel, wie einem Niederalkanol, einer Niederalkansäure oder einem aliphatischen Aether, z.B. in Aethanol, Essigsäure oder Dioxan, und/oder bei erhöhter Temperatur, reduziert werden.

Ketonische Oxogruppen, Sulfonyloxygruppen und/oder verätherte Mercaptogruppen aufweisende Reste $Y_3'$ und/oder $Y_4'$ können ferner durch übliche Umsetzung mit, z.B. wie vorstehend angegeben erzeugten, nascierendem Wasserstoff, beispielsweise nach der Verfahrensweise von Clemmensen, vorzugsweise mit Zink und Salzsäure, reduziert werden.

Halogen, gegebenenfalls veräthertes Hydroxy, mindestens eine C-N-Doppelbindung und/oder amidische Oxogruppen aufweisende Reste $Y_3'$, X" und/oder

$Y_4'$ kann man beispielsweise durch übliche Umsetzung mit einem geeigneten Dileichtmetallhydrid, wie einem der genannten, erforderlichenfalls in einem inerten Lösungsmittel und/oder bei erhöhter Temperatur, z.B. bei Siedetemperatur, ausgehend von Halogenverbindungen beispielsweise mit Natriumborhydrid in Wasser, Alkoholen, wie Aethanol, oder Aethylenglykolmonomethyläther, oder Aminen, wie Pyridin oder Triäthylamin, mit Lithiumtriäthylborhydrid, Natrium-bis-(2-methoxyäthoxy)-aluminiumhydrid in aromatischen oder araliphatischen Kohlenwasserstoffatomen, wie Benzol oder Toluol, oder mit Natrium-tris-(dimethylaminoäthoxy)-aluminiumhydrid, oder ausgehend von Lactamen oder Amiden beispielsweise mit Lithiumaluminiumhydrid in einem aliphatischen Aether, z.B. in Diäthyläther, Tetrahydrofuran oder Dioxan, erforderlichenfalls in der Siedehitze reduzieren.

Wie angegeben substituierte Hydrazonogruppen, z.B. β-(p-Toluolsulfonyl)-hydrazono, aufweisende Reste $Y_3'$, X" und/oder $Y_4'$ können insbesondere durch übliche Umsetzung mit einem Dileichtmetallhydrid, z.B. mit Natriumcyanoborhydrid in Hexamethylphosphorsäuretriamid, erforderlichenfalls bei erhöhter Temperatur, durch Wasserstoff ersetzt werden. Semicarbazono- oder unsubstituierte Hydrazonogruppen aufweisende Reste X", $Y_3'$ und/oder $Y_4'$ können beispielsweise durch übliche Umsetzung mit einer starken Base, beispielsweise nach der Verfahrensweise von Wolff-Kishner, mit einem Alkalialkoholat, z.B. mit Natriummethanolat, erforderlichenfalls unter erhöhtem Druck und/oder bei erhöhter Temperatur oder nach der Modifikation von Huang-Minlon mit einem Alkalimetallhydroxid, z.B. Kaliumhydroxid, in einem inerten, hochsiedenden Lösungsmittel, z.B. in Di- oder Triäthylenglykol oder Diäthylenglykolmonomethyläther, reduziert werden.

Azomethingruppen der Formeln $-C(R_2)=N-alk_1-$ bzw. $-CH(R_2)-N=alk''-$ und/oder $-alk_2-N=C(R_7)-$ bzw. $-alk''=N-CH(R_7)-$ können beispielsweise durch katalytisch erregten Wasserstoff wie mit Wasserstoff in Gegenwart von Platinoxid in Aethanol bei normalem oder leicht erhöhtem Druck,

z.B. bei etwa 0 bis 10 bar Ueberdruck, oder mit Wasserstoff in Gegenwart von Raney-Nickel bei erhöhtem Druck, z.B. bei etwa 20 bis 150 bar, vorzugsweise etwa 100 bar, Ueberdruck, durch Umsetzung mit Lithiumaluminiumhydrid, vorzugsweise in Tetrahydrofuran, oder mittels nascierenden Wasserstoffs bzw. durch metallische Reduktion, z.B. durch Behandeln mit Zink und Essigsäure, Eisen und Salzsäure und dergleichen, reduziert werden.

Ausgehend von Azomethinverbindungen der Formel VI, worin $Y_3'$ eine Gruppe der Formeln $-CH=N-alk_1-$ oder $-CH(R_2)-N=alk''-$ und/oder $Y_4'$ eine Gruppe der Formeln $-alk_2-N=CH-$ oder $-alk''=N-CH(R_2)-$ bedeutet, kommen als weitere Reduktionsmittel ferner Niederalkylmetallverbindungen, ausgehend von Verbindungen der Formel VI, worin $Y_3'$ eine Gruppe der Formel $-CH=N-alk_1-$ und/oder $Y_4'$ eine Gruppe der Formel $-alk_2-N=CH-$ bedeutet, ferner Phenylmetallverbindungen, die auch durch aliphatisch bzw. cycloaliphatische Reste, veräthertes Hydroxy, Diniederalkyl- oder Niederalkylenamino und/oder Trifluormethyl substituiert sein können, in Betracht. Unter "Metallverbindungen" sind dabei beispielsweise Alkalimetall-, wie Lithium- und Natriumverbindungen sowie Halogen-, wie Brom-, Chlor- oder Jodmagnesiumverbindungen zu verstehen. Die Umsetzung mit solchen Metallverbindungen erfolgt in üblicher Weise, beispielsweise in einem Diniederalkyl- oder Niederalkylenäther, z.B. in Diäthyläther, tert.-Butoxymethan, Dioxan oder Tetrahydrofuran, erforderlichenfalls unter Kühlen oder Erwärmen, z.B. bei etwa 0 bis 100°C, und/oder unter Inertgas, wie Stickstoff.

In einer bevorzugten Ausführungsform des vorstehenden Verfahrens geht man beispielsweise von einer Verbindung der Formel VI aus, worin $Y_3$ eine Gruppe $-C(=O)-N(R_3)-alk_1'-$ oder $-CH(R_2)-N(R_3)-C(=O)-$ und $Y_4$ eine Gruppe $-alk_2-N(R_6)-CH(R_7)-$, $-alk_2-N(R_6)-C(=O)-$ oder $-C(=O)-N(R_6)-CH(R_7)-$ bedeutet aus, und reduziert die Oxogruppe(n), z.B. durch Umsetzung mit einem geeigneten Dileichtmetallhydrid, z.B. mit Lithiumaluminiumhydrid in einem Aether, z.B. in Diäthyläther oder Tetrahydrofuran, erforderlichenfalls bei erhöhter Temperatur, z.B. in der Siedehitze.

In einer anderen bevorzugten Anführungsform des vorstehenden Verfahrens geht man beispielsweise von einer Verbindung der Formel VI aus, worin $Y_3$ eine Gruppe der Formel $-C(R_2)=N-alk_1-$ und $Y_4$ eine Gruppe der Formeln $-alk_2-N(R_6)-CH(R_7)-$ oder $-alk_2-N=C(R_7)-$ ist aus und reduziert die C-N-Doppelbindung(en), z.B. mittels Wasserstoff in Gegenwart von Platinoxid bei etwa 20 bis 30°C und etwa 0,5 bis 1,25 bar, oder durch Umsetzung mit Lithiumaluminiumhydrid in Tetrahydrofuran, vorzugsweise bei etwa 60°C bis Siedetemperatur.

Ein weiterer durch Wasserstoff ersetzbarer Rest $Y_5$ ist die gegebenenfalls in Salzform, z.B. als Alkalimetallsalz, wie Natriumsalz, Kupfersalzoder Ammoniumsalz, vorliegende Carboxygruppe. Der Ersatz derselben durch Wasserstoff erfolgt vorzugsweise durch Decarboxylierung, beispielsweise durch Erhitzen auf etwa 100 bis 250°C, erforderlichenfalls in einem hochsiedenden Lösungsmittel, z.B. in Aethylenglykol, Dimethylformamid, Aethylenglykolmonomethyläther oder Diphenyläther.

Weitere in Gruppen der Formel $-CH(R_2)-N(R_3)-alk_1-$ bzw. $-alk_2-N(R_6)-CH(R_7)-$, worin $R_2$ bzw. $R_6$ für Wasserstoff steht, überführbare Reste sind solche der Formel $-CH(R_2)N(Y_7)-alk_1-$ bzw. $-alk_2-N(Y_7)-CH(R_7)-$, worin $Y_7$ einen einwertigen durch Wasserstoff ersetzbaren Rest bedeutet. In Verbindungen der Formel VI, worin X' Methylen, eine direkte Bindung oder unsubstituiertes oder wie angegeben substituiertes o-Phenylen darstellt, kommen als weitere in die Gruppe der Formel $-CH(R_2)-NH-alk_1-X'-alk_2-NH-CH(R_7)-$ überführbare Reste solche der Formel

$$\begin{array}{c} alk_1-X'-alk_2 \\ | \qquad\qquad | \\ -CH(R_2)-N—Y_8—N-CH(R_7- \end{array} \qquad (VIa)$$

in Betracht, worin $Y_8$ einen zweiwertigen durch Wasserstoff ersetzbaren Rest bedeutet.

Einwertige durch Wasserstoff ersetzbare Reste $Y_7$ sind beispielsweise

Acylgruppen, Triarylmethylgruppen, 2-Acyl-niederalk-1-enylreste und Silylgruppen. Zweiwertige durch Wasserstoff ersetzbare Reste sind beispielsweise Carbonyl- bzw. Thiocarbonylgruppen.

Acylgruppen sind beispielsweise von einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder einem Kohlensäurehalbester abgeleitete Acylgruppen. Solche Acylgruppen sind beispielsweise Niederalkanoyl, wie Formyl, Acetyl, oder Propionyl, Halogenniederalkanoyl, wie 2-Halogenacetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung der Niederalkylrestes verzweigtes oder 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.-Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitrobenzyloxycarbonyl, oder substituiertes Diphenylmethoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichloräthoxycarbonyl, 2-Bromäthoxycarbonyl oder 2-Jodäthoxycarbonyl, oder 2-(trisubstituiertes Silyl)-äthoxycarbonyl, worin die Substituenten unabhängig voneinander je einen gegebenenfalls substituierten, z.B. durch Niederalkyl, Niederalkoxy, Aryl, Halogen oder Nitro, substituierten, aliphatischen, araliphatischen, cycloaliphatischen oder aromatischen Kohlenwasserstoffrest mit z.B. bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilyläthoxycarbonyl, wie 2-Trimethylsilyläthoxycarbonyl oder 2-(Di-n-butyl-methyl-silyl)-äthoxycarbonyl, oder 2-Triarylsilyläthoxycarbonyl, wie 2-Triphenylsilyläthoxycarbonyl.

Weitere Acylreste $Y_7$ sind auch entsprechende Reste organischer Phosphor-, Phosphon- oder Phosphinsäuren, wie Diniederalkylphosphoryl, z.B. Dimethylphosphoryl, Diäthylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-phenylniederalkylphosphoryl, z.B. Dibenzylphosphoryl oder Di-4-nitrobenzylphosphoryl, gegebenenfalls substituiertes Phenyloxyphenylphosphonyl, z.B. Phenyloxy-phenyl-phosphonyl, Diniederalkylphosphinyl, z.B. Diäthylphosphinyl, oder gegebenenfalls substituiertes Diphenylphosphinyl, z.B. Diphenylphosphinyl.

In einer Triarylmethylgruppe sind die Arylreste insbesondere gegebenenfalls substituierte Phenylreste. Eine solche Gruppe ist insbesondere Trityl.

In einem 2-Acyl-niederalk-1-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Aethoxycarbonyl-prop-1-en-2-yl.

Eine Silylgruppe ist in erster Linie eine organische Silylgruppe, worin das Silicium vorzugsweise Niederalkyl, insbesondere Methyl,

ferner Niederalkoxy, z.B. Methoxy, und/oder Halogen, z.B. Chlor, als Substituenten enthält. Entsprechende Silylgruppen sind in erster Linie Triniederalkylsilyl, insbesondere Trimethylsilyl, ferner Dimethyl-tert.-butyl-silyl, Niederalkoxy-niederalkyl-halogen-silyl, z.B. Methoxy-methyl-chlor-silyl, oder Diniederalkyl-halogensilyl, z.B. Dimethyl-chlor-silyl.

Bevorzugte Gruppen $Y_7$ sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichloräthoxycarbonyl, ferner Trityl oder Formyl.

Die Abspaltung der genannten Gruppen $Y_7$ bzw. $Y_8$ erfolgt in üblicher Weise, beispielsweise durch Solvolyse, insbesondere Hydrolyse, Alkoholyse oder Ammono- bzw. Aminolyse, oder durch Reduktion, insbesondere mittels chemischer Reduktionsmittel.

2-Halogen-niederalkoxycarbonyl (gegebenenfalls nach Umwandlung einer 2-Brom-niederalkoxycarbonylgruppe in eine 2-Jod-niederalkoxycarbonylgruppe), Aroylmethoxycarbonyl oder 4-Nitrobenzyloxycarbonyl kann z.B. durch Behandeln mit einem geeigneten chemischen Reduktionsmittel, wie Zink in Gegenwart einer geeigneten Carbonsäure, wie wässriger Essigsäure, abgespalten werden. Aroylmethoxycarbonyl kann auch durch Behandeln mit einem nucleophilen, vorzugsweise salzbildenden Reagens, wie Natriumthiophenolat, und 4-Nitro-benzyloxycarbonyl auch durch Behandeln mit einem Alkalimetall-, z.B. Natriumdithionit, gespalten werden. Gegenbenenfalls substituiertes Diphenylmethoxycarbonyl, tert.-Niederalkoxycarbonyl oder 2-trisubstituiertes Silyläthoxycarbonyl kann durch Behandeln mit einer geeigneten Säure, z.B. Ameisen- oder Trifluoressigsäure, gegebenenfalls substituiertes Benzyloxycarbonyl z.B. mittels Hydrogenolyse, d.h. durch milde Behandlung mit Wasserstoff in Gegenwart eines geeigneten

Hydrierkatalysators, wie eines Palladiumkatalysators, gegebenenfalls substituiertes Triarylmethyl, Formyl oder 2-Acyl-niederalk-1-enyl, z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine organische Silyl z.B. mittels Hydrolyse oder Alkoholye abgespalten werden. 2-Halogenacetyl, z.B. 2-Chloracetyl, kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. 2-Substituiertes Silyläthoxycarbonyl kann auch durch Behandeln mit einem Fluoridanionen liefernden Salz der Fluorwasserstoffsäure, z.B. mit Kaliumfluorid, abgespalten werden. Eine Phosphoro-, Phosphono- oder Phosphinogruppe kann z.B. durch Behandeln mit einer Phosphor-haltigen Säure, wie einer Phosphor-, Phosphon- oder Phosphinsäure, z.B. Orthophosphorsäure oder Polyphosphorsäure, einem sauren Ester, z.B. Monomethyl-, Monoäthyl-, Dimethyl- oder Diäthylphosphat oder Monomethylphosphonsäure, oder einem Anhydrid davon, wie Phosphorpentoxid, abgespalten werden.

Die Ausgangsstoffe können nach an sich bekannten Methoden hergestellt werden.

So erhält man Verbindungen der Formel VI, worin $Y'_3$ eine Gruppe der Formel $-C(=Y_6)-N(R_3)-alk_1-$ bedeutet, beispielsweise, indem man nach üblichen Methoden der Esteraminolyse, Aminoacylierung bzw. Umamidierung Verbindungen der Formeln

$$R_1-C(=Y_6)-Z \quad (VII) \quad und \quad H-N(R_3)-alk_1-X'-Y_4-R_8 \quad (VIII) \quad ,$$

worin Z veräthertes oder reaktionsfähiges verestertes Hydroxy oder eine quaternäre Ammoniumgruppe, z.B. Niederalkoxy, Halogen oder Pyridinium, bedeutet, miteinander kondensiert. Verbindungen der Formel

VIII, worin $Y_4$ eine Gruppe $-alk_2-N(R_6)-CH(R_7)-R_8$ bedeutet, können ihrerseits z.B. durch Kondensation von Verbindungen der Formeln

$Y_1-alk_1-X'-alk_2-Y_1'$ (IX) und $Y_2'-CH(R_7)-R_8$ (V),

worin $Y_1$ eine Gruppe $H-N(R_3)-$, $Y_1'$ einen nukleophil ersetzbaren Rest Y, z.B. Halogen und $Y_2'$ eine Gruppe $H-N(R_6)-$ darstellt, hergestellt werden, z.B. wie für die Umsetzung von Verbindungen der Formeln II und III angegeben.

Verbindungen der Formel VI, worin $Y_3'$ eine Gruppe $-C(=Y_6)-N(R_3)-alk_1-$ und $Y_4'$ eine Gruppe $-alk_2-N(R_6)-C(=Y_6)-$ bedeutet, und worin $R_7$ gleich $R_2$ sowie $R_8$ gleich $R_1$ ist, können z.B. hergestellt werden, indem man eine Verbindung der Formel IX, worin $Y_1$ eine Gruppe $H-N(R_3)-$ und $Y_1'$ eine Gruppe $-N(R_6)-H$ bedeutet, mit der doppeltmolaren Menge einer Verbindung der Formel V umsetzt, worin $Y_2'$ einen nukleophil ersetzbaren Rest Y, z.B. Halogen, darstellt.

Ausgehend von Verbindungen der Formel IX, worin $Y_1$ eine Gruppe $H-N(R_3)-$ und $Y_1'$ eine Gruppe $-N(R_6)-H$ bedeutet und worin diese beiden Gruppen unterschiedliche Acylierbarkeit aufweisen, kann man zunächst eine leichter acylierbare Gruppe $H-N(R_3)-$ durch Umsetzung mit einer Verbindung der Formel VII bzw. eine leichter acylierbare Gruppe $H-N(R_6)-$ durch Umsetzung mit einer Verbindung der Formel

$Z-(Y_6=)C-R_8$ (X)

acylieren und in der erhaltenen Verbindung der Formel IX, worin $Y_1$ eine Gruppe $R_1-C(=Y_6)-N(R_3)-$ und $Y_1'$ eine Gruppe $-N(R_6)-H$ bzw. $Y_1$ eine Gruppe $H-N(R_3)-$ und $Y_1'$ eine Gruppe $-N(R_6)-C(=Y_6)-R_8$ ist, die Gruppe $-N(R_6)-H$ bzw. $H-N(R_3)-$ durch Umsetzung mit einer Verbindung der Formel V, bzw.

$R_1-CH(R_2)-Y_2$ (III)

worin $Y_2'$ bzw. $Y_2$ ein nukleophil ersetzbarer Rest Y, z.B. Halogen ist,

phenalkylieren oder durch Umsetzung mit einer Verbindung der Formel X bzw. VII acylieren. Man kann aber auch zunächst die leichter phenalkylierbare Gruppe H-N($R_3$)- bzw. -N($R_6$)-H zunächst durch Umsetzung mit der Verbindung der Formel III bzw. V phenylalkylieren und das Primärprodukt dann durch Umsetzung mit der Verbindung der Formel X bzw. VII acylieren.

In analoger Weise erhält man auch Ausgangsstoffe der Formel VI, worin $Y_3'$ eine Gruppe -CH($R_2$)-N($R_3$)-C(=$Y_6$)- und $Y_4'$ eine Gruppe -$alk_2$-N($R_6$)-CH($R_7$)-, -C(=$Y_6$)-N($R_6$)-CH($R_7$)- oder -$alk_2$-N($R_6$)-C(=$Y_6$)-($R_7$)- bedeutet, indem man eine Verbindung der Formel

$$Z_1-C(=Y_6)-X'-C(=Y_6)-Z_2 \qquad (XI)$$

worin $Z_1$ und $Z_2$ unabhängig voneinander gegebenenfalls veräthertes oder reaktionsfähiges verestertes Hydroxy, wie Hydroxy, Niederalkoxy oder Halogen, bedeuten oder, sofern X' gegebenenfalls substituiertes o-Phenylen bedeutet und $Y_6$ Oxo ist, gemeinsam Oxy darstellen, zunächst mit einem Amin der Formel

$$R_1-CH(R_2)-Y_2 \qquad (III),$$

worin $Y_2$ -N($R_3$)-H ist, und anschliessend mit einem Amin der Formel

$$Y_2'-CH(R_7)-R_8 \qquad (V),$$

worin $Y_2'$ H-N($R_6$)- ist, oder eine Verbindung der Formel

$$Z_1-C(=Y_6)-alk_2-N(R_6)-H \qquad (XII)$$

zunächst mit einer Verbindung der Formel

$$R_1-CH(R_2)-Y_2 \qquad (III),$$

worin $Y_2$ $-N(R_3)-H$ ist, und anschliessend mit einer Verbindung der Formel

$$Y_2'-CH(R_7)-R_8 \quad (V),$$

worin $Y_2'$ einen nukleophil ersetzbaren Rest Y, z.B. Halogen bedeutet, oder mit einer Verbindung der Formel X umsetzt.

Verbindungen der Formel VI, worin $Y_3$ eine Gruppe $-C(OH)(R_2)-N(R_3)-alk_1-$ bzw. $-CH(R_2)-N(R_3)-alk_1'-$ ($alk_1'$=α-Hydroxyniederalkyliden) bedeutet, werden vorteilhaft <u>in situ</u> hergestellt, beispielsweise indem man eine Verbindung der Formel VIII unter reduzierenden Bedingungen, z.B. in Gegenwart von Ameisensäure oder eines ihrer Salze oder von, beispielsweise durch Platin oder Platinverbindungen, aktiviertem Wasserstoff, vorzugsweise in sauren Medien, z.B. in essigsaurer Lösung, mit einer Verbindung der Formel

$$(R_1)(R_2)C(=O) \quad (XIII)$$

oder eine Verbindung der Formel

$$R_1-CH(R_2)-Y_2 \quad (III),$$

ist, worin $Y_2$=$N(R_3)-H$ mit einer Verbindung der Formel

$$O=alk''-X'-Y_4-R_8 \quad (XIV)$$

umsetzt. Die dabei primär gebildete Verbindung der Formel VI wird dabei verfahrensgemäss direkt oder, sofern $R_3$ Wasserstoff ist über das Azomethin ($Y_3$=$-C(R_2)=N-alk_1-$ bzw. $-CH(R_2)-N=alk_1''-$) zu dem gewünschten Endprodukt reduziert.

In einer Abwandlung dieser Verfahrensvariante kann man zur Herstellung

von Verbindungen der Formel I, worin $R_7$ gleich $R_2$ und $R_8$ gleich $R_1$, vorteilhaft eine Verbindung der Formel

$Y_1$-$alk_1$-X-$alk_2$-$Y_2$ (IV),

worin $Y_1$ eine Gruppe

H-N($R_3$)- und $Y_1'$ eine Gruppe -N($R_6$)-H bedeutet, mit der doppeltmolaren Menge einer Verbindung der Formel XIII oder zur Herstellung von Verbindungen der Formel I, worin zusätzlich $R_6$ gleich $R_3$ ist, eine Verbindung der Formel

O=alk"-X-alk"=O (XV)

mit der doppeltmolaren Menge einer Verbindung der Formel

$R_1$-CH($R_2$)-$Y_2$ (III),

worin $Y_2$=H-N($R_3$) ist, umsetzen. Dabei entsteht intermediär ein Zwischenprodukt der Formel VI, worin $Y_3$ eine Gruppe -C(OH)($R_2$)-N($R_3$)-$alk_1$- oder, sofern $R_3$=H, eine Gruppe -C($R_2$)=N-$alk_1$- und $Y_4$ eine Gruppe -$alk_2$-N($R_3$)-C(OH)$R_7$)- oder, sofern $R_6$=H, eine Gruppe -$alk_2$-N=C($R_7$)- bedeutet, bzw. worin $Y_3$ eine Gruppe -CH($R_2$)-N($R_3$)-$alk_1'$- oder, sofern $R_3$=H, eine Gruppe -CH($R_2$)-N=$alk_1''$- und $Y_4$ eine Gruppe -$alk_2'$-N($R_6$)-CH($R_7$)- oder, sofern $R_6$=H, eine Gruppe -$alk_2''$=N-CH($R_7$)- darstellt, dass dann verfahrensgemäss reduziert wird.

Verbindungen der Formel VI, worin $Y_3'$ eine Gruppe -C($R_2$)=N-$alk_1$- bzw. -CH($R_2$)-N=$alk_1''$- bedeutet, werden erhalten, indem man eine Verbindung der Formel

H-N($R_3$)-$alk_1$-X-$Y_4$-$R_8$ (VIII),

worin $R_3$ Wasserstoff ist, oder ein Salz davon in üblicher Weise, beispielsweise in Gegenwart eines sauren Katalysators, z.B. von p-Toluolsulfonsäure, mit einer Verbindung der Formel

$(R_1)(R_2)C(=O)$ (XIII)

oder eine Verbindung der Formel

$O{=}alk_1''{-}X{-}Y_4{-}R_8$ (XIV)

mit einer Verbindung der Formel

$R_1{-}CH(R_2){-}Y_2$ (III),

worin $Y_2$ eine Gruppe $-NH_2$ ist, oder einem Salz davon kondensiert. Verbindungen der Formel VIII, worin $Y_4$ eine Gruppe $-alk_2''{=}N{-}CH(R_7)-$ bedeutet, können z.B. erhalten werden, indem man eine Verbindung der Formel

$H_2N{-}alk_1{-}X{-}alk_2''{=}O$ (XVI)

mit einer Verbindung der Formel

$Y_2'{-}CH(R_7){-}R_8$ (V),

worin $Y_2'$ Amino ist, umsetzt. Verbindungen der Formel XIV, worin $Y_4$ eine Gruppe $-alk_2{-}N{=}C(R_7)-$ ist, können in analoger Weise durch Umsetzung einer Verbindung der Formel

$H_2N{-}alk_1{-}X{-}alk_2'{=}O$ (XVII)

mit einer Verbindung der Formel

$Y_2'{-}CH(R_7){-}R_8$ (V),

worin $Y_2'$ Amino ist, und anschliessend mit einer Verbindung der Formel XIII hergestellt werden. Nach einer dieser Bildungsweisen können Aromethine der Formel VI schrittweise aufgebaut werden. Zur Herstellung symmetrischer Aromethine der Formel VI, worin $R_7$ gleich

$R_2$ und $R_8$ gleich $R_1$ ist bringt jedoch die Variante, derzufolge man entweder eine Verbindung der Formel

$Y_1$-alk$_1$-X-alk$_2$-$Y_1'$ (IV),

worin $Y_1$ und $Y_2$ Amino bedeuten, oder ein Salz davon mit der doppeltmolaren Menge einer Verbindung der Formel

$(R_1)(R_2)C(=O)$ (XIII)

oder eine Verbindung der Formel

O=alk$_1''$-X-alk$_2''$=O (XV)

mit der doppeltmolaren Menge einer Verbindung der Formel

$R_1$-CH($R_2$)-$Y_2$ (III),

worin $Y_2$ Amino ist, oder ein Salz davon zur Umsetzung bringt, praktische Vorteile

Verbindungen der Formel VI, worin X' einen in Methylen überführbaren Rest X" der Formel -CH($Y_5$)- bzw. -C(=$Y_6$)- bedeutet, können z.B. hergestellt werden, indem man Verbindungen der Formeln

$R_1$-CH($R_2$)-$Y_2$ (III) und $Y_1$-alk$_1$-X"-alk$_2$-N($R_6$)-CH($R_7$)-$R_8$ (XIX)

worin einer der Reste $Y_1$ und $Y_2$ eine Gruppe H-N($R_3$) und der andere einen nukleophil ersetzbaren Rest, z.B. Halogen bedeutet, miteinander kondensiert, insbesondere wie für die Umsetzung von Verbindungen der Formeln II und III angegeben. Verbindungen der Formel XIX, worin $Y_1$ Halogen und X" eine Gruppe -C(=O)- ist, werden z.B. erhalten, indem man eine Verbindung der Formel

H-alk$_1$-C(=O)-alk$_2$-Y (XX)

worin Y einen nukleophil ersetzbaren Rest, z.B. Halogen bedeutet, mit

einer Verbindung der Formel

$Y_2'-CH(R_7)-R_8$ (V),

worin $Y_2'$ eine Gruppe $H-N(R_6)-$ bedeutet, umsetzt und das Reaktionsprodukt in α-Stellung der Gruppe $H-alk_1-$ halogeniert, z.B. durch Bromeinwirkung. Verbindungen der Formel VI, worin $alk_2$ Methylen und X" eine Gruppe -CH(OH)- ist, können erhalten werden, indem man eine Verbindung der Formel

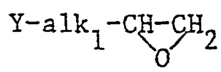 (XXI),

worin Y einen nukleophil ersetzbaren Rest, z.B. Halogen, bedeutet, zunächst mit einer Verbindung der Formel

$R_1-CH(R_2)-Y_2$ (III),

worin $Y_2$ eine Gruppe $-N(R_3)-H$ ist, und anschliessend mit einer Verbindung der Formel

$Y_2'-CH(R_7)-R_8$ (V),

worin $Y_2'$ eine Gruppe $H-N(R_6)-$ ist, umsetzt.

Zur Herstellung von Verbindungen der Formel VI, worin X' eine Gruppe X" bedeutet, und worin $R_7$ gleich $R_2$ und $R_8$ gleich $R_1$, ist es vorteilhaft, beide Bildungsschritte zusammenzufassen, indem man Verbindungen der Formeln

$R_1-CH(R_2)-Y_2$ (III) und $Y_1-alk_1-X''-alk_2-Y_1'$ (XXII)

worin $Y_2$ einen Rest Y, $Y_1$ eine Gruppe $H-N(R_3)-$ und $Y_1'$ eine Gruppe $-N(R_6)-H$ bedeutet oder zur Herstellung von Verbindungen, worin $R_6$ gleich $R_3$ ist, $Y_2$ eine Gruppe $-N(R_3)-H$ bedeutet und $Y_1$ sowie $Y_1'$ Reste Y darstellen, wobei Y für einen nukleophil ersetzbaren Rest, wie Halogen steht.

Ausgangsstoffe der Formel VI, worin $Y_3$ eine Gruppe der Formel $-CH(R_2)-N(R_3)-alk_1-$, X' eine Gruppe X und $Y_4$ eine Gruppe der Formel $-alk_2-N(Y_7)-CH(R_7)-$ darstellt, kann man beispielsweise herstellen, indem man eine Verbindung der Formel

$$Y_1-alk_1-X-alk_2-Y_1' \qquad (IV) \quad ,$$

worin $Y_1$ Hydroxy und $Y_1'$ einen nukleophil ersetzbaren Rest, z.B. Halogen, bedeutet, mit einer Verbindung der Formel

$$H-N(Y_7)-CH(R_7)-R_8 \qquad (XXIII)$$

umsetzt, in dem gebildeten Kondensationsprodukt die Hydroxygruppe $Y_1$ reaktionsfähig verestert, z.B. durch Umsetzung mit Thionylchlorid, Phosphortribromid oder einem organischen Sulfonylchlorid, wie p-Toluolsulfonylchlorid, und anschliessend mit einer Verbindung der Formel

$$R_1-CH(R_2)-Y_2 \qquad (III) \quad ,$$

worin $Y_2$ eine Gruppe $-N(R_3)-H$ ist, umsetzt. In einer Abwandlung dieses Verfahrens kann man aber auch von Verbindungen der Formel IV ausgehen, worin $Y_1$ und $Y_1'$ nukleophil ersetzbare Reste, z.B. Halogen, bedeuten, und worin $alk_1$ gleich $alk_2$ oder $Y_1'$ reaktiver als $Y_1$ ist.

Ausgangsstoffe der Formel VI, worin $Y_3-X'-Y_4$ eine Gruppe der Formel VIa bedeutet, können beispielsweise hergestellt werden, indem man eine Verbindung der Formel

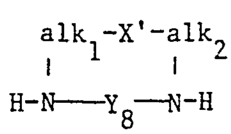

(XXIV) ,

worin $alk_1$ gleich $alk_2$ ist, zunächst mit einer Verbindung der Formel V und anschliessend mit einer Verbindung der Formel III, worin $Y_2'$ bzw. $Y_2$ einen nukleophil ersetzbaren Rest, z.B. Halogen darstellt.

Erfindungsgemäss erhältliche Verbindungen können in an sich bekannter Weise in andere Verbindungen der Formel I überführt werden, worin $R_4$ eine Gruppe der Formel $-alk_1-X-alk_2-H(R_6)-CH(R_7)-R_8$ darstellt.

So kann man beispielsweise in einer erfindungsgemäss erhältlichen Verbindung, worin $R_3$ und $R_6$ Wasserstoff bedeuten, $R_3$ durch Niederalkyl bzw. $R_6$ durch Niederalkyl oder gegebenenfalls substituiertes Phenylniederalkyl ersetzen, beispielsweise durch Umsetzung mit einem reaktionsfähigen Niederalkyl- oder Phenylniederalkylester. Reaktionsfähige Niederalkylester sind beispielsweise Niederalkylhalogenide, wie Niederalkylchloride, -bromide oder -iodide, Niederalkylsulfonate, z.B. Niederalkylniederalkansulfonate, wie -methansulfonate oder äthansulfonate, Niederalkylbenzolsulfonate, wie -benzolsulfonate, -p-toluolsulfonate oder -p-bromsulfonate, ferner Niederalkylfluorsulfonate, oder Diniederalkylsulfate. Die Umsetzung mit den genannten reaktionsfähigen Estern erfolgt beispielsweise in Gegenwart eines basischen Kondensationsmittels, erforderlichenfalls in einem inerten Lösungsmittel, unter Vakuum oder Erwärmen und/oder unter Inertgas, wie Stickstoff. Basische Kondensationsmittel sind beispielsweise Hydroxide oder Carbonate eines Alkali- oder Erdalkalimetalles, z.B. Natrium-, Kalium- oder Calciumhydroxid, Natrium- oder Kaliumcarbonat, oder tertiäre organische Stickstoffbasen, wie Triniederalkylamine, z.B. Triäthylamin oder Diisopropyl-methyl-amin, oder heteroaromatische Stickstoffbasen, z.B. Pyridin. Inerte Lösungsmittel sind beispielsweise Niederalkanole, wie Methanol, Butanol oder Amylalkohol, Diniederalkylketone, wie Aceton, Diniederalkyl- oder Niederalkylenäther, wie tert.-Butoxymethan, Dioxan oder Tetrahydrofuran, Niederalkansäureamide oder -lactame, wie Dimethylformamid oder N-Methylpyrrolidon, und Diniederalkylsulfoxide, z.B. Dimethylsulfoxid, ebenso Gemische derselben untereinander und/oder mit Wasser. Der Ersatz von Wasserstoff $R_3$ oder $R_6$ durch Niederalkyl bzw. von Wasserstoff $R_6$ durch Phenylalkyl kann aber auch durch Umsetzung mit einem Niederalkanal bzw. Phenylniederalkanal in Gegenwart eines Reduktionsmittels, z.B. von Ameisensäure oder einem ihrer Salze, erfolgen.

Ferner kann man beispielsweise eine freie Carboxylgruppe $R_2$ und/oder $R_7$ in üblicher Weise, z.B. durch Umsetzung mit dem entsprechenden Alkohol oder einem reaktionsfähigen Derivat, wie einem Carbon-, Phosphorig-,Schweflig- oder Kohlensäureester,z.B. einem Niederalkancarbonsäureester, Triniederalkylphosphit, Diniederalkylsulfit oder dem Pyrocarbonat des entsprechenden Alkohols, einem Diazoniederalkan, z.B. mit Diazomethan, einem Olefin, wie Niederalken, oder einem Amidacetal, wie einem Niederalkancarbonsäureamid-diniederalkylacetal, z.B. mit Dimethylformamid-diäthylacetal, in eine veresterte Carboxygruppe $R_2$ und/oder $R_7$ umwandeln.

Die Umsetzung mit dem entsprechenden Alkohol selbst erfolgt vorteilhaft in Gegenwart eines sauren Katalysators, wie einer Protonensäure, z.B. von Chlor- oder Bromwasserstoff-, Schwefel-, Phosphor-, Bor-, Benzolsulfon- und/oder Toluolsulfonsäure, oder eine Lewissäure, z.B. von Bortrifluorid-Aetherat, in einem inerten Lösungsmittel, insbesondere einem Ueberschuss des eingesetzten Alkohols und erforderlichenfalls in Gegenwart eines wasserbindenden Mittels und/oder unter destillativer, z.B. azeotroper, Entfernung des Reaktionswassers und/oder bei erhöhter Temperatur.

Die Umsetzung mit einem reaktionsfähigen Derivat des entsprechenden Alkohols kann in üblicher Weise durchgeführt werden, ausgehend von einem Carbon-, Phosphorig-, Schweflig- oder Kohlensäureester beispielsweise in Gegenwart eines sauren Katalysators, wie eines der vorstehend genannten, in einem inerten Lösungsmittel, wie einem aromatischen Kohlenwasserstoff, z.B. in Benzol oder Toluol, oder einem Ueberschuss des eingesetzten Alkoholderivates oder des entsprechenden Alkohols. Die Umsetzung mit Amidacetalen erfolgt vorteilhaft unter neutralen Bedingungen, beispielsweise in Acetonitril oder Dimethylformamid als Lösungsmittel.

Die Umsetzung mit einem Olefin kann beispielsweise in Gegenwart eines sauren Katalysators, z.B. einer Lewissäure, z.B. von Bortrifluorid, einer Sulfonsäure, z.B. von p-Toluolsulfonsäure, oder vor allem eines basischen Katalysators, z.B. von Natrium oder Kaliumhydroxid, vorteilhaft in einem inerten Lösungsmittel, wie einem Aether, z.B. in Diäthyläther oder Tetrahydrofuran, erfolgen.

Die vorstehend beschriebenen Umwandlungen freier in veresterte Carboxylgruppen können aber auch so durchgeführt werden, dass man eine Verbindung der Formel I, worin $R_2$ und/oder $R_7$ Carboxyl ist, zunächst in üblicher Weise in ein reaktionsfähiges Derivat, vorzugsweise ausgehend von einem Bis-Säureadditionssalz durch Umsetzung mit einem Halogenid des Phosphors oder Schwefels, z.B. mit Phosphortrichlorid oder -bromid, Phosphorpentachlorid oder Thionylchlorid, in ein Säurehalogenid, oder durch Umsetzung mit einem entsprechenden Alkohol oder Amin in einen reaktiven Ester, d.h. Ester mit elektronenanziehenden Strukturen, wie den Ester mit Phenol, Thiophenol, p-Nitrophenol, 2,4-Dinitrophenol oder Cyanmethylalkohol, oder ein reaktives Amid, z.B. das von Imidazol oder 3,5-Dimethylpyrazol abgeleitetes Amid, überführt und das erhaltene reaktionsfähige Derivat dann in üblicher Weise, vorzugsweise unter neutralen oder sauren Bedingungen, mit einem entsprechenden Alkohol umsetzt.

Eine veresterte Carboxylgruppe $R_2$ und/oder $R_7$ kann in üblicher Weise, z.B. durch Umsetzung mit einem Metallsalz, wie dem Natrium- oder Kaliumsalz, eines entsprechenden Alkohols oder mit diesem selbst in Gegenwart eines Katalysators, beispielsweise einer starken Base, z.B. von Natrium- oder Kaliumhydroxid, oder einer starken Säure, wie einer

Mineralsäure, z.B. von Salzsäure, Schwefelsäure oder Phosphorsäure, oder einer organischen Sulfonsäure, z.B. von p-Toluolsulfonsäure, oder einer Lewissäure, z.B. von Bortrifluorid-Aetherat, zu einer anderen veresterten Carboxylgruppe umgeestert werden.

Die neuen Verbindungen können, je nach der Wahl der Ausgangsstoffe und Arbeitsweisen, in Form eines der möglichen Isomeren oder als Gemisch derselben, z.B. je nach der Anzahl der asymmetrischen Kohlenstoffatome als reine optische Isomere, wie Antipoden oder als Isomerengemische, wie Racemate, Diastereoisomerengemische oder Racematgemische, vorliegen.

Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen, oder durch Umsetzung mit einer mit der racemischen Base Salze bildenden optisch aktiven Säure und Trennung der auf diese Weise erhaltenen Salze, z.B. auf Grund ihrer verschiedenen Löslichkeiten, in die Diasteromeren, aus denen die Antipoden durch Einwirkung geeigneter Mittel freigesetzt werden können, zerlegen. Vorteilhaft isoliert man den wirksameren der beiden Antipoden.

Erhaltene freie Verbindungen der Formel I, worin $R_4$ eine Gruppe der Formel $-alk_1-X-alk_2-N(R_6)-CH(R_7)-R_8$ darstellt, können in an sich bekannter Weise in Salze übergeführt werden, u.a. durch Behandeln mit einer Säure, oder, ausgehend von einer Säure ($R_2$ und/oder $R_7$=Carboxy), mit einer Base oder mit einem geeigneten Salz einer Carbonsäure, üblicherweise in Gegenwart eines Lösungs- oder Verdünnungsmittels.

Erhaltene Salze können in an sich bekannter Weise in die freien Verbindungen umgewandelt werden, z.B. durch Behandeln mit einem basischen bzw. sauren Reagens, wie einem Alkalimetallhydroxid bzw. einer Mineralsäure.

Die Verbindungen einschliesslich ihrer Salze können auch in Form ihrer Hydrate erhalten werden oder das zur Kristallisation verwendete Lösungsmittel einschliessen.

Infolge der engen Beziehung zwischen den neuen Verbindungen in freier Form und in Form ihrer Salze sind im vorausgegangenen und nachfolgend unter den freien Verbindungen oder ihren Salzen sinn- und zweckgemäss gegebenenfalls auch die entsprechenden Salze bzw. freien Verbindungen zu verstehen.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu ihrer Herstellung sind ebenfalls Gegenstand der vorliegenden Erfindung.

Pharmazeutische Präparate, welche eine der erfindungsgemässen Verbindungen der Formel I oder ein pharmazeutisch verwendbares Salz davon enthalten, sind beispielsweise solche, die zur enteralen, wie oralen oder rektalen, sowie parenteralen, wie intravenösen Verabreichung an und zur Inhalation durch Warmblüter, bestimmt sind und den pharmakologischen Wirkstoff allein oder zusammen mit einem pharmazeutisch anwendbaren Trägermaterial enthalten. Die Dosierung des Wirkstoffs hängt von der Warmblüter-Spezies, dem Alter und dem individuellen Zustand, sowie von der Applikationsweise, aber auch vom Molgewicht des Wirkstoffes ab. Für einen Warmblüter von etwa 75 kg Körpergewicht kommen beispielsweise Tagesdosen von etwa 1 bis etwa 25, vorzugsweise von etwa 4 bis etwa 20, insbesondere von etwa 5 bis etwa 15 mg (bezogen auf die freie Base) in Betracht, die erforderlichenfalls in mehrere, z.B. bis zu 4, vorzugsweise bis zu 3, Einzeldosen aufgeteilt werden können.

Die neuen pharmazeutischen Präparate enthalten z.B. von etwa 1 % bis etwa 50 %, vorzugsweise von etwa 4 % bis etwa 20 Gew.-% des Wirkstoffs. Erfindungsgemässe pharmazeutische Präparate sind z.B. solche in Dosiseinheitsformen, vorzugsweise in oraler Dosiseinheitsform, wie Dragées, Tabletten, Kapseln oder Suppositorien, ferner Ampullen, wobei Präparate in oraler Dosiseinheitsform jeweils beispielsweise etwa 2 bis etwa 25, vor allem etwa 5 bis 15 mg, Wirkstoff enthalten.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister z.B. Mais-, Weizen-, Reis- oder

Kartoffelstärkekleister, Gelatine, Tragakanth, Methylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten. Lacklösungen in geeigneten organischen Lösungsmitteln oder Lösungsmittelgemischen oder, zur Herstellung von Magensaft-resistenten Ueberzügen, Lösungen von geeigneten Cellulosepräparate, wie Acetylcellulosephthalat oder Hydroxypropylmethylcellulosephthalat, .verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit

einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässrige Injektionssuspensionen, welche viskositätserhöhende Stoffe, z.B. Natrium-carboxymethylcellulose, Sorbit und/oder Dextran und gegebenenfalls auch Stabilisatoren enthalten.

Die nachfolgenden Beispiele illustrieren die oben beschriebenene Erfindung; sie sollen jedoch diese in ihrem Umfang in keiner Weise einschränken. Temperaturen werden in Celsiusgraden, Drucke in mbar angegeben.

Beispiel 1: 180 g N,N'-Bis-(Diphenylmethylen)-1,2-propylendiamin werden in 540 ml Essigsäure gelöst. Man fügt 3 g Platinoxid hinzu, spült gründlich mit Argon und leitet dann unter kräftigem Rühren so lange

Wasserstoff ein, bis dünnschichtchromatographisch (Kieselgel 60, Diäthyläter: Rf = 0,62) kein Ausgangsmaterial mehr nachweisbar ist (meist 24 bis 48 Stunden). Die Reaktionslösung wird mit 150 ml Aethanol verdünnt, vom Katalysator abfiltriert, und unter vermindertem Druck im Wasserbad zur Trockene eingedampft. Der ölige Eindampfrückstand wird mit 375 ml 10%-iger Natronlauge verrieben und zweimal mit je 375 ml Diäthyläther ausgezogen. Die Aetherphasen werden vereinigt, zweimal mit je 70 ml gesättigter Kochsalzlösung gewaschen, über Magnesiumsulfat getrocknet und zunächst bei etwa 20 mbar, dann bei etwa 0,03 mbar im Wasserbad zur Trockene eingedampft. Der Rückstand wird in etwa 300 ml Aethanol gelöst, bis zur beginnenden Trübung mit Wasser versetzt (etwa 20 ml) und im Eisbad zur Kristallisation gestellt. Die ausgefallenen Kristalle werden abgesaugt, mit wenig Methanol gewaschen und bei etwa 0,03 mbar gründlich getrocknet. Man erhält das N,N'-Bis-(Diphenylmethyl)-1,2-propylendiamin vom Smp. 72-75°, welches durch Umkristallisieren aus etwa der doppelten Menge Methanol und Anfällen mit Wasser weitergereinigt werden kann und dann bei 75-77° schmilzt.

Das Ausgangsmaterial kann beispielsweise folgendermassen hergestellt werden:

Man gibt 255,1 g Benzophenon, 298 ml 1,2-Diaminopropan und 0,2 g p-Toluolsulfonsäure zusammen und erhitzt 30 Minuten zum Rückfluss. Dann destilliert man etwa 250 ml ab, lässt auf etwa 80° abkühlen, versetzt mit etwa 350 ml 2-Aethoxyäthanol (Aethylenglykolmonoäthyläther), gibt unter Rühren 70 ml Wasser hinzu und lässt im Eisbad kristallisieren. Man erhält N,N'-Bis-(Diphenylmethylen)-1,2-propylendiamin, das durch Umkristallisieren aus Aethanol (etwa 180 ml) gereinigt werden kann und vor der Weiterumsetzung gründlich getrocknet werden sollte.

In analoger Weise erhält man ausgehend von Benzophenon durch Umsetzung

mit m-Xylylendiamin über N,N'-Bis-(Diphenylmethylen)-m-xylylendiamin das N,N'-Bis-(Diphenylmethyl)-m-xylylendiamin Smp. 89-90° (aus Isopropanol), mit 1,3-Propylendiamin über N,N'-Bis-(Diphenylmethylen)-1,3-propylendiamin das N,N'-Bis-(Diphenylmethyl)-1,3-propylendiamin, Smp. 83-84° (aus Aceton), und mit p-Xylylendiamin über N,N'-Bis-(Diphenylmethylen)-p-xylylendiamin das N,N'-Bis-(Diphenylmethyl)-p-xylylendiamin, Smp. 92-94° (aus Diäthyläther).

Beispiel 2: Zu 240 ml Acetonitril fügt man unter Rühren nacheinander 20,2 g Triäthylamin, 7,4 g N-Methyläthylendiamin und 25,2 g Benzylchlorid hinzu, lässt etwa 48 Stunden am Rückfluss sieden und filtriert vom ausgefallenen Triäthylammoniumchlorid ab. Das Filtrat wird unter vermindertem Druck eingedampft. Der ölige Rückstand wird in 100 ml Diäthyläther aufgenommen und mit 50 ml 10%-iger Natronlauge ausgeschüttelt. Die wässrige Phase wird mit 100 ml Aether gewaschen. Die Aetherphasen werden vereinigt, zweimal mit je 50 ml gesättigter Natriumchloridlösung gewaschen, über Magnesiumsulfat getrocknet und schonend zur Trockene eingedampft. Der Eindampfungsrückstand wird unter vermindertem Druck (unter 0,1 mbar) destilliert. Man erhält das N,N'-Dibenzyl-N-methyl-äthylendiamin von $Kp_{0,028} = 123°$.

In analoger Weise kann man ferner herstellen:

durch Umsetzung von Aethylendiamin mit o-Methylbenzylchlorid das N,N'-Di-(o-Methylbenzyl)-äthylendiamin, Smp. 38-39°,

und durch Umsetzung von 1,2- bzw. 1,3-Propylendiamin bzw. m- bzw. p-Xylylendiamin mit Benzhydrylchlorid das N,N'-Bis-(Diphenylmethyl)-1,2-propylendiamin, Smp. 75-77° (aus Methanol/Wasser), das N,N'-Bis-(Diphenylmethyl)-1,3-propylendiamin, Smp. 83-84° (aus Aceton), das N,N'-Bis-(Diphenylmethyl)-m-xylylendiamin, Smp. 89-90° (aus Isopropanol), und das N,N'-Bis-(Diphenylmethyl)-p-xylylendiamin, Smp. 92-94° (aus Diäthyläther).

Beispiel 3: Zu einer Suspension von 2,0 g Lithiumaluminiumhydrid in 30 ml Tetrahydrofuran werden tropfenweise 5,3 g N,N'-Di-(o-Methylbenzyliden)-äthylendiamin, gelöst in 75 ml Tetrahydrofuran zugetropft. Man erhitzt 30 Minuten zum Rückfluss, zersetzt den Reduktionsmittelüberschuss unter Eiskühlung mit Wasser, giesst in 200 ml 10%-ige Natriumhydroxidlösung und schüttelt dreimal mit je 150 ml Diäthyläther aus. Durch Zugabe von in Diäthyläther gelöstem Chlorwasserstoff zu den vereinigten Aetherextrakten fällt man das Hydrochlorid der neuen Base in Form eines weissen Pulvers aus. Dieses wird abfiltriert und mit je 150 ml Diäthyläther und 10%-iger Natriumhydroxidlösung geschüttelt. Die Aetherphase wird über Magnesiumsulfat getrocknet und eingedampft. Der Eindampfrückstand wird aus Essigsäureäthylester umkristallisiert. Man erhält das N,N'-Di-(o-Methylbenzyl)-äthylendiamin vom Smp. 38-39°.

Das Ausgangsmaterial kann z.B. folgendermassen hergestellt werden:

Ein Gemisch von 24 g 2-Methyl-benzaldehyd, 200 ml Toluol und 5,8 g Aethylendiamin wird über Nacht gerührt. Dann werden unter vermindertem Druck etwa 125 ml abdestilliert. Das ausgefallene N,N'-Di-(o-Methylbenzyliden)-äthylendiamin wird abgesaugt. Es schmilzt bei 51-52°.

Beispiel 4: 6,15 g N,N'-Dibenzyl-N-methyl-äthylendiamin werden in 60 ml Diäthyläther gelöst. Dann leitet man unter Rühren und Kühlen (erforderlichenfalls im Aceton/Trockeneisbad) so lange Chlorwasserstoff ein, bis eine Probe, auf der Tonscherbe getrocknet, bei 227-229° schmilzt. Das ausgefallene Produkt wird abgesaugt, mit Aether gewaschen und unter vermindertem Druck (unter 0,1 mbar) bis zu Gewichtskonstanz getrocknet. Man erhält das N,N'-Dibenzyl-N-methyl-äthylendiamin-dihydrochlorid, Smp. 227-229°.

Beispiel 5: Zu einem Gemisch von 11,3 g N-Diphenylmethyläthylendiamin, 5,05 g Triäthylamin und 70 ml Acetonitril tropft man unter Rühren innerhalb von 10 Minuten eine Lösung von 9,2 g α-Chlorphenylessigsäuremethylester in 10 ml Acetonitril hinzu und erhitzt anschliessend 18 Stunden zum Rückfluss. Dann dampft man unter vermindertem Druck zur Trockne ein, verreibt mit 100 ml 10%-iger Natronlauge und schüttelt zweimal mit je 100 ml

Diäthyläther aus. Die Aetherphasen werden vereinigt, dreimal mit je 30 ml gesättigter Natriumchloridlösung gewaschen, über Magnesiumsulfat getrocknet und eingedampft.Der Eindampfrückstand wird erneut mit 10%-iger Natronlauge und Diäthyläther ausgeschüttelt, um Triäthylaminreste zu entfernen. Die Aetherphasen werden wie vorstehend angegeben weiterbehandelt. Zuletzt wird bei etwa 0,15 mbar getrocknet. Man erhält das N-(α-Methoxycarbonyl-benzyl)-N'-diphenylmethyl-äthylendiamin, welches zur Charakterisierung in das Diacetat überführt wird. Dazu werden 14,8 g Base in 15 ml Diäthyläther und 15 ml Hexan gelöst und unter Rühren und Kühlen tropfenweise mit Eisessig versetzt. Der Niederschlag wird abgesaugt, aus etwa 360 ml Diäthyläther und 90 ml Hexan umkristallisiert und unter vermindertem Druck (unter 0,2 mbar) getrocknet. Das N-(α-Methoxycarbonyl-benzyl)-N'-diphenylmethyl-äthylendiamin-diacetat schmilzt bei 78-81°. Aus diesem kann reines N-(α-Methoxycarbonylbenzyl)-N'-diphenylmethyl-äthylendiamin durch Behandeln mit 10%-iger Natronlauge, Ausschütteln mit Diäthyläther, Trocknen über Magnesiumsulfat und Abdampfen des Lösungsmittels freigesetzt werden.

Das als Ausgangsmaterial verwendete N-Diphenylmethyläthylendiamin kann z.B. folgendermassen hergestellt werden:

Ein Gemisch von 90,15 g Aethylendiamin, 50,7 g Benzhydrylchlorid und 300 ml Acetonitril wird unter Rühren 24 Stunden zum Rückfluss erhitzt. Man verteilt zwischen 400 ml Diäthyläther und 175 ml 10%-iger Natronlauge, trennt die Aetherphase ab, trocknet über Magnesiumsulfat, dampft zur Trockene ein und destilliert unter vermindertem Druck. Man erhält das N-Diphenylmethyläthylendiamin von $Kp_{0,13}$ = 116-124°, Smp. 23-24°.

Der als Ausgangsmaterial verwendete α-Chlorphenylessigsäuremethylester kann z.B. folgendermassen hergestellt werden:

94,5 g α-Chlorphenylacetylchlorid werden unter Rühren tropfenweise mit 16 g Methanol versetzt. Man gibt 5 Tropfen konzentrierte Schwefelsäure hinzu, erhitzt unter Rühren 2 Stunden zum Sieden, lässt abkühlen und giesst auf 150 ml Eiswasser. Man trennt die organische Phase ab und schüttelt die wässrige Phase zweimal mit je 50 ml Diäthyläther aus. Die organischen Phasen werden vereinigt, mit 50 ml gesättigter Kaliumcarbonatlösung neutralisiert, mit 50 ml Wasser gewaschen, über Natriumsulfat getrocknet und unter vermindertem Druck eingedampft. Der Eindampfrückstand wird unter vermindertem Druck (unter 0,2 mbar) destilliert. Der α-Chlorphenylessigsäuremethylester siedet unter 0,13 mbar bei 62°;($n_D^{20}$: 1,5268).

In analoger Weise kann man durch Umsetzung von N-Diphenylmethyläthylendiamin mit Benzylchlorid bzw. 1-Phenyläthylchlorid das N,N-Dibenzyl-N'-diphenylmethyl-äthylendiamin, Smp. 89-91° (aus Diisopropyläther), bzw. das N-Diphenylmethyl-N'-(1-phenyläthyl)-äthylendiamin, Smp. des Dihydrochlorides 160° (Zers.), herstellen.

Beispiel 6: 8,5 g N-(α-Methoxycarbonylbenzyl)-N'-diphenylmethyläthylendiamin-diacetat werden mit 85 ml 10 %iger Natronlauge versetzt und dreimal mit je 85 ml Diäthyläther ausgeschüttelt. Die Aetherlösungen des N-(α-Methoxycarbonyl-benzyl)-N'-diphenylmethyl-äthylendiamins werden vereinigt und über Magnesiumsulfat getrocknet. Dann wird unter gutem Kühlen (erforderlichenfalls im Aceton/Trockeneisbad) bis zur vollständigen Ausfällung der Base als Dihydrochlorid Chlorwasserstoff eingeleitet. Das Salz wird abgesaugt, mehrmals mit Aether nachgewaschen und unter vermindertem Druck (unter 0,1 mbar) bis zu Gewichtskonstanz getrocknet. Man erhält das N-(α-Methoxycarbonylbenzyl)-N'-diphenylmethyläthylendiamin-dihydrochlorid vom Smp. 148° (Zers.).

Beispiel 7: Zu einem siedenden Gemisch von 13,2 g 1,4-Bis-(brommethyl)-benzol in 60 ml Acetonitril wird ein Gemisch von 18,3 g Benzhydrylamin, 10 g Triäthylamin und 60 ml Acetonitril zugetropft. Man lässt über Nacht sieden, engt stark ein, verteilt zwischen je 200 ml Aether und

10 %-iger Natronlauge, dampft ein und kristallisiert aus Diäthyläther um. Man erhält das N,N'-Bis-(Diphenylmethyl)-p-xylylendiamin vom Smp. 92-94°.

In analoger Weise erhält man durch Umsetzung von o-Methylbenzylamin mit 1,2-Dibromäthan das N,N'-Di-(o-methylbenzyl)-äthylendiamin, Smp. 38-39° (aus Essigsäureäthylester), und durch Umsetzung von Benzhydrylamin mit 1,2- bzw. 1,3-Dibrompropan bzw. 1,3-Bis-(brommethyl)-benzol das N,N'-Bis-(diphenylmethyl)-1,2-propylendiamin, Smp. 75-77° (aus Methanol/Wasser), das N,N'-Bis-(Diphenylmethyl)-1,3-propylendiamin, Smp. 83-84° (aus Aceton) bzw. das N,N'-Bis-(Diphenylmethyl)-m-xylylendiamin, Smp. 89-90° (aus Isopropanol).

Beispiel 8: 11 g N-Diphenylmethyl-äthylendiamin, 10,1 g Triäthylamin und 7,0 g 1-Phenyläthylchlorid werden unter Rühren in 85 ml Acetonitril gelöst und 30 Stunden zum Rückfluss erhitzt. Man dampft unter vermindertem Druck ein, versetzt mit 50 ml 10 %iger Natronlauge und schüttelt gründlich mit Diäthyläther aus. Die Aetherauszüge werden vereinigt, über Natriumsulfat getrocknet und eingedampft. Man erhält das N-Diphenylmethyl-N'-(1-phenyläthyl) -äthylendiamin in Form eines gelben Oeles, welches an Kieselgel mit Methanol/Essigsäureäthylester (1:1) als Laufmittel chromatographisch gereinigt und zur Charakterisierung wie in Beispiel 4 beschrieben in das Dihydrochlorid vom Smp 160° überführt werden kann.

Beispiel 9: 5,6 g N-Diphenylmethyl-äthylendiamin in 30 ml siedendem Acetonitril werden mit einer Lösung von 2,9 g Benzylchlorid in 25 ml Acetonitril versetzt. Man erhitzt 12 Stunden zum Rückfluss, engt ein und verteilt zwischen Diäthyläther und 10%-iger Natronlauge. Die organischen Phasen werden über Natriumsulfat getrocknet und eingedampft. Der Rückstand kann aus Methanol oder Diisopropyläther umkristallisiert werden. Man erhält das N,N-Dibenzyl-N'-diphenylmethyl-äthylendiamin vom Smp. 89-91°.

Beispiel 10: Zu einer Lösung von 12,05 g Methansulfonsäure in 175 ml Wasser werden unter kräftigem Rühren bei 10° 25 g N,N'-Bis-(Diphenylmethyl)-1,2-propylendiamin, gelöst in 75 ml Methanol zugetropft. Man lässt 15 Minuten bei Raumtemperatur nachrühren, impft an und lässt über Nacht kristallisieren. Dann zieht man das Methanol unter vermindertem Druck ab, lässt bei 0° zu Ende kristallisieren, saugt ab, wäscht mit 40 ml Eiswasser nach und lässt unter 0,1 bis 0,4 mbar, zunächst einige Stunden bei Raumtemperatur und dann über Nacht bei 50° trocknen. Man erhält das N,N'-Bis-(diphenylmethyl)-1,2-propylendiamin-bis-methansulfonat vom Smp. 218-219° (Zers.).

Beispiel 11: Tabletten enthaltend 5 mg N,N'-Bis-(Disphenylmethyl)-1,2-propylendiamin-bis-methansulfonat können z.B. wie folgt erhalten werden:

Zusammensetzung: (für 10'000 Tabletten):

| | |
|---|---|
| Wirkstoff | 50 g |
| Lactose | 460 g |
| Maisstärke | 450 g |
| Polyvinylpyrrolidon | 20 g |
| Magnesiumstearat | 10 g |
| kolloidales Siliciumoxid | 10 g |
| Wasser | q.s. |

Der Wirkstoff, die Lactose und 400 g der Maisstärke werden gemischt und mit einer wässrigen Lösung von Polyvinylpyrrolidon befeuchtet. Das Gemisch wird granuliert und getrocknet, und mit dem Magnesiumstearat, dem kolloidalen Siliciumoxid und dem Rest der Maisstärke versetzt. Das Gemisch wird durch ein Sieb getrieben, gemischt und zu Tabletten von 100 mg Gewicht (Durchmesser: 6 mm) verpresst.

Beispiel 12: Tabletten enthaltend 20 mg N,N'-Bis-(Diphenylmethyl)-1,2-propylendiamin-bis-methansulfonat (Wirkstoff) können beispielsweise in folgender Zusammensetzung hergestellt werden:

| Zusammensetzung (für 10'000 Tabletten) | |
|---|---|
| Wirkstoff | 200 g |
| Milchzucker | 500 g |
| Weizenstärke | 773 g |
| Kolloidale Kieselsäure | 13 g |
| Talk | 12 g |
| Magnesiumstearat | 2 g |

Herstellung: Der Wirkstoff wird mit dem Milchzucker, einem Teil der Weizenstärke und mit kolloidaler Kieselsäure gemischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastiche Masse entstanden ist. Diese Masse wird durch ein Sieb von etwa 1 mm Maschenweite getrieben, getrocknet, und das trockene Granulat nochmals durch ein Sieb getrieben. Dann werden die restliche Weizenstärke, der Talk und das Magnesiumstearat zugemischt. Die erhaltene Tablettiermischung wird zu Tabletten von je 150 mg mit Bruchkerbe verpresst.

Beispiel 13: Tabletten enthaltend 10 mg N,N'-Bis-(Diphenylmethyl)-1,2-propylendiamin-bis-methansulfonat (Wirkstoff) können z.B. in folgender Zusammensetzung hergestellt werden:

| Zusammensetzung (für 10'000 Tabletten) | |
|---|---|
| Wirkstoff | 100 g |
| Weizenstärke | 295 g |
| Milchzucker | 500 g |
| Kolloidale Kieselsäure | 50 g |
| Talk | 50 g |
| Magnesiumstearat | 5 g |

Herstellung: Der Wirkstoff wird mit einem Teil der Weizenstärke, mit Milchzucker und kolloidaler Kieselsäure vermischt und die Mischung durch ein Sieb getrieben. Ein weiterer Teil der Weizenstärke wird mit der 5-fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das trockene Granulat nochmals durch ein Sieb getrieben. Darauf werden die restliche Weizenstärke, Talk und Magnesiumstearat zugemischt und die erhaltene Mischung zu Tabletten von 100 mg Gewicht (mit Bruchkerbe) verpresst.

Beispiel 14: Kapseln mit einem Gehalt an 10 mg N,N'-Bis-(Diphenylmethyl)-1,2-propylendiamin-bis-methansulfonat (Wirkstoff) werden z.B. wie folgt hergestellt:

| Ingredienzien (für 10'000 Kapseln) | |
|---|---|
| Wirkstoff | 100 g |
| Milchzucker | 360 g |
| Magnesiumstearat | 40 g |

Die Ingredienzien werden in einem geeigneten Mischer gemischt, durch ein Nr. 40-Sieb gesiebt und nochmals gemischt. Je 0,05 g der Mischung werden in Gelatine-Kapseln Nr. 3 abgefüllt.

Beispiel 15: In analoger Weise wie in den Beispielen 11 - 14 beschrieben, können auch pharmazeutische Präparate enthaltend eine der in den Beispielen 1 bis 9 oder in der Beschreibung genannten Verbindungen der Formel I hergestellt werden.

Patentansprüche (für alle benannten Länder ausser Oesterreich)

1. Eine neue Verbindung der Formel I

$$R_1-CH(R_2)-N(R_3)-R_4 \quad (I),$$

worin $R_1$ unsubstituiertes oder substituiertes Phenyl bedeutet, $R_2$ Wasserstoff, Niederalkyl, unsubstituiertes oder substituiertes Phenyl oder freies oder verestertes Carboxy darstellt, $R_3$ Wasserstoff oder Niederalkyl ist und $R_4$ eine Gruppe der Formel $-alk_1-X-alk_2-N(R_6)CH(R_7)-R_8$ darstellt, worin $alk_1$ und $alk_2$ Niederalkyliden bedeuten, X eine direkte Bindung, Methylen oder unsubstituiertes oder substituiertes Phenylen darstellt, $R_6$ für Wasserstoff steht oder, sofern $R_3$ Wasserstoff ist, auch Niederalkyl oder unsubstituiertes oder substituiertes Phenylniederalkyl bedeuten kann, $R_7$ Wasserstoff, Niederalkyl, unsubstituiertes oder substituiertes Phenyl oder freies oder verestertes Carboxy darstellt und $R_8$ unsubstituiertes oder substituiertes Phenyl bedeutet, mit der Massgabe, dass in Verbindungen der Formel I, worin X unsubstituiertes oder substituiertes Phenylen darstellt, mindestens einer der Reste $R_1$ und $R_8$ von unsubstituiertem oder N-substituiertem Aminoalkoxyphenyl verschieden ist, wenn $R_2$, $R_3$, $R_6$ und $R_7$ für Wasserstoff oder Niederalkyl stehen, dass ferner in Verbindungen der Formel I, worin $alk_1$ und $alk_2$ Methylen bedeuten und X Methylen oder eine direkte Bindung darstellen, mindestens einer der Reste $R_1$ und $R_8$ von unsubstituiertem oder durch Methoxy und/oder Chlor mono- oder disubstituiertem Phenyl und von p-Hydroxyphenyl verschieden ist, wenn $R_2$, $R_3$, $R_6$ und $R_7$ Wasserstoff darstellen, und dass weiterhin in Verbindungen der Formel I, worin $alk_1$ und $alk_2$ Methylen bedeuten und X eine direkte Bindung darstellt, mindestens einer der Reste $R_1$ und $R_8$ von Phenyl oder mindestens einer der Reste $R_3$ und $R_6$ von Wasserstoff verschieden ist, wenn beide Reste $R_2$ und $R_7$ jeweils Wasserstoff, Phenyl oder Methyl bedeuten oder $R_2$ Aethyl und $R_7$ Wasserstoff ist, wobei $R_1$ und $R_8$ keine gleichen aus unsubstituiertem oder in 3-, 4-, 5- und/oder 6-Stellung durch Isopropyl, Methoxy, Hydroxy, Chlor, Diäthylamino, Dimethylamino

und/oder Nitro substituierten Phenylresten ausgewählte Substituenten bedeuten, wenn $R_2$, $R_3$, $R_6$ und $R_7$ Wasserstoff bedeuten und X für Methylen steht, $R_1$ und $R_8$ keine gleichen aus p-Anilino, p-Hydroxyphenyl und p-Methylphenyl ausgewählten Substituenten darstellen, wenn $R_2$, $R_3$, $R_6$ und $R_7$ für Wasserstoff stehen und X eine direkte Bindung bedeutet, und $R_1$ und $R_8$ keine gleichen aus p-Aminophenoxy- und p-Nitrophenoxyphenyl ausgewählte Reste bedeuten, wenn $R_2$ und $R_7$ Wasserstoff und $R_3$ und $R_6$ Wasserstoff oder Niederalkyl bedeuten und X für Methylen oder eine direkte Bindung steht, oder ein Salz davon.

2. Eine Verbindung gemäss Anspruch 1 der Formel I, worin $R_1$ unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenyl bedeutet, $R_2$ Wasserstoff, Niederalkyl, unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenyl oder aliphatisch, cycloaliphatisch oder araliphatisch verestertes Carboxy darstellt, $R_3$ Wasserstoff oder Niederalkyl ist und $R_4$ eine Gruppe der Formel $-alk_1-X-alk_2-N(R_6)-CH(R_7)-R_8$ darstellt, worin $alk_1$ und $alk_2$ Niederalkyliden bedeuten, X eine direkte Bindung, Methylen oder unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenylen darstellt, $R_6$ für Wasserstoff steht oder, sofern $R_3$ Wasserstoff ist, auch Niederalkyl oder unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenylniederalkyl bedeuten kann, $R_7$ Wasserstoff, Niederalkyl, unsubstituiertes oder durch aliphatische, cycloaliphatische oder araliphatische

Reste, Hydroxy, veräthertes oder verestertes Hydroxy, unsubstituiertes oder aliphatisch substituiertes Amino, Acylamino, Nitro und/oder Trifluormethyl substituiertes Phenyl oder aliphatisch, cycloaliphatisch oder araliphatisch verestertes Carboxy darstellt, und $R_8$ unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenyl darstellt, mit der Massgabe, dass in Verbindungen der Formel I, worin X unsubstituiertes oder wie angegeben substituiertes Phenylen ist, mindestens einer der Reste $R_1$ und $R_8$ von unsubstituiertem oder N-substituiertem Aminoalkoxyphenyl verschieden ist, wenn $R_2$, $R_3$, $R_6$ und $R_7$ Wasserstoff oder Niederalkyl bedeuten , dass ferner in Verbindungen, worin $alk_1$ und $alk_2$ Methylen bedeuten und X Methylen oder eine direkte Bindung darstellt, mindestens einer der Reste $R_1$ und $R_8$ von unsubstituiertem oder durch Methoxy und/oder Chlor mono- oder disubstituiertes Phenyl und von p-Hydroxyphenyl verschieden ist, wenn $R_2$, $R_3$, $R_6$ und $R_7$ Wasserstoff darstellen, und mit der weiteren Massgabe, dass in Verbindungen der Formel I, worin $alk_1$ und $alk_2$ Methylen bedeuten und X eine direkte Bindung darstellt, mindestens einer der Reste $R_1$ und $R_8$ von Phenyl oder mindestens einer der Reste $R_3$ und $R_6$ von Wasserstoff verschieden ist, wenn beide Reste $R_2$ und $R_7$ jeweils Wasserstoff, Phenyl oder Methyl bedeuten oder $R_2$ Aethyl und $R_7$ Wasserstoff ist, oder mindestens einer der Reste $R_3$ und $R_6$ von Wasserstoff verschieden ist, wenn $R_1$, $R_2$, $R_7$ und $R_8$ unsubstituiertes Phenyl darstellen, wobei $R_1$ und $R_8$ keine gleichen aus unsubstituiertem oder in 3-, 4-, 5- und/oder 6-Stellung durch Isopropyl, Methoxy, Hydroxy, Chlor, Diäthylamino und/oder Dimethylamino oder in 4- und/oder 6-Stellung durch Chlor und/oder Nitro substituierten Phenylresten ausgewählte Substituenten bedeuten, wenn $R_2$, $R_3$, $R_6$ und $R_7$ Wasserstoff bedeuten und X für Methylen steht, $R_1$ und $R_8$ keine gleichen aus p-Anilino, p-Hydroxyphenyl und p-Methylphenyl ausgewählten Substituenten darstellen, wenn $R_2$, $R_3$, $R_6$ und $R_7$ für Wasserstoff stehen und X eine direkte Bindung ist, und $R_1$ und $R_8$ keine gleichen aus p-Aminophenoxy- und p-Nitrophenoxyphenyl ausgewählte Reste bedeuten, wenn $R_2$ und $R_7$ Wasserstoff und $R_3$ und $R_6$ Wasserstoff oder Niederalkyl bedeuten und X für Methylen oder eine direkte Bindung steht, oder ein Salz davon.

3. Eine Verbindung gemäss Anspruch 1 der Formel I, worin $R_1$ unsubstituiertes oder durch Niederalkyl, Niederalkenyl, Niederalkinyl, Mono- oder Dihydroxyniederalkyl, Niederalkoxyniederalkyl, Niederalkanoyloxyniederalkyl, 3- bis 8-gliedriges Cycloalkyl, Hydroxy, Niederalkoxy, Niederalkenyloxy, Niederalkinyloxy, Mono- oder Dihydroxyniederalkoxy, Niederalkoxyniederalkoxy, an benachbarte Kohlenstoffatome gebundenes Niederalkyl(id)endioxy, Halogen der Atomnummer bis und mit 35, Niederalkanoyloxy, Amino, Mono- oder Diniederalkylamino, und/oder Mono- oder Diniederalkanoylamino substituiertes Phenyl bedeutet, $R_2$ Wasserstoff, Niederalkyl, unsubstituiertes oder wie für $R_1$ angegeben substituiertes Phenyl, Niederalkoxycarbonyl oder 3- bis 8-gliedriges Cycloalkoxycarbonyl darstellt, $R_3$ Wasserstoff oder Niederalkyl ist und $R_4$ eine Gruppe der Formel $-alk_1-X-alk_2-N(R_6)-CH(R_7)-R_8$ darstellt, in der $alk_1$ und $alk_2$ Niederalkyliden bedeuten, X Methylen, eine direkte Bindung oder unsubstituiertes oder durch Niederalkyl, Niederalkoxy, Halogen und/oder Trifluormethyl substituiertes Phenylen bedeutet, $R_6$ Wasserstoff oder Niederalkyl ist, $R_7$ Wasserstoff, Niederalkyl oder unsubstituiertes oder wie für $R_2$ angegeben substituiertes Phenyl, Niederalkoxycarbonyl oder 3- bis 8-gliedriges Cycloalkoxycarbonyl darstellt und $R_8$ unsubstituiertes oder wie für $R_1$ angegeben substituiertes Phenyl bedeutet, mit der Massgabe, dass in Verbindungen der Formel I, worin $alk_1$ und $alk_2$ Methylen bedeuten und X Methylen oder eine direkte Bindung darstellt mindestens einer der Reste $R_1$ und $R_8$ von unsubstituiertem oder durch Methoxy und/oder Chlor mono- oder disubstituiertem Phenyl und von p-Hydroxyphenyl verschieden ist, wenn $R_2$, $R_3$, $R_6$ und $R_7$ Wasserstoff darstellen, und mit der weiteren Massgabe, dass in Verbindungen der Formel I, worin $alk_1$ und $alk_2$ Methylen bedeuten und X eine direkte Bindung darstellt, mindestens einer der Reste $R_1$

und $R_8$ von Phenyl oder mindestens einer der Reste $R_3$ und $R_6$ von Wasserstoff verschieden ist, wenn beide Reste $R_2$ und $R_7$ jeweils Wasserstoff, Methyl oder Phenyl bedeuten, oder $R_2$ Aethyl und $R_7$ Wasserstoff ist, wobei $R_1$ und $R_8$ keine gleichen aus unsubstituiertem oder in 3-, 4-, 5- und/oder 6-Stellung durch Isopropyl, Methoxy, Hydroxy, Chlor, Diäthylamin substituierten Phenylresten ausgewählte Substituenten bedeuten, wenn $R_2$, $R_3$, $R_6$ und $R_7$ Wasserstoff bedeuten und X für Methylen steht, und $R_1$ und $R_8$ keine gleichen aus p-Anilino, p-Hydroxyphenyl und p-Methylphenylausgewählte Substituenten darstellen, wenn $R_2$, $R_3$, $R_6$ und $R_7$ für Wasserstoff stehen und X eine direkte Bindung bedeutet, oder ein Salz davon.

4. Eine Verbindung gemäss Anspruch 1 der Formel I, worin $R_1$ unsubstituiertes oder durch Niederalkyl, Niederalkenyl, 3- bis 8-gliedriges Cycloalkyl, Niederalkoxy, Niederalkenyloxy, an benachbarte Kohlenstoffatome gebundenes, durch Niederalkylendioxy, Halogen der Atomnummer bis und mit 35, Amino und/oder Mono- oder Diniederalkylamino substituiertes Phenyl bedeutet, $R_2$ Wasserstoff oder unsubstituiertes oder wie für $R_1$ angegeben substituiertes Phenyl darstellt, $R_3$ Wasserstoff oder Niederalkyl, wie Methyl, ist und $R_4$ eine Gruppe der Formel $-alk_1-X-alk_2-N(R_6)-CH(R_7)-R_8$ darstellt, $alk_1$ und $alk_2$ Niederalkyliden bedeuten, X eine direkte Bindung, Methylen oder unsubstituiertes Phenylen ist, $R_6$ Wasserstoff oder Niederalkyl ist, $R_7$ Wasserstoff, Niederalkyl, unsubstituiertes oder wie für $R_2$ angegeben substituiertes Phenyl, Niederalkoxycarbonyl, oder 3- bis 8-gliedriges Cycloalkoxycarbonyl darstellt und $R_8$ unsubstituiertes oder wie für $R_1$ angegeben substituiertes Phenyl bedeutet, mit der Massgabe, dass in Verbindungen der Formel I, worin $alk_1$ und $alk_2$ Methylen bedeuten, und X Methylen oder eine direkte Bindung darstellt, mindestens einer der Reste $R_1$ und $R_8$ von unsubstituiertem oder durch Methoxy und/oder Chlor mono- oder disubstituiertem Phenyl verschieden ist, wenn $R_2$, $R_3$, $R_6$ und $R_7$ Wasserstoff darstellen, und mit der weiteren Massgabe, dass in Verbindungen der Formel I, worin $alk_1$ und $alk_2$ Methylen bedeuten

und X eine direkte Bindung darstellt, mindestens einer der Reste $R_1$ und $R_8$ von Phenyl oder mindestens einer der Reste $R_3$ und $R_6$ von Wasserstoff verschieden ist, wenn beide Reste $R_2$ und $R_7$ jeweils Wasserstoff, Phenyl oder Methyl bedeuten, wobei $R_1$ und $R_8$ keine gleichen aus unsubstituiertem oder in 3-, 4-, 5- und/oder 6-Stellung durch Isopropyl, Methoxy, Chlor, Diäthylamino und/oder Dimethylamino substituierten Phenylresten ausgewählte Substituenten bedeuten, wenn $R_2$, $R_3$, $R_6$ und $R_7$ Wasserstoff bedeuten und X für Methylen steht, und $R_1$ und $R_8$ keine gleichen aus p-Anilino und p-Methylphenyl ausgewählten Reste bedeuten, wenn $R_2$, $R_3$, $R_6$ und $R_7$ für Wasserstoff stehen und X eine direkte Bindung ist, oder ein Säureadditionssalz davon.

5. Eine Verbindung gemäss Anspruch 1 der Formel I, worin $R_1$ unsubstituiertes oder durch Niederalkyl mit bis und mit 4 Kohlenstoffatomen, wie Methyl und/oder Halogen der Atomnummer bis und mit 35, wie Chlor, substituiertes Phenyl bedeutet, $R_2$ Wasserstoff oder gegebenenfalls wie für $R_1$ angegeben substituiertes Phenyl bedeutet, $R_3$ Wasserstoff oder Niederalkyl mit bis und mit 4 Kohlenstoffatomen, wie Methyl, bedeutet und $R_4$ eine Gruppe der Formel $-CH_2-X-CH_2-R_5$ ist, in der X eine direkte Bindung, Methylen oder Phenylen bedeutet, $R_5$ Amino oder substituiertes Amino der Formel $-N(R_6)-CH(R_7)-R_8$ ist, $R_6$ für Wasserstoff steht oder, sofern $R_3$ Wasserstoff ist, auch Niederalkyl mit bis und mit 4 Kohlenstoffatomen, wie Methyl, bedeuten kann, $R_7$ Wasserstoff, Niederalkyl mit bis und mit 4 Kohlenstoffatomen, wie Methyl, unsubstituiertes oder wie für $R_1$ angegeben substituiertes Phenyl oder Niederalkoxycarbonyl mit bis und mit 4 Kohlenstoffatomen im Alkylteil, wie Methoxycarbonyl bedeutet und $R_8$ unsubstituiertes oder wie für $R_1$ angegeben substituiertes Phenyl darstellt, mit der Massgabe, dass mindestens einer der Reste $R_1$ und $R_8$ von unsubstituiertem oder durch Chlor mono- oder disubstituiertem Phenyl oder mindestens einer der Reste $R_2$, $R_3$, $R_6$ und $R_7$ von Wasserstoff verschieden ist, wenn X für Methylen oder eine direkte Bindung steht, und mit der weiteren Massgabe, dass mindestens einer der Reste $R_1$ und $R_8$

von Phenyl oder mindestens einer der Reste $R_3$ und $R_6$ von Wasserstoff verschieden ist, wenn beide Reste $R_2$ und $R_7$ Wasserstoff oder Phenyl darstellen und X eine direkte Bindung bedeutet, und mindestens einer der Reste $R_1$ und $R_8$ von p-Methylphenyl verschieden ist, wenn $R_2$, $R_3$, $R_6$ und $R_7$ Wasserstoff bedeuten und X Methylen ist, wobei $R_1$ und $R_8$ keine gleichen aus unsubstituiertem oder in p-Stellung, den m-Stellungen oder einer o-Stellung durch Isopropyl oder Chlor substituiertem Phenyl ausgewählte Substituenten bedeuten, wenn $R_2$, $R_3$, $R_6$ und $R_7$ Wasserstoff bedeuten und X Methylen darstellt oder ein Säureadditionssalz davon.

6. N,N'-Bis-(diphenylmethyl)-1,3-propylendiamin oder ein Säureadditionssalz davon.

7. N,N'-Bis-(diphenylmethyl)-p-xylylen-diamin oder ein Säureadditionssalz davon.

8. N-Diphenylmethyl-N'-(1-phenyläthyl)-äthylendiamin oder ein Säureadditionssalz davon.

9. N-Diphenylmethyl-N'-(α-methoxycarbonylbenzyl)-äthylendiamin oder ein Säureadditionssalz davon.

10. N,N'-Bis-(diphenylmethyl)-1,2-propylendiamin oder ein Säureadditionssalz davon.

11. N,N'-Bis-(diphenylmethyl)-m-xylylen-diamin oder ein Säureadditionssalz davon.

12. N,N'-Di-(o-methylbenzyl)-äthylendiamin oder ein Säureadditionssalz davon.

13. N,N'-Dibenzyl-N-methyl-äthylendiamin oder ein Säureadditionssalz davon.

14. N,N-Dibenzyl-N'-diphenylmethyl-äthylendiamin oder ein Säureadditionssalz davon.

15. Verfahren zur Herstellung neuer Verbindungen der Formel I

$$R_1-CH(R_2)-N(R_3)-R_4 \qquad (I)$$ ,

worin $R_1$ unsubstituiertes oder substituiertes Phenyl bedeutet, $R_2$ Wasserstoff, Niederalkyl, unsubstituiertes oder substituiertes Phenyl oder freies oder verestertes Carboxy darstellt, $R_3$ Wasserstoff oder Niederalkyl ist und $R_4$ eine Gruppe der Formel $-alk_1-X-alk_2-N(R_6)CH(R_7)-R_8$ darstellt, worin $alk_1$ und $alk_2$ Niederalkyliden bedeuten, X eine direkte Bindung, Methylen oder unsubstituiertes oder substituiertes Phenylen darstellt, $R_6$ für Wasserstoff steht oder, sofern $R_3$ Wasserstoff ist, auch Niederalkyl oder unsubstituiertes oder substituiertes Phenylniederalkyl bedeuten kann, $R_7$ Wasserstoff, Niederalkyl, unsubstituiertes oder substituiertes Phenyl oder freies oder verestertes Carboxy darstellt und $R_8$ unsubstituiertes oder substituiertes Phenyl bedeutet, mit der Massgabe, dass in Verbindungen der Formel I, worin X unsubstituiertes oder substituiertes Phenylen darstellt, mindestens einer der Reste $R_1$ und $R_8$ von unsubstituiertem oder N-substituiertem Aminoalkoxyphenyl verschieden ist, wenn $R_2$, $R_3$, $R_6$ und $R_7$ für Wasserstoff oder Niederalkyl stehen, dass ferner in Verbindungen der Formel I, worin $alk_1$ und $alk_2$ Methylen bedeuten und X Methylen oder eine direkte Bindung darstellen, mindestens einer der Reste $R_1$ und $R_8$ von unsubstituiertem oder durch Methoxy und/oder Chlor mono- oder disubstituiertem Phenyl und von p-Hydroxyphenyl verschieden ist, wenn $R_2$, $R_3$, $R_6$ und $R_7$ Wasserstoff darstellen, und dass weiterhin in Verbindungen der Formel I, worin $alk_1$ und $alk_2$ Methylen bedeuten und X eine direkte Bindung darstellt, mindestens einer der Reste $R_1$ und $R_8$ von Phenyl oder mindestens einer der Reste $R_3$ und $R_6$ von Wasserstoff verschieden ist, wenn beide Reste $R_2$ und $R_7$ jeweils Wasserstoff, Phenyl oder Methyl bedeuten oder $R_2$ Aethyl und $R_7$ Wasserstoff ist, wobei $R_1$ und $R_8$ keine gleichen aus unsubstituier-

tem oder in 3-, 4-, 5- und/oder 6-Stellung durch Isopropyl, Methoxy, Hydroxy, Chlor, Diäthylamino, Dimethylamino und/oder Nitro substituierten Phenylresten ausgewählte Substituenten bedeuten, wenn $R_2$, $R_3$, $R_6$ und $R_7$ Wasserstoff bedeuten und X für Methylen steht, $R_1$ und $R_8$ keine gleichen aus p-Anilino, p-Hydroxyphenyl und p-Methylphenyl ausgewählten Substituenten darstellen, wenn $R_2$, $R_3$, $R_6$ und $R_7$ für Wasserstoff stehen und X eine direkte Bindung bedeutet, und $R_1$ und $R_8$ keine gleichen aus p-Aminophenoxy- und p-Nitrophenoxyphenyl ausgewählte Reste bedeuten, wenn $R_2$ und $R_7$ Wasserstoff und $R_3$ und $R_6$ Wasserstoff oder Niederalkyl bedeuten und X für Methylen oder eine direkte Bindung steht, und ihrer Salze, dadurch gekennzeichnet, dass man eine Verbindung der Formel

$$Y_1\text{-alk}_1\text{-X-alk}_2\text{-N}(R_6)\text{-CH}(R_7)\text{-}R_8 \qquad \text{(II)}$$

mit einer Verbindung der Formel

$$R_1\text{-CH}(R_2)\text{-}Y_2 \qquad \text{(III)}$$

kondensiert, wobei einer der Reste $Y_1$ und $Y_2$ eine nukleophil ersetzbare Gruppe Y und der andere eine Gruppe der Formel $-N(R_3)-H$ darstellt oder in einer Verbindung der Formel

$$R_1\text{-}Y_3\text{-X'-}Y_4\text{-}R_8 \qquad \text{(VI)},$$

worin $Y_3$ einen in eine Gruppe der Formel $-CH(R_2)-N(R_3)-alk_1-$ überführbaren Rest $Y_3'$, X'eine Gruppe X oder einen in Methylen überführbaren Rest X" darstellt oder $Y_3$ eine Gruppe der Formel $-CH(R_2)-N(R_3)-alk_1-$ und X' einen in Methylen überführbaren Rest bedeutet, und worin $Y_4$ für eine Gruppe der Formel $-alk_2-N(R_6)-CH(R_7)-$ oder für einen in diese überführbaren Rest $Y_4'$ steht, oder in einem Salz davon $Y_3'$ in eine Gruppe der Formel $-CH(R_2)-N(R_3)-alk_1-$, X" in Methylen und/oder $Y_4'$ in eine Gruppe der Formel $-alk_2-N(R_6)-CH(R_7)-$ überführt, erforderlichenfalls

ein verfahrensgemäss erhältliches Isomerengemisch in die Komponenten auftrennt und gewünschtenfalls eine verfahrensgemäss erhältliche Verbindung in eine andere Verbindung der Formel I und/oder eine verfahrensgemäss erhältliche freie Verbindung in ein Salz oder ein verfahrensgemäss erhältliches Salz in die freie Verbindung oder in ein anderes Salz überführt.

16. Die nach dem Verfahren des Anspruches 15 erhältlichen Verbindungen.

17. Eine Verbindung der Formel I

$$R_1\text{-CH}(R_2)\text{-N}(R_3)\text{-}R_4 \qquad (I) \quad ,$$

worin $R_1$ unsubstituiertes oder substituiertes Phenyl bedeutet, $R_2$ Wasserstoff, Niederalkyl, unsubstituiertes oder substituiertes Phenyl oder freies oder verestertes Carboxy darstellt, $R_3$ Wasserstoff oder Niederalkyl und $R_4$ eine Gruppe der Formel $-alk_1\text{-X-}alk_2\text{-}R_5$ bedeutet oder $R_3$ und $R_4$ gemeinsam eine Gruppe der Formel $-alk_3\text{-NH-}alk_4-$ darstellen, wobei $alk_1$ und $alk_2$ Niederalkyliden bedeuten, $alk_3$ und $alk_4$ für Niederalkylen stehen, X eine direkte Bindung, Methylen oder unsubstituiertes oder substituiertes Phenylen darstellt, $R_5$ Amino oder substituiertes Amino der Formel $-N(R_6)\text{-CH}(R_7)\text{-}R_8$ bedeutet, in der $R_6$ für Wasserstoff steht oder, sofern $R_3$ Wasserstoff ist, auch Niederalkyl oder unsubstituiertes oder substituiertes Phenylniederalkyl bedeuten kann, $R_7$ Wasserstoff, Niederalkyl, unsubstituiertes oder substituiertes Phenyl oder freies oder verestertes Carboxy darstellt und $R_8$ unsubstituiertes oder substituiertes Phenyl bedeutet, mit der Massgabe, dass in Verbindungen, worin $R_3$ Wasserstoff oder Niederalkyl, $R_4$ eine Gruppe $-alk_1\text{-X-}alk_2\text{-}R_5$ und $R_5$ eine Gruppe $-N(R_6)\text{-CH}(R_7)\text{-}R_8$ bedeutet, mindestens einer der Reste $R_1$ und $R_8$ von unsubstituiertem oder N-substituiertem Aminoalkoxyphenyl oder mindestens einer der Reste $R_2$, $R_3$, $R_6$ und $R_7$ von Wasserstoff und Niederalkyl verschieden ist, wenn X unsubstituiertes oder substituiertes Phenylen bedeutet, und mindestens einer der Reste $R_1$ und $R_8$ von unsubstituiertem oder

durch Methoxy und/oder Chlor mono- oder disubstituiertes Phenyl oder mindestens einer der Reste $R_2$, $R_3$, $R_6$ und $R_7$ von Wasserstoff verschieden ist, wenn $alk_1$ und $alk_2$ für Methylen stehen und X Methylen oder eine direkte Bindung bedeutet, und mit der weiteren Massgabe, dass in Verbindungen, worin $R_3$ und $R_4$ gemeinsam eine Gruppe $-alk_3-NH-alk_4-$ darstellen und $alk_3$ Aethylen ist, $R_1$ von Phenyl oder $R_2$ von Phenyl und Wasserstoff und ferner $R_1$ von 2,3,4-Trimethoxyphenyl, o-Amino-dichlor-phenyl, o-Acylamino-dichlor-phenyl und o-Diacylamino-dichlor-phenyl oder $R_2$ von Wasserstoff verschieden ist, wenn $alk_4$ Aethylen bedeutet, und $R_1$ von Phenyl oder $R_2$ von Wasserstoff verschieden ist, wenn $alk_4$ 1,3-Propylen ist, wobei im Falle, dass $R_4$ eine Gruppe $-alk_1-X-alk_2-R_5$, $alk_1$ und $alk_2$ Methylen und $R_5$ eine Gruppe der Formel $-N(R_6)-CH(R_7)-R_8$ darstellt, $R_1$ und $R_8$ keine gleichen aus unsubstituiertem oder in 3-, 4-, 5- und/oder 6-Stellung durch Isopropyl, Methoxy, Hydroxy, Chlor, Diäthylamino, Dimethylamino und/oder Nitro substituierten Phenylresten ausgewählte Substituenten bedeuten, wenn $R_2$, $R_3$, $R_6$ und $R_7$ Wasserstoff bedeuten und X für Methylen steht, und $R_1$ und $R_8$ keine gleichen aus p-Aminophenoxy- und p-Nitrophenoxyphenyl ausgewählte Reste bedeuten, wenn $R_2$ und $R_7$ Wasserstoff und $R_3$ und $R_6$ Wasserstoff oder Niederalkyl bedeuten und X für Methylen oder eine direkte Bindung steht, oder eines ihrer pharmazeutisch verwendbaren Salze, mit der Massgabe, dass Säureadditionssalze mit organischen Carbonsäuren im Anion keinen Lactamring aufweisen, zur Anwendung in einem Verfahren zur therapeutischen Behandlung des menschlichen oder tierischen Körpers.

18. Eine Verbindung gemäss Anspruch 17, worin $R_1$ unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenyl bedeutet, $R_2$ Wasserstoff, Niederalkyl, unsubstituiertes oder durch aliphatische, cycloaliphatische oder araliphatische Reste, Hydroxy, veräthertes oder verestertes Hydroxy,

unsubstituiertes oder aliphatisch substituiertes Amino, Acylamino, Nitro und/oder Trifluormethyl substituiertes Phenyl oder aliphatisch, cycloaliphatisch oder araliphatisch verestertes Carboxy darstellt, $R_3$ Wasserstoff oder Niederalkyl und $R_4$ eine Gruppe der Formel $-alk_1-X-alk_2-R_5$ bedeutet oder $R_3$ und $R_4$ gemeinsam eine Gruppe der Formel $-alk_3-NH-alk_4-$ darstellen, wobei $alk_1$ und $alk_2$ Niederalkyliden bedeuten, $alk_3$ und $alk_4$ für Niederalkylen stehen, X eine direkte Bindung, Methylen oder unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenylen darstellt, $R_5$ Amino oder substituiertes Amino der Formel $-N(R_6)-CH(R_7)-R_8$ bedeutet, in der $R_6$ für Wasserstoff steht oder, sofern $R_3$ Wasserstoff ist, auch Niederalkyl oder unsubstituiertes oder durch aliphatische, cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenylniederalkyl bedeuten kann, $R_7$ Wasserstoff, Niederalkyl, unsubstituiertes oder durch aliphatische,cycloaliphatische oder araliphatische Reste, Hydroxy, veräthertes oder verestertes Hydroxy, unsubstituiertes oder aliphatisch substituiertes Amino, Acylamino, Nitro und/oder Trifluormethyl substituiertes Phenyl oder aliphatisch, cycloaliphatisch oder araliphatisch verestertes Carboxy darstellt, und $R_8$ unsubstituiertes oder durch aliphatische, cycloaliphatische oder araliphatische Reste, Hydroxy,veräthertes oder verestertes Hydroxy, unsubstituiertes oder aliphatisch substituiertes Amino, Acylamino, Nitro und/oder Trifluormethyl substituiertes Phenyl darstellt, mit der Massgabe, dass in Verbindungen, worin $R_3$ Wasserstoff oder Niederalkyl, $R_4$ eine Gruppe $-alk_1-X-alk_2-R_5$ und $R_5$ eine Gruppe $-N(R_6)-CH(R_7)-R_8$ bedeutet, mindestens einer der Reste $R_1$ und $R_8$ von unsubstituiertem oder N-substituiertem Aminoalkoxyphenyl oder mindestens einer der Reste $R_2$, $R_3$, $R_6$ und $R_7$ von Wasserstoff und Niederalkyl verschieden ist, wenn X unsubstituiertes oder wie angegeben substituiertes Phenylen bedeutet, und mindestens einer der Reste $R_1$ und $R_8$ von unsubstituiertem oder durch Methoxy und/oder Chlor mono- oder disubstituiertes Phenyl oder mindestens einer der Reste $R_2$, $R_3$, $R_6$ und $R_7$ von Wasserstoff verschieden ist, wenn $alk_1$ und $alk_2$ für Methylen stehen und X Methylen oder eine direkte Bindung darstellt, und mit der weiteren Massgabe, dass in Verbindungen, worin $R_3$ und $R_4$ gemeinsam eine Gruppe $-alk_3-NH-alk_4-$ darstellen und $alk_3$ Aethylen ist, $R_1$ von Phenyl oder $R_2$ von Phenyl und Wasserstoff und ferner $R_1$ von 2,3,4-Trimethoxyphenyl, o-Amino-dichlor-phenyl, o-Acylamino-dichlor-phenyl und o-Diacylamino-dichlor-phenyl oder $R_2$ von Wasserstoff verschieden ist, wenn $alk_4$ Aethylen bedeutet, und $R_1$ von Phenyl oder $R_2$ von Wasserstoff verschieden ist, wenn $alk_4$ 1,3-Propylen ist, wobei im Falle dass $R_4$ eine Gruppe $-alk_1-X-alk_2-R_5$, $alk_1$ und $alk_2$ Methylen und $R_5$ eine Gruppe der Formel $-N(R_6)-CH(R_7)-R_8$ darstellt, $R_1$ und $R_8$ keine gleichen aus unsubstituiertem oder in 3-, 4-. 5- und/oder 6-Stellung durch Isopropyl, Methoxy, Hydroxy, Chlor, Diäthylamino, und/oder Dimethylamino substituierten Phenylresten ausgewählte Substituenten bedeuten, wenn $R_2$, $R_3$, $R_6$ und $R_7$ Wasserstoff bedeuten und X für Methylen steht, und $R_1$ und $R_8$ keine gleichen aus p-Aminophenoxy- und p-Nitrophenoxyphenyl ausgewählte Reste bedeuten, wenn $R_2$ und $R_7$ Wasserstoff und $R_3$ und $R_6$ Wasserstoff oder Niederalkyl bedeuten und X für Methylen oder eine direkte Bindung steht, oder eines ihrer pharmazeutisch verwendbaren Salze, mit der Massgabe, dass Säureadditionssalze mit organischen Carbonsäuren im Anion keinen Lactamring aufweisen, zur Anwendung in einem Verfahren zur therapeutischen Behandlung des menschlichen oder tierischen Körpers.

19. Eine Verbindung gemäss Anspruch 17, worin $R_1$ unsubstituiertes oder durch Niederalkyl, Niederalkenyl, Niederalkinyl, Mono- oder Dihydroxyniederalkyl, Niederalkoxyniederalkyl, Niederalkanoyloxyniederalkyl, 3- bis 8-gliedriges Cycloalkyl, Hydroxy, Niederalkoxy, Niederalkenyloxy, Niederalkinyloxy, Mono- oder Dihydroxyniederalkoxy, Niederalkoxyniederalkoxy, an benachbarte Kohlenstoffatome gebundenes Niederalkyl(id)-endioxy, Halogen der Atomnummer bis und mit 35, Niederalkanoyloxy,

Amino, Mono- oder Diniederalkylamino und/oder Mono- oder Diniederalkanoylamino substituiertes Phenyl bedeutet, $R_2$ Wasserstoff, Niederalkyl, unsubstituiertes oder wie für $R_1$ angegeben substituiertes Phenyl, Niederalkoxycarbonyl oder 3- bis 8-gliedriges Cycloalkoxycarbonyl darstellt, $R_3$ Wasserstoff, Niederalkyl oder Phenylniederalkyl ist und $R_4$ eine Gruppe der Formel $-alk_1-X-alk_2-R_5$ bedeutet oder $R_3$ und $R_4$ gemeinsam eine Gruppe der Formel $-alk_3-NH-alk_4-$ darstellen, wobei $alk_1$ und $alk_2$ Niederalkyliden bedeuten, $alk_3$ und $alk_4$ für Niederalkylen stehen, X Methylen, eine direkte Bindung oder unsubstituiertes oder durch Niederalkyl, Niederalkoxy, Halogen und/oder Trifluormethyl substituiertes Phenylen darstellt, $R_5$ Amino oder substituiertes Amino der Formel $-N(R_6)-CH(R_7)-R_8$ bedeutet, $R_6$ Wasserstoff oder Niederalkyl ist, $R_7$ Wasserstoff, Niederalkyl oder unsubstituiertes oder wie für $R_2$ angegeben substituiertes Phenyl, Niederalkoxycarbonyl oder 3- bis 8-gliedriges Cycloalkoxycarbonyl darstellt und $R_8$ unsubstituiertes oder wie für $R_1$ angegeben substituiertes Phenyl bedeutet, mit der Massgabe, dass in Verbindungen der Formel I, worin $R_3$ und $R_4$ gemeinsam eine Gruppe $-alk_3-NH-alk_4-$ darstellen und $alk_3$ Aethylen bedeutet, $R_1$ von Phenyl oder $R_2$ von Phenyl und Wasserstoff und ferner $R_1$ von 2,3,4-Trimethoxyphenyl, o-Amino-dichlorphenyl, o-Niederalkanoylamino-dichlor-phenyl und o-Diniederalkanoylamino-dichlor-phenyl oder $R_2$ von Wasserstoff verschieden ist, wenn $alk_4$ Aethylen bedeutet, und $R_1$ von Phenyl oder $R_2$ von Wasserstoff verschieden ist, wenn $alk_4$ 1,3-Propylen ist, und mit der weiteren Massgabe, dass in Verbindungen der Formel I, worin $R_4$ eine Gruppe $-alk_1-X-alk_2-R_5$ und $R_5$ eine Gruppe $-N(R_6)-CH(R_7)-R_8$ bedeutet, mindestens einer der Reste $R_1$ und $R_8$ von unsubstituiertem oder durch Methoxy und/oder Chlor mono- oder disubstituiertem Phenyl oder mindestens einer der Reste $R_2$, $R_3$, $R_6$ und $R_7$ von Wasserstoff verschieden ist, wenn $alk_1$ und $alk_2$ für Methylen stehen und X Methylen oder eine direkte Bindung darstellt, wobei im Falle dass $R_4$ eine Gruppe $-alk_1-X-alk_2-R_5$, $alk_1$ und $alk_2$ Methylen und $R_5$ eine Gruppe der Formel $-N(R_6)-CH(R_7)-R_8$ darstellt, $R_1$ und $R_8$ keine gleichen aus unsubstituiertem oder in 3-

4-, 5- und/oder 6-Stellung durch Isopropyl, Methoxy, Hydroxy, Chlor, Diäthylamino und/oder Dimethylamino substituierten Phenylresten ausgewählte Substituenten bedeuten, wenn $R_2$, $R_3$ und $R_7$ Wasserstoff bedeuten und X für Methylen steht, oder eines ihrer pharmazeutisch verwendbaren Säureadditionssalze, mit der Massgabe, dass Additionssalze mit organischen Carbonsäuren keinen Lactamring aufweisen, zur Anwendung in einem Verfahren zur therapeutischen Behandlung des menschlichen oder tierischen Körpers.

20. Eine Verbindung gemäss Anspruch 17, worin $R_1$ unsubstituiertes oder durch Niederalkyl, Niederalkenyl, 3- bis 8-gliedriges Cycloalkyl, Niederalkoxy, Niederalkenyloxy, an benachbarte Kohlenstoffatome gebundenes Niederalkylendioxy, Halogen der Atomnummer bis und mit 35, Amino und/oder Mono- oder Diniederalkylamino substituiertes Phenyl bedeutet, $R_2$ Wasserstoff oder unsubstituiertes oder wie für $R_1$ angegeben substituiertes Phenyl darstellt, $R_3$ Wasserstoff oder Niederalkyl, wie Methyl ist und $R_4$ eine Gruppe der Formel $-alk_1-X-alk_2-R_5$ bedeutet, oder $R_3$ und $R_4$ gemeinsam eine Gruppe der Formel $alk_3-NH-alk_4-$ darstellt, wobei $alk_1$ und $alk_2$ Niederalkyliden bedeuten, $alk_3$ und $alk_4$ Niederalkylen bedeuten, X eine direkte Bindung, Methylen oder unsubstituiertes Phenylen ist, $R_5$ eine Gruppe $-N(R_6)-CH(R_7)-R_8$ darstellt, $R_6$ Wasserstoff oder Niederalkyl ist, $R_7$ Wasserstoff, Niederalkyl, unsubstituiertes oder wie für $R_2$ angegeben substituiertes Phenyl, Niederalkoxycarbonyl oder 3- bis 8-gliedriges Cycloalkoxycarbonyl darstellt und $R_8$ unsubstituiertes oder wie für $R_1$ angegeben substituiertes Phenyl bedeutet, mit der Massgabe, dass in Verbindungen der Formel I, worin $R_3$ und $R_4$ gemeinsam eine Gruppe $-alk_3-NH-alk_4-$ darstellen und $alk_3$ Aethylen bedeutet, $R_1$ von Phenyl oder $R_2$ von Phenyl und Wasserstoff und ferner $R_1$ von 2,3,4-Trimethoxyphenyl und o-Amino-dichlor-phenyl oder $R_2$ von Wasserstoff verschieden ist, wenn $alk_4$ Aethylen bedeutet, und dass $R_1$ von Phenyl oder $R_2$ von Wasserstoff verschieden ist, wenn $alk_4$ 1,3-Propylen ist, und mit der weiteren Massgabe, dass in Verbindungen der Formel I, worin $R_4$

eine Gruppe $-alk_1-X-alk_2-R_5$ und $R_5$ eine Gruppe $-N(R_6)-CH(R_8)-R_7$ bedeutet, mindestens einer der Reste $R_1$ und $R_8$ von unsubstituiertem oder durch Methoxy und/oder Chlor mono- oder disubstituiertem Phenyl oder mindestens einer der Reste $R_2$, $R_3$, $R_6$ und $R_7$ von Wasserstoff verschieden ist, wenn $alk_1$ und $alk_2$ für Methylen stehen und X Methylen oder eine direkte Bindung darstellt, wobei im Falle dass $R_4$ eine Gruppe $-alk_1-X-alk_2-R_5$, $alk_1$ und $alk_2$ Methylen und $R_5$ eine Gruppe der Formel $-N(R_6)-CH(R_7)-R_8$ darstellt, $R_1$ und $R_8$ keine gleichen aus unsubstituiertem oder in 3-, 4-, 5- und/oder 6-Stellung durch Isopropyl, Methoxy, Hydroxy, Chlor, Diäthylamino und/oder Dimethylamino substituierten Phenylresten ausgewählte Substituenten bedeuten, wenn $R_2$, $R_3$, $R_6$ und $R_7$ Wasserstoff bedeuten und X für Methylen steht, oder eines ihrer pharmazeutisch verwendbaren Säureadditionssalze, mit der Massgabe, dass Additionssalze mit organischen Carbonsäuren keinen Lactamring aufweisen, zur Anwendung in einem Verfahren zur therapeutischen Behandlung des menschlichen oder tierischen Körpers.

21. Eine Verbindung gemäss Anspruch 17, worin $R_1$ unsubstituiertes oder durch Niederalkyl mit bis und mit 4 Kohlenstoffatomen und/oder Halogen der Atomnummer bis und mit 35 substituiertes Phenyl bedeutet, $R_2$ Wasserstoff oder gegebenenfalls wie für $R_1$ angegebenes Phenyl bedeutet, $R_3$ Wasserstoff oder Niederalkyl mit bis und mit 4 Kohlenstoffatomen bedeutet und $R_4$ eine Gruppe der Formel $-CH_2-X-CH_2-R_5$ ist oder $R_3$ und $R_4$ gemeinsam eine Gruppe der Formel $-alk_3-NH-CH_2CH_2-$ darstellen, worin X eine direkte Bindung, Methylen oder Phenylen bedeutet, $R_5$ Amino oder substituiertes Amin der Formel $-N(R_6)-CH(R_7)-R_8$ ist, $R_6$ für Wasserstoff steht oder, sofern $R_3$ Wasserstoff ist, auch Niederalkyl mit bis und mit 4 Kohlenstoffatomen bedeuten kann, $R_7$ Wasserstoff, Niederalkyl mit bis und mit 4 Kohlenstoffatomen, unsubstituiertes oder wie für $R_1$ angegeben substituiertes Phenyl oder Niederalkoxycarbonyl mit bis und mit 4 Kohlenstoffatomen im Alkylteil bedeutet, $R_8$ unsubstituiertes oder wie für $R_1$ angegeben substituiertes Phenyl darstellt und $alk_3$ Niederalkylen mit 2 bis 4 Kohlenstoffatomen,

welches die beiden Stickstoffatome durch 2 oder 3 Kohlenstoffatome trennt, darstellt, mit der Massgabe, dass in Verbindungen, worin $R_3$ Wasserstoff oder Niederalkyl mit bis und mit 4 Kohlenstoffatomen und $R_4$ eine Gruppe der Formel $-CH_2-X-CH_2-N(R_6)-CH(R_7)-R_8$ bedeutet, mindestens einer der Reste $R_1$ und $R_8$ von unsubstituiertem oder durch Chlor mono- oder disubstituiertem Phenyl oder mindestens einer der Reste $R_2$, $R_3$, $R_6$ und $R_7$ von Wasserstoff verschieden ist, wenn X für Methylen oder eine direkte Bindung steht, und mit der weiteren Massgabe, dass in Verbindungen, worin $R_3$ und $R_4$ gemeinsam eine Gruppe $-alk_3-NH-CH_2CH_2-$ bedeuten, $R_1$ von Phenyl oder $R_2$ von Phenyl und Wasserstoff verschieden ist, wenn $alk_3$ Aethylen ist, und $R_1$ von Phenyl oder $R_2$ von Wasserstoff verschieden ist, wenn $alk_4$ 1,3-Propylen ist, wobei $R_1$ und $R_8$ keine gleichen aus unsubstituiertem oder in p-Stellung, den m-Stellungen oder einer o-Stellung durch Isopropyl oder Chlor substituiertem Phenyl ausgewählte Substituenten bedeuten, wenn $R_2$, $R_3$, $R_6$ und $R_7$ Wasserstoff bedeuten und X Methylen darstellt, oder eines ihrer pharmazeutisch verwendbaren Säureadditionssalze, mit der Massgabe, dass Additionssalze mit organischen Carbonsäuren keinen Lactamring aufweisen, zur Anwendung in einem Verfahren zur therapeutischen Behandlung des menschlichen oder tierischen Körpers.

22. N,N'-Bis-(diphenylmethyl)-äthylendiamin oder eines seiner pharmazeutisch verwendbaren Säureadditionssalze, mit der Massgabe, dass Additionssalze mit organischen Carbonsäuren im Anion keinen Lactamring aufweisen, zur Anwendung in einem Verfahren zur therapeutischen Behandlung des menschlichen oder tierischen Körpers.

23. N-Diphenylmethyl-äthylendiamin oder eines seiner pharmazeutisch verwendbaren Säureadditionssalze, mit der Massgabe, dass Additionssalze mit organischen Carbonsäuren im Anion keinen Lactamring aufweisen, zur Anwendung in einem Verfahren zur therapeutischen Behandlung des menschlichen oder tierischen Körpers.

24. N,N'-Di-(p-methylbenzyl)-äthylendiamin oder eines seiner pharmazeutisch verwendbaren Säureadditionssalze, mit der Massgabe, dass Additionssalze mit organischen Carbonsäuren im Anion keinen Lactamring aufweisen, zur Anwendung in einem Verfahren zur therapeutischen Behandlung des menschlichen oder tierischen Körpers.

25. N,N'-Dibenzyl-N,N'-dimethyl-äthylendiamin oder eines seiner pharmazeutisch verwendbaren Säureadditionssalze, mit der Massgabe, dass Additionssalze mit organischen Carbonsäuren im Anion keinen Lactamring aufweisen, zur Anwendung in einem Verfahren zur therapeutischen Behandlung des menschlichen oder tierischen Körpers.

26. Eine Verbindung gemäss einem der Ansprüche 1 - 14, gegebenenfalls in Form eines pharmazeutisch verwendbaren Salzes zur Anwendung in einem Verfahren zur therapeutischen Behandlung des menschlichen oder tierischen Körpers.

27. Eine Verbindung gemäss einem der Ansprüche 1 - 14 und 16 - 25 als atmungserleichterndes Mittel.

28. Pharmazeutische Präparate enthaltend mindestens eine Verbindung gemäss einem der Ansprüche 1 - 14 und 16 - 25 neben üblichen pharmazeutischen Hilfs- und/oder Trägerstoffen.

29. Verwendung einer Verbindung gemäss einem der Ansprüche 1 - 14 und 16 - 25 zur Herstellung eines Arzneimittels.

Patentansprüche (für Oesterreich)

1. Verfahren zur Herstellung neuer Verbindungen der Formel I

$$R_1-CH(R_2)-N(R_3)-R_4 \qquad (I) \quad ,$$

worin $R_1$ unsubstituiertes oder substituiertes Phenyl bedeutet, $R_2$ Wasserstoff, Niederalkyl, unsubstituiertes oder substituiertes Phenyl oder freies oder verestertes Carboxy darstellt, $R_3$ Wasserstoff oder Niederalkyl ist und $R_4$ eine Gruppe der Formel $-alk_1-X-alk_2-N(R_6)CH(R_7)-R_8$ darstellt, worin $alk_1$ und $alk_2$ Niederalkyliden bedeuten, X eine direkte Bindung, Methylen oder unsubstituiertes oder substituiertes Phenylen darstellt, $R_6$ für Wasserstoff steht oder, sofern $R_3$ Wasserstoff ist, auch Niederalkyl oder unsubstituiertes oder substituiertes Phenylniederalkyl bedeuten kann, $R_7$ Wasserstoff, Niederalkyl, unsubstituiertes oder substituiertes Phenyl oder freies oder verestertes Carboxy darstellt und $R_8$ unsubstituiertes oder substituiertes Phenyl bedeutet, mit der Massgabe, dass in Verbindungen der Formel I, worin X unsubstituiertes oder substituiertes Phenylen darstellt, mindestens einer der Reste $R_1$ und $R_8$ von unsubstituiertem oder N-substituiertem Aminoalkoxyphenyl verschieden ist, wenn $R_2$, $R_3$, $R_6$ und $R_7$ für Wasserstoff oder Niederalkyl stehen, dass ferner in Verbindungen der Formel I, worin $alk_1$ und $alk_2$ Methylen bedeuten und X Methylen oder eine direkte Bindung darstellen, mindestens einer der Reste $R_1$ und $R_8$ von unsubstituiertem oder durch Methoxy und/oder Chlor mono- oder disubstituiertem Phenyl und von p-Hydroxyphenyl verschieden ist, wenn $R_2$, $R_3$, $R_6$ und $R_7$ Wasserstoff darstellen, und dass weiterhin in Verbindungen der Formel I, worin $alk_1$ und $alk_2$ Methylen bedeuten und X eine direkte Bindung darstellt, mindestens einer der Reste $R_1$ und $R_8$ von Phenyl oder mindestens einer der Reste $R_3$ und $R_6$ von Wasserstoff verschieden ist, wenn beide Reste $R_2$ und $R_7$ jeweils Wasserstoff, Phenyl oder Methyl bedeuten oder $R_2$ Aethyl und $R_7$ Wasserstoff ist, wobei $R_1$ und $R_8$ keine gleichen aus unsubstituier-

tem oder in 3-, 4-, 5- und/oder 6-Stellung durch Isopropyl, Methoxy, Hydroxy, Chlor, Diäthylamino, Dimethylamino und/oder Nitro substituierten Phenylresten ausgewählte Substituenten bedeuten, wenn $R_2$, $R_3$, $R_6$ und $R_7$ Wasserstoff bedeuten und X für Methylen steht, $R_1$ und $R_8$ keine gleichen aus p-Anilino, p-Hydroxyphenyl und p-Methylphenyl ausgewählten Substituenten darstellen, wenn $R_2$, $R_3$, $R_6$ und $R_7$ für Wasserstoff stehen und X eine direkte Bindung bedeutet, und $R_1$ und $R_8$ keine gleichen aus p-Aminophenoxy- und p-Nitrophenoxyphenyl ausgewählte Reste bedeuten, wenn $R_2$ und $R_7$ Wasserstoff und $R_3$ und $R_6$ Wasserstoff oder Niederalkyl bedeuten und X für Methylen oder eine direkte Bindung steht, und ihrer Salze, dadurch gekennzeichnet, dass man eine Verbindung der Formel

$$Y_1\text{-}alk_1\text{-}X\text{-}alk_2\text{-}N(R_6)\text{-}CH(R_7)\text{-}R_8 \qquad \text{(II)}$$

mit einer Verbindung der Formel

$$R_1\text{-}CH(R_2)\text{-}Y_2 \qquad \text{(III)}$$

kondensiert, wobei einer der Reste $Y_1$ und $Y_2$ eine nukleophil ersetzbare Gruppe Y und der andere eine Gruppe der Formel $-N(R_3)-H$ darstellt oder in einer Verbindung der Formel

$$R_1\text{-}Y_3\text{-}X'\text{-}Y_4\text{-}R_8 \qquad \text{(VI)},$$

worin $Y_3$ einen in eine Gruppe der Formel $-CH(R_2)-N(R_3)-alk_1-$ überführbaren Rest $Y_3'$, X' eine Gruppe X oder einen in Methylen überführbaren Rest X" darstellt oder $Y_3$ eine Gruppe der Formel $-CH(R_2)-N(R_3)-alk_1-$ und X' einen in Methylen überführbaren Rest bedeutet, und worin $Y_4$ für eine Gruppe der Formel $-alk_2-N(R_6)-CH(R_7)-$ oder für einen in diese überführbaren Rest $Y_4'$ steht, oder in einem Salz davon $Y_3'$ in eine Gruppe der Formel $-CH(R_2)-N(R_3)-alk_1-$, X" in Methylen und/oder $Y_4'$ in eine Gruppe der Formel $-alk_2-N(R_6)-CH(R_7)-$ überführt, erforderlichenfalls

ein verfahrensgemäss erhältliches Isomerengemisch in die Komponenten auftrennt und gewünschtenfalls eine verfahrensgemäss erhältliche Verbindung in eine andere Verbindung der Formel I und/oder eine verfahrensgemäss erhältliche freie Verbindung in ein Salz oder ein verfahrensgemäss erhältliches Salz in die freie Verbindung oder in ein anderes Salz überführt.

2. Verfahren gemäss Anspruch 1, dadurch gekennzeichnet, dass man Verbindungen der Formel I, worin $R_1$ unsubstituiertes oder durch Niederalkyl, Niederalkenyl, Niederalkinyl, Mono- oder Dihydroxyniederalkyl, Niederalkoxyniederalkyl, Niederalkanoyloxyniederalkyl, 3- bis 8-gliedriges Cycloalkyl, Hydroxy, Niederalkoxy, Niederalkenyloxy, Niederalkinyloxy, Mono- oder Dihydroxyniederalkoxy, Niederalkoxyniederalkoxy, an benachbarte Kohlenstoffatome gebundenes Niederalkyl(id)endioxy, Halogen der Atomnummer bis und mit 35, Niederalkanoyloxy, Amino, Mono- oder Diniederalkylamino, und/oder Mono- oder Diniederalkanoylamino substituiertes Phenyl bedeutet, $R_2$ Wasserstoff, Niederalkyl, unsubstituiertes oder wie für $R_1$ angegeben substituiertes Phenyl, Niederalkoxycarbonyl oder 3- bis 8-gliedriges Cycloalkoxycarbonyl darstellt, $R_3$ Wasserstoff oder Niederalkyl ist und $R_4$ eine Gruppe der Formel $-alk_1-X-alk_2-N(R_6)-CH(R_7)-R_8$ darstellt, in der $alk_1$ und $alk_2$ Niederalkyliden bedeuten, X Methylen, eine direkte Bindung oder unsubstituiertes oder durch Niederalkyl, Niederalkoxy, Halogen und/oder Trifluormethyl substituiertes Phenylen bedeutet, $R_6$ Wasserstoff oder Niederalkyl ist, $R_7$ Wasserstoff, Niederalkyl oder unsubstituiertes oder wie für $R_2$ angegeben substituiertes Phenyl, Niederalkoxycarbonyl oder 3- bis 8-gliedriges Cycloalkoxycarbonyl darstellt und $R_8$ unsubstituiertes oder wie für $R_1$ angegeben substituiertes Phenyl bedeutet, mit der Massgabe, dass in Verbindungen der Formel I, worin $alk_1$ und $alk_2$ Methylen bedeuten und X Methylen oder eine direkte Bindung darstellt mindestens einer der Reste $R_1$ und $R_8$ von unsubstituiertem oder durch Methoxy und/oder Chlor mono- oder disubstituiertem Phenyl und von p-Hydroxyphenyl verschieden ist, wenn $R_2$, $R_3$,

$R_6$ und $R_7$ Wasserstoff darstellen, und mit der weiteren Massgabe, dass in Verbindungen der Formel I, worin $alk_1$ und $alk_2$ Methylen bedeuten und X eine direkte Bindung darstellt, mindestens einer der Reste $R_1$ und $R_8$ von Phenyl oder mindestens einer der Reste $R_3$ und $R_6$ von Wasserstoff verschieden ist, wenn beide Reste $R_2$ und $R_7$ jeweils Wasserstoff, Methyl oder Phenyl bedeuten, oder $R_2$ Aethyl und $R_7$ Wasserstoff ist, wobei $R_1$ und $R_8$ keine gleichen aus unsubstituiertem oder in 3-, 4-, 5- und/oder 6-Stellung durch Isopropyl, Methoxy, Hydroxy, Chlor, Diäthylamino und/oder Dimethylamin substituierten Phenylresten ausgewählte Substituenten bedeuten, wenn $R_2$, $R_3$, $R_6$ und $R_7$ Wasserstoff bedeuten und X für Methylen steht, und $R_1$ und $R_8$ keine gleichen aus p-Anilino, p-Hydroxyphenyl und p-Methylphenyl ausgewählte Substituenten darstellen, wenn $R_2$, $R_3$, $R_6$ und $R_7$ für Wasserstoff stehen und X eine direkte Bindung bedeutet, oder ein Salz davon herstellt.

3. Eine Verbindung gemäss Anspruch 1, dadurch gekennzeichnet, dass man Verbindungen der Formel I, worin $R_1$ unsubstituiertes oder durch Niederalkyl mit bis und mit 4 Kohlenstoffatomen, wie Methyl und/oder Halogen der Atomnummer bis und mit 35, wie Chlor, substituiertes Phenyl bedeutet, $R_2$ Wasserstoff oder gegebenenfalls wie für $R_1$ angegeben substituiertes Phenyl bedeutet, $R_3$ Wasserstoff oder Niederalkyl mit bis und mit 4 Kohlenstoffatomen, wie Methyl, bedeutet und $R_4$ eine Gruppe der Formel $-CH_2-X-CH_2-R_5$ ist, in der X eine direkte Bindung, Methylen oder Phenylen bedeutet, $R_5$ Amino oder substituiertes Amino der Formel $-N(R_6)-CH(R_7)-R_8$ ist, $R_6$ für Wasserstoff steht oder, sofern $R_3$ Wasserstoff ist, auch Niederalkyl mit bis und mit 4 Kohlenstoffatomen, wie Methyl, bedeuten kann, $R_7$ Wasserstoff, Niederalkyl mit bis und mit 4 Kohlenstoffatomen, wie Methyl, unsubstituiertes oder wie für $R_1$ angegeben substituiertes Phenyl oder Niederalkoxycarbonyl mit bis und mit 4 Kohlenstoffatomen im Alkylteil, wie Methoxycarbonyl bedeutet und $R_8$ unsubstituiertes oder wie für $R_1$ angegeben substituiertes Phenyl darstellt, mit

der Massgabe, dass mindestens einer der Reste $R_1$ und $R_8$ von unsubstituiertem oder durch Chlor mono- oder disubstituiertem Phenyl oder mindestens einer der Reste $R_2$, $R_3$, $R_6$ und $R_7$ von Wasserstoff verschieden ist, wenn X für Methylen oder eine direkte Bindung steht, und mit der weiteren Massgabe, dass mindestens einer der Reste $R_1$ und $R_8$ von Phenyl oder mindestens einer der Reste $R_3$ und $R_6$ von Wasserstoff verschieden ist, wenn beide Reste $R_2$ und $R_7$ Wasserstoff oder Phenyl darstellen und X eine direkte Bindung bedeutet, und mindestens einer der Reste $R_1$ und $R_8$ von p-Methylphenyl verschieden ist, wenn $R_2$, $R_3$, $R_6$ und $R_7$ Wasserstoff bedeuten und X Methylen ist, wobei $R_1$ und $R_8$ keine gleichen aus unsubstituiertem oder in p-Stellung, den m-Stellungen oder einer o-Stellung durch Isopropyl oder Chlor substituiertem Phenyl ausgewählte Substituenten bedeuten, wenn $R_2$, $R_3$, $R_6$ und $R_7$ Wasserstoff bedeuten und X Methylen darstellt oder ein Säureadditionssalz davon herstellt.

4. Verfahren gemäss Anspruch 1, dadurch gekennzeichnet dass man N,N'-Bis-(diphenylmethyl)-1,3-propylendiamin oder ein Säureadditionssalz davon herstellt.

5. Verfahren gemäss Anspruch 1, dadurch gekennzeichnet, dass man N,N'-Bis-(diphenylmethyl)-p-xylylen-diamin oder ein Säureadditionssalz davon herstellt.

6. Verfahren gemäss Anspruch 1, dadurch gekennzeichnet, dass man N-Diphenylmethyl-N'-(1-phenyläthyl)-äthylendiamin oder ein Säureadditionssalz davon herstellt.

7. Verfahren gemäss Anspruch 1, dadurch gekennzeichnet, dass man N-Diphenylmethyl-N'-(α-methoxycarbonylbenzyl)-äthylendiamin oder ein Säureadditionssalz davon herstellt.

8. Verfahren gemäss Anspruch 1, dadurch gekennzeichnet, dass man N,N'-Bis-(diphenylmethyl)-1,2-propylendiamin oder ein Säureadditionssalz davon herstellt.

9. Verfahren gemäss Anspruch 1, dadurch gekennzeichnet, dass man N,N'-Bis-(diphenylmethyl)-m-xylylen-diamin oder ein Säureadditionssalz davon herstellt.

10. Verfahren gemäss Anspruch 1, dadurch gekennzeichnet, dass man N,N'-Di-(o-methylbenzyl)-äthylendiamin oder ein Säureadditionssalz davon herstellt.

11. Verfahren gemäss Anspruch 1, dadurch gekennzeichnet, dass man N,N'-Dibenzyl-N-methyl-äthylendiamin oder ein Säureadditionssalz davon herstellt.

12. Verfahren gemäss Anspruch 1, dadurch gekennzeichnet, dass man N,N'-Dibenzyl-N-diphenylmethyl-äthylendiamin oder ein Säureadditionssalz davon herstellt.

13. Verfahren zur Herstellung von pharmazeutischen Präparaten, dadurch gekennzeichnet, dass man mindestens eine Verbindung gemäss einem der Ansprüchen 1 - 12 mit üblichen pharmazeutischen Hilfs- und/oder Trägerstoffen vermischt.

14. Verfahren zur Herstellung pharmazeutischer Präperate, als Wirkstoff enthaltend mindestens eine Verbindung der Formel I

$$R_1-CH(R_2)-N(R_3)-R_4 \qquad (I) \quad ,$$

worin $R_1$ unsubstituiertes oder substituiertes Phenyl bedeutet, $R_2$ Wasserstoff, Niederalkyl, unsubstituiertes oder substituiertes Phenyl oder freies oder verestertes Carboxy darstellt, $R_3$ Wasserstoff oder Niederalkyl und $R_4$ eine Gruppe der Formel $-alk_1-X-alk_2-R_5$ bedeutet oder $R_3$ und $R_4$ gemeinsam eine Gruppe der Formel $-alk_3-NH-alk_4-$ darstellen, wobei $alk_1$ und $alk_2$ Niederalkyliden bedeuten, $alk_3$ und $alk_4$ für Niederalkylen stehen, X eine direkte Bindung, Methylen oder

unsubstituiertes oder substituiertes Phenylen darstellt, $R_5$ Amino oder substituiertes Amino der Formel $-N(R_6)-CH(R_7)-R_8$ bedeutet, in der $R_6$ für Wasserstoff steht oder, sofern $R_3$ Wasserstoff ist, auch Niederalkyl oder unsubstituiertes oder substituiertes Phenylniederalkyl bedeuten kann, $R_7$ Wasserstoff, Niederalkyl, unsubstituiertes oder substituiertes Phenyl oder freies oder verestertes Carboxy darstellt und $R_8$ unsubstituiertes oder substituiertes Phenyl bedeutet, mit der Massgabe, dass in Verbindungen, worin $R_3$ Wasserstoff oder Niederalkyl, $R_4$ eine Gruppe $-alk_1-X-alk_2-R_5$ und $R_5$ eine Gruppe $-N(R_6)-CH(R_7)-R_8$ bèdeutet, mindestens einer der Reste $R_1$ und $R_8$ von unsubstituiertem oder N-substituiertem Aminoalkoxyphenyl oder mindestens einer der Reste $R_2$, $R_3$, $R_6$ und $R_7$ von Wasserstoff und Niederalkyl verschieden ist, wenn X unsubstituiertes oder substituiertes Phenylen bedeutet, und mindestens einer der Reste $R_1$ und $R_8$ von unsubstituiertem oder durch Methoxy und/oder Chlor mono- oder disubstituiertes Phenyl oder mindestens einer der Reste $R_2$, $R_3$, $R_6$ und $R_7$ von Wasserstoff verschieden ist, wenn $alk_1$ und $alk_2$ für Methylen stehen und X Methylen oder eine direkte Bindung bedeutet, und mit der weiteren Massgabe, dass in Verbindungen, worin $R_3$ und $R_4$ gemeinsam eine Gruppe $-alk_3-NH-alk_4-$ darstellen und $alk_3$ Aethylen ist, $R_1$ von Phenyl oder $R_2$ von Phenyl und Wasserstoff und ferner $R_1$ von 2,3,4-Trimethoxyphenyl, o-Amino-dichlor-phenyl, o-Acylamino-dichlor-phenyl und o-Diacylamino-dichlor-phenyl oder $R_2$ von Wasserstoff verschieden ist, wenn $alk_4$ Aethylen bedeutet, und $R_1$ von Phenyl oder $R_2$ von Wasserstoff verschieden ist, wenn $alk_4$ 1,3-Propylen ist, wobei im Falle, dass $R_4$ eine Gruppe $-alk_1-X-alk_2-R_5$, $alk_1$ und $alk_2$ Methylen und $R_5$ eine Gruppe der Formel $-N(R_6)-CH(R_7)-R_8$ darstellt, $R_1$ und $R_8$ keine gleichen aus unsubstituiertem oder in 3-, 4-, 5- und/oder 6-Stellung durch Isopropyl, Methoxy, Hydroxy, Chlor, Diäthylamino, Dimethylamino und/oder Nitro substituierten Phenylresten ausgewählte Substituenten bedeuten, wenn $R_2$, $R_3$, $R_6$ und $R_7$ Wasserstoff bedeuten und X für Methylen steht, und $R_1$ und $R_8$ keine gleichen aus p-Aminophenoxy- und p-Nitrophenoxyphenyl ausgewählte Reste bedeuten, wenn $R_2$ und $R_7$

Wasserstoff und $R_3$ und $R_6$ Wasserstoff oder Niederalkyl bedeuten und X für Methylen oder eine direkte Bindung steht, oder eines ihrer pharmazeutisch verwendbaren Salze, mit der Massgabe, dass Säureadditionssalze mit organischen Carbonsäuren im Anion keinen Lactamring aufweisen, dadurch gekennzeichnet dass man eine Verbindung der Formel

$$Y_1\text{-alk}_1\text{-X-alk}_2\text{-N}(R_6)\text{-CH}(R_7)\text{-}R_8 \qquad \text{(II)}$$

mit einer Verbindung der Formel

$$R_1\text{-CH}(R_2)\text{-}Y_2 \qquad \text{(III)}$$

oder eine Verbindung der Formel

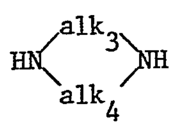

(II')

mit einer Verbindung der Formel

$$R_1\text{-CH}(R_2)\text{-Y} \qquad \text{(III')}$$

kondensiert, wobei einer der Reste $Y_1$ und $Y_2$ eine Gruppe Y und der andere eine Gruppe der Formel $-N(R_3)-H$ darstellt, und Y eine nukleophil ersetzbare Gruppe bedeutet oder in einer Verbindung der Formel

$$R_1\text{-}Y_3\text{-X'-}Y_4\text{-}R_8 \qquad \text{(VI)},$$

worin $Y_3$ einen in eine Gruppe der Formel $-CH(R_2)-N(R_3)-alk_1-$ überführbaren Rest $Y_3'$, X' eine Gruppe X oder einen in Methylen überführbaren Rest X" darstellt oder $Y_3$ eine Gruppe der Formel $-CH(R_2)-N(R_3)-alk_1-$ und X' einen in Methylen überführbaren Rest bedeutet, und worin $Y_4$ für eine Gruppe der Formel $-alk_2-N(R_6)-CH(R_7)-$ oder für einen in diese überführbaren Rest $Y_4'$ steht, oder in einem Salz davon $Y_3'$ in eine Gruppe der Formel $-CH(R_2)-N(R_3)-alk_1'-$, X" in Methylen und/oder $Y_4'$ in eine Gruppe der Formel $-alk_2-N(R_6)-CH(R_7)-$ überführt, erforderlichenfalls

ein verfahrensgemäss erhältliches Isomerengemisch in die Komponenten auftrennt und gewünschtenfalls eine verfahrensgemäss erhältliche Verbindung in eine andere Verbindung der Formel I und/oder eine verfahrensgemäss erhältliche freie Verbindung in ein Salz oder ein verfahrensgemäss erhältliches Salz in die freie Verbindung oder in ein anderes Salz überführt und das Reaktionsprodukt mit üblichen pharmazeutischen Hilfs- und/oder Trägerstoffen zu pharmazeutischen Präparaten verarbeitet.

15. Verfahren gemäss Anspruch 14, dadurch gekennzeichnet, dass man mindestens eine Verbindung der Formel I, worin $R_1$ unsubstituiertes oder durch Niederalkyl, Niederalkenyl, 3- bis 8-gliedriges Cycloalkyl, Niederalkoxy, Niederalkenyloxy, an benachbarten Kohlenstoffatomen durch Niederalkylendioxy, Halogen der Atomnummer bis und mit 35, Amino und/oder Mono- oder Diniederalkylamino substituiertes Phenyl bedeutet, $R_2$ Wasserstoff oder unsubstituiertes oder wie für $R_1$ angegeben substituiertes Phenyl darstellt, $R_3$ Wasserstoff oder Niederalkyl, wie Methyl ist und $R_4$ eine Gruppe der Formel $-alk_1-X-alk_2-R_5$ bedeutet, oder $R_3$ und $R_4$ gemeinsam eine Gruppe der Formel $alk_3-NH-alk_4-$ darstellt, wobei $alk_1$ und $alk_2$ Niederalkyliden bedeuten, $alk_3$ und $alk_4$ Niederalkylen bedeuten, X eine direkte Bindung, Methylen oder unsubstituiertes Phenylen ist, $R_5$ eine Gruppe $-N(R_6)-CH(R_7)-R_8$ darstellt, $R_6$ Wasserstoff oder Niederalkyl ist, $R_7$ Wasserstoff, Niederalkyl, unsubstituiertes oder wie für $R_2$ angegeben substituiertes Phenyl, Niederalkoxycarbonyl oder 3- bis 8-gliedriges Cycloalkoxycarbonyl darstellt und $R_8$ unsubstituiertes oder wie für $R_1$ angegeben substituiertes Phenyl bedeutet, mit der Massgabe, dass in Verbindungen der Formel I, worin $R_3$ und $R_4$ gemeinsam eine Gruppe $-alk_3-NH-alk_4-$ darstellen und $alk_3$ Aethylen bedeutet, $R_1$ von Phenyl oder $R_2$ von Phenyl und Wasserstoff und ferner $R_1$ von 2,3,4-Trimethoxyphenyl und o-Amino-dichlor-phenyl oder $R_2$ von Wasserstoff verschieden ist, wenn $alk_4$ Aethylen bedeutet, und dass $R_1$ von Phenyl oder $R_2$ von Wasserstoff verschieden ist, wenn $alk_4$ 1,3-Propylen ist, und mit der weiteren Massgabe, dass in Verbindungen der Formel I, worin $R_4$

eine Gruppe $-alk_1-X-alk_2-R_5$ und $R_5$ eine Gruppe $-N(R_6)-CH(R_8)-R_7$ bedeutet, mindestens einer der Reste $R_1$ und $R_8$ von unsubstituiertem oder durch Methoxy und/oder Chlor mono- oder disubstituiertem Phenyl oder mindestens einer der Reste $R_2$, $R_3$, $R_6$ und $R_7$ von Wasserstoff verschieden ist, wenn $alk_1$ und $alk_2$ für Methylen stehen und X Methylen oder eine direkte Bindung darstellt, wobei im Falle dass $R_4$ eine Gruppe $-alk_1-X-alk_2-R_5$, $alk_1$ und $alk_2$ Methylen und $R_5$ eine Gruppe der Formel $-N(R_6)-CH(R_7)-R_8$ darstellt, $R_1$ und $R_8$ keine gleichen aus unsubstituiertem oder in 3-, 4-, 5- und/oder 6-Stellung durch Isopropyl, Methoxy, Hydroxy, Chlor, Diäthylamino und/oder Dimethylamino substituierten Phenylresten ausgewählte Substituenten bedeuten, wenn $R_2$, $R_3$, $R_6$ und $R_7$ Wasserstoff bedeuten und X für Methylen steht, oder eines ihrer pharmazeutisch verwendbaren Säureadditionssalze, mit der Massgabe, dass Additionssalze mit organischen Carbonsäuren keinen Lactamring aufweisen, herstellt und mit üblichen pharmazeutischen Hilfs- und/oder Trägerstoffen in pharmazeutischen Präperaten verarbeitet.

16. Verfahren gemäss Anspruch 14, dadurch gekennzeichnet, dass man mindestens eine Verbindung der Formel I, worin $R_1$ unsubstituiertes oder durch Niederalkyl mit bis und mit 4 Kohlenstoffatomen und/oder Halogen der Atomnummer bis und mit 35 substituiertes Phenyl bedeutet, $R_2$ Wasserstoff oder gegebenenfalls wie für $R_1$ angegebenes Phenyl bedeutet, $R_3$ Wasserstoff oder Niederalkyl mit bis und mit 4 Kohlenstoffatomen bedeutet und $R_4$ eine Gruppe der Formel $-CH_2-X-CH_2-R_5$ ist oder $R_3$ und $R_4$ gemeinsam eine Gruppe der Formel $-alk_3-NH-CH_2CH_2-$ darstellen, worin X eine direkte Bindung, Methylen oder Phenylen bedeutet, $R_5$ Amino oder substituiertes Amin der Formel $-N(R_6)-CH(R_7)-R_8$ ist, $R_6$ für Wasserstoff steht oder, sofern $R_3$ Wasserstoff ist, auch Niederalkyl mit bis und mit 4 Kohlenstoffatomen bedeuten kann, $R_7$ Wasserstoff, Niederalkyl mit bis und mit 4 Kohlenstoffatomen, unsubstituiertes oder wie für $R_1$ angegeben substituiertes Phenyl oder Niederalkoxycarbonyl mit bis und mit 4 Kohlenstoffatomen im Alkylteil be-

deutet, $R_8$ unsubstituiertes oder wie für $R_1$ angegeben substituiertes Phenyl darstellt und $alk_3$ Niederalkylen mit 2 bis 4 Kohlenstoffatomen, welches die beiden Stickstoffatome durch 2 oder 3 Kohlenstoffatome trennt, darstellt, mit der Massgabe, dass in Verbindungen, worin $R_3$ Wasserstoff oder Niederalkyl mit bis und mit 4 Kohlenstoffatomen und $R_4$ eine Gruppe der Formel $-CH_2-X-CH_2-N(R_6)-CH(R_7)-R_8$ bedeutet, mindestens einer der Reste $R_1$ und $R_8$ von unsubstituiertem oder durch Chlor mono- oder disubstituiertem Phenyl oder mindestens einer der Reste $R_2$, $R_3$, $R_6$ und $R_7$ von Wasserstoff verschieden ist, wenn X für Methylen oder eine direkte Bindung steht, und mit der weiteren Massgabe, dass in Verbindungen, worin $R_3$ und $R_4$ gemeinsam eine Gruppe $-alk_3-NH-CH_2CH_2-$ bedeuten, $R_1$ von Phenyl oder $R_2$ von Phenyl und Wasserstoff verschieden ist, wenn $alk_3$ Aethylen ist, und $R_1$ von Phenyl oder $R_2$ von Wasserstoff verschieden ist, wenn $alk_4$ 1,3-Propylen ist, wobei $R_1$ und $R_8$ keine gleichen aus unsubstituiertem oder in p-Stellung, den m-Stellungen oder einer o-Stellung durch Isopropyl oder Chlor substituiertem Phenyl ausgewählte Substituenten bedeuten, wenn $R_2$, $R_3$, $R_6$ und $R_7$ Wasserstoff bedeuten und X Methylen darstellt, oder eines ihrer pharmazeutisch verwendbaren Säureadditionssalze, mit der Massgabe, dass Additionssalze mit organischen Carbonsäuren keinen Lactamring aufweisen, herstellt und mit üblichen pharmazeutischen Hilfs- und/ oder Trägerstoffen zu pharmazeutischen Präperaten verarbeitet.

17. Verfahren gemäss Anspruch 14, dadurch gekennzeichnet, dass man das N,N'-Bis-(diphenylmethyl)-äthylendiamin oder eines seiner pharmazeutisch verwendbaren Säureadditionssalze, mit der Massgabe, dass Additionssalze mit organischen Carbonsäuren im Anion keinen Lactamring aufweisen, herstellt und mit üblichen pharmazeutischen Hilfs- und/ oder Trägerstoffen zu pharmazeutischen Präperaten verarbeitet.

18. Verfahren gemäss Anspruch 14, dadurch gekennzeichnet, dass man das N-Diphenylmethyl-äthylendiamin oder eines seiner pharmazeutisch verwendbaren Säureadditionssalze, mit der Massgabe, dass Additionssalze mit organischen Carbonsäuren im Anion keinen Lactamring aufweisen, herstellt und mit üblichen pharmazeutischen Hilfs- und/oder Trägerstoffen zu pharmazeutischen Präperaten verarbeitet.

19. Verfahren gemäss Anspruch 14, dadurch gekennzeichnet, dass man das N,N'-Di-(p-methylbenzyl)-äthylendiamin oder eines seiner pharmazeutisch verwendbaren Säureadditionssalze, mit der Massgabe, dass Additionssalze mit organischen Carbonsäuren im Anion keinen Lactamring aufweisen, herstellt und mit üblichen pharmazeutischen Hilfs- und/oder Trägerstoffen zu pharmazeutischen Präperaten verarbeitet.

20. Verfahren gemäss Anspruch 14, dadurch gekennzeichnet, dass man das N,N'-Dibenzyl-N,N'-dimethyl-äthylendiamin oder eines seiner pharmazeutisch verwendbaren Säureadditonssalze, mit der Massgabe, dass Additionssalze mit organischen Carbonsäuren im Anion keinen Lactamring aufweisen, herstellt und mit üblichen pharmazeutischen Hilfs- und/oder Trägerstoffen zu pharmazeutischen Präperaten verarbeitet.

21. Verfahren gemäss einem der Ansprüche 14-20 zur Herstellung atmungserleichternder pharmazeutischer Präparate.

0058373

Europäisches Patentamt

# EUROPÄISCHER RECHERCHENBERICHT

Nummer der Anmeldung
EP 82100924.8

| EINSCHLÄGIGE DOKUMENTE | | | KLASSIFIKATION DER ANMELDUNG (Int. Cl.) |
|---|---|---|---|
| Kategorie | Kennzeichnung des Dokuments mit Angabe, soweit erforderlich, der maßgeblichen Teile | betrifft Anspruch | |
| X | GB - A - 786 215 (ALLEN & HANBURYS)<br>* Beispiele 1,2,6-8,21,22 *<br>-- | 1-5,15, 16 | C 07 C 87/28<br>C 07 C 101/24<br>C 07 C 85/04<br>C 07 C 85/00<br>A 61 K 31/135<br>A 61 K 31/195<br>A 61 K 31/22 |
| X | CH - A - 373 397 (MILES LABORATORIES)<br>* Beispiele 1,2,9,15,25 *<br>-- | 1-4,15-20,26 | |
| X | DE - A - 1 543 905 (STANDARD OIL)<br>* Beispiele 1,4,6,7 *<br>-- | 1 | RECHERCHIERTE SACHGEBIETE (Int Cl.³) |
| A | DE - A1 - 2 438 288 (KNOLL AG)<br>* Anspruch; Tabelle 3; Beispiel 1, Seiten 10,11 *<br>-- | 1,15-17,26, 28,29 | C 07 C 87/00<br>C 07 C 101/00<br>C 07 C 85/00<br>A 61 K |
| A | GB - A - 987 438 (FROSST & CO)<br>* Ansprüche 1,30; Beispiele XVI, XVII, XXVI; Seite 2, Zeilen 19-66 *<br>-- | 1,17,26, 28,29 | |
| A | DE - B - 1 008 308 (ASTA-WERKE)<br>* Gesamt *<br>---- | 1,15-17, 26 | |

KATEGORIE DER GENANNTEN DOKUMENTE

- X: von besonderer Bedeutung allein betrachtet
- Y: von besonderer Bedeutung in Verbindung mit einer anderen Veröffentlichung derselben Kategorie
- A: technologischer Hintergrund
- O: nichtschriftliche Offenbarung
- P: Zwischenliteratur
- T: der Erfindung zugrunde liegende Theorien oder Grundsätze
- E: älteres Patentdokument, das jedoch erst am oder nach dem Anmeldedatum veröffentlicht worden ist
- D: in der Anmeldung angeführtes Dokument
- L: aus andern Gründen angeführtes Dokument
- &: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 10-05-1982 | KÖRBER |